# EUROPEAN PATENT SPECIFICATION

(11) **EP 3 989 810 B1**
(45) Date of publication and mention of the grant of the patent: **09.04.2025**
(21) Application number: 20756949.2
(22) Date of filing: 29.06.2020
(51) Int. Cl.: A61B 5/053, A61B 18/14, A61B 18/00, A61N 1/36, A61N 1/05, A61B 5/0538, A61B 5/00

(54) **SYSTEMS FOR TARGETED THERAPEUTIC NASAL NEUROMODULATION**
SYSTEME ZUR GEZIELTEN THERAPEUTISCHEN NASALEN NEUROMODULATION
SYSTÈMES DE NEUROMODULATION NASALE THÉRAPEUTIQUE CIBLÉE

(30) Priority: 28.06.2019 US 201962868351 P; 28.06.2019 US 201962868365 P; 28.06.2019 US 201962868357 P
(43) Date of publication of application: 04.05.2022
(73) Proprietor: Neurent Medical Limited, Oranmore, Galway (IE)
(72) Inventor: TOWNLEY, David, Newmarket-on-Fergus County Clare (IE)
(74) Representative: HGF
(86) International application number: PCT/IB2020/000544
(87) International publication number: WO 2020/260950

(56) References cited:
- WO-A2-2007/008954
- WO-A2-2015/048806
- US-A1- 2005 283 148
- US-A1- 2016 331 459
- US-A1- 2018 133 460

## Description

### Field of the Invention

The invention generally relates to systems for providing precision targeting of neural tissue in a nasal region of a patient for the treatment of a rhinosinusitis condition while minimizing or avoiding collateral damage to surrounding tissue, such as blood vessels.

### Background

Rhinitis is an inflammatory disease of the nose and is reported to affect up to 40% of the population. It is the fifth most common chronic disease in the United States. The most common and impactful symptoms of rhinitis are congestion and rhinorrhea. Allergic rhinitis accounts for up to 65% of all rhinitis patients. Allergic rhinitis is an immune response to an exposure to allergens, such as airborne plant pollens, pet dander or dust. Non-allergic rhinitis is the occurrence of common rhinitis symptoms of congestion and rhinorrhea. As non-allergic rhinitis is not an immune response, its symptoms are not normally seasonal and are often more persistent. The symptoms of rhinitis include a runny nose, sneezing, and nasal itching and congestion.

Allergen avoidance and pharmacotherapy are relatively effective in the majority of mild cases, but these medications need to be taken on a long-term basis, incurring costs and side effects and often have suboptimal efficacy. For example, pharmaceutical agents prescribed for rhinosinusitis have limited efficacy and undesirable side effects, such as sedation, irritation, impairment to taste, sore throat, dry nose, and other side effects.

There are two modern surgical options: the delivery of thermal energy to the inflamed soft tissue, resulting in scarring and temporary volumetric reduction of the tissue to improve nasal airflow; and microdebrider resection of the inflamed soft tissue, resulting in the removal of tissue to improve nasal airflow. Both options address congestion as opposed to rhinorrhea and have risks ranging from bleeding and scarring to the use of general anesthetic. Importantly, these surgical procedures cannot precisely target neural tissue, thereby causing significant collateral damage to surrounding non-neural tissue (such as blood vessels) in order to treat rhinitis

Document US2018/133460 discloses a system for treating a condition within a nasal cavity of a patient.

### Summary

The invention is defined by the independent claim 1. The invention recognizes that knowing a depth, as well as specific location, of neural tissue within the nasal anatomy provides an ability to more precisely target neural tissue and minimize collateral damage to surrounding non-neural tissue (e.g., blood vessels). For example, the depth of neural tissues in the nasal anatomy varies depending on the nasal region and where in the nasal cavity treatment is being applied (e.g., there is up to a 6 times increase in depth of neural tissue on a nasal turbinate as compared to off of the nasal turbinate). The invention provides systems and methods with an ability to tune micro-lesion depths to accommodate location (e.g., depth) of the neural tissue within the nasal anatomy, which is important to prevent excessive damage and destruction to non-neural tissue when generating micro-lesions. In that manner, the invention solves the problem of causing collateral damage to non-neural tissue when generating micro-lesions within the nasal cavity and provides systems and methods for targeting neural tissue in a nasal region of a patient for the treatment of a rhinosinusitis condition that avoid or minimize creating collateral damage to surrounding or adjacent non-neural tissue, such as blood vessels as well as non-targeted neural tissue.

Aspects of the invention are accomplished with systems that include a treatment device and a controller for providing precise control and targeting of energy output from the treatment device. The treatment device includes an end effector comprising a micro-electrode array arranged about a plurality of struts. Each of the plurality of struts is able to conform to and accommodate an anatomical structure within the nasal cavity. When positioned within the nasal cavity, the struts contact multiple locations along multiple portions of a target site. The micro-electrode array includes at least a subset of electrodes configured to sense the presence of neural tissue at a respective position of each of the electrodes, as well as a depth of the neural tissue, and further convey such data to the controller. In turn, the controller processes such data and determines a level of therapeutic energy to be delivered by each of the plurality of electrodes sufficient to ablate neural tissue at each position while minimizing and/or preventing damage to a surrounding or adjacent structure, such as an artery or arterial wall adjacent to the neural tissue at each position.

The electrodes are configured to be independently controlled and activated by the controller to thereby deliver energy independent of one another. Accordingly, the neuromodulation energy can be applied to the tissue in a highly targeted manner and form micro-lesions to selectively modulate the target structure, while avoiding non-targeted structures (e.g., other neural tissue, blood vessels, etc.) and allowing the surrounding tissue structure to remain healthy for effective wound healing. In that manner, the present invention provides a treatment device that is capable of highly conforming to anatomical variations within a nasal cavity and further provides a controller providing unprecedented control to an operator so that an operator can perform an accurate, minimally invasive, and localized application of energy to target sites within the nasal cavity to cause multi-point interruption of neural signal without causing collateral damage or disruption to other, non-targeted tissue (e.g., blood vessels, other non-targeted neural tissue, etc.). More specifically, based on feedback data from the treatment device, the controller allows for the tuning of micro-lesion depths at a given position to accommodate variations in neural tissue, including depth of neural tissue as well as other physiological, bioelectric, and/or thermal properties, which is critical in preventing excessive damage and destruction to surrounding structures during a treatment procedure. For example, the depth/location of neural tissue can vary greatly depending on the region and position within the nasal cavity. A depth of neural tissue within a turbinate location can be six times greater than a non-turbinate location.

Accordingly, the system of the present invention provides a user-friendly, minimally-invasive means of treating rhinosinusitis conditions, including precise and focused application of energy to the intended target sites for therapeutic modulation of intended neural tissue, specifically the creation of multiple micro-lesion resulting in multi-point interruption of neural signals, without causing collateral damage or disruption to non-neural tissue (e.g., blood vessels) or non-targeted neural tissue. The creation of micro-lesions along a neural tissue is critical for various reasons. The creation of multiple micro-lesions, and resulting multi-point interruption, accounts for the various entry points and pathways associated with certain nerves, including the microforamina. The multiple micro-lesions further creates more than a single break along a nerve pathway, which is vital for longevity of the effect of treatment as it prevents functional alignment and regeneration of the nerve tissue. Furthermore, the controller is able to tailor the size and shape of any given micro-lesion at a respective location along a neural tissue by way of independent control of electrodes of the electrode array, thereby further enhancing the effectiveness of treatment by accounting for variations in neural tissue depth, for example, at any given location. The creation of multiple micro-lesions is much more effective than a single, continuous lesion, which presents further potential risks, such as an acute and latent risk of hemorrhage, which carries a high mortality risk due to the arteries and blood supply present in the nasal cavity.

One aspect of the invention provides a system for treating a condition within a nasal cavity of a patient. The system includes a device comprising an end effector including a plurality of electrodes and a controller operably associated with the device. The controller is configured to receive data from the device associated at least with presence and/or a depth/location of neural tissue at a position of each of the plurality of electrodes within the nasal cavity. The controller is further configured to process the data to determine a level of energy to be delivered by each of the plurality of electrodes such that the energy delivered at each position by each of the plurality of electrodes is sufficient to ablate the neural tissue at each position and minimize and/or prevent damage to an artery or arterial wall adjacent to the neural tissue at each position.

In some embodiments, a subset of the plurality of electrodes is configured to sense at least the presence and/or depth/location of neural tissue at the position of each of the plurality of electrodes and deliver data associated with the depths to the controller. For example, in one embodiment, a subset of the plurality of electrodes is configured to deliver non-therapeutic stimulating energy to respective positions within the nasal cavity at a frequency for locating neural tissue and further sense one or more properties, including at least the presence and/or depth/location of the neural tissue in response to the non-therapeutic stimulating energy.

In some embodiments, the controller is configured to tune energy output from each of the plurality of electrodes after an initial level of energy has been delivered based, at least in part, on feedback data received from the device. The feedback data may be associated with efficacy of ablation of the neural tissue at each position during and/or after delivery of initial energy from each of the plurality of electrodes. The feedback data includes one or more properties associated with neural tissue at respective positions within the nasal cavity. The one or more properties may include, but are not limited to, physiological properties, bioelectric properties, and thermal properties.

In some embodiments, a subset of the plurality of electrodes is configured to deliver non-therapeutic stimulating energy to respective positions within the nasal cavity at a frequency for locating neural tissue and sense one or more properties associated with the neural tissue in response to the non-therapeutic stimulating energy, wherein the properties comprise at least one of physiological properties, bioelectric properties, and thermal properties. For example, the bioelectric properties may include, but are not limited to, complex impedance, resistance, reactance, capacitance, inductance, permittivity, conductivity, nerve firing voltage, nerve firing current, depolarization, hyperpolarization, magnetic field, and induced electromotive force.

In some embodiments, at least some of the electrodes are configured to be independently controlled and activated by the controller to thereby deliver energy independent of one another. For example, at least some of the electrodes have different levels of energy to be delivered at respective positions sufficient to ablate neural tissue at the respective positions.

Another non-claimed aspect provides a method for treating a condition within a nasal cavity of a patient. The method includes providing a device comprising an end effector including a plurality of electrodes and a controller operably associated with the device and advancing the end effector within a nasal cavity of a patient. The method further includes receiving, via the controller, data from the device associated at least with a presence and/or depth/location of neural tissue at a position of each of the plurality of electrodes within the nasal cavity. The method further includes processing, via the controller, the data to determine a level of energy to be delivered by each of the plurality of electrodes such that the energy delivered at each position by each of the plurality of electrodes is sufficient to ablate the neural tissue at each position and minimize and/or prevent damage to an artery or arterial wall adjacent to the neural tissue at each position.

In some embodiments, a subset of the plurality of electrodes is configured to sense at least the presence and/or depth/location of neural tissue at the position of each of the plurality of electrodes and deliver data associated with the depths to the controller. For example, in some embodiments, a subset of the plurality of electrodes is configured to deliver non-therapeutic stimulating energy to respective positions within the nasal cavity at a frequency for locating neural tissue and further sense one or more properties, including at least the presence and/or depth/location of the neural tissue in response to the non-therapeutic stimulating energy.

In some embodiments, the controller is configured to tune energy output from each of the plurality of electrodes after an initial level of energy has been delivered based, at least in part, on feedback data received from the device. The feedback data is associated with efficacy of ablation of the neural tissue at each position during and/or after delivery of initial energy from each of the plurality of electrodes. For example, the feedback data may include one or more properties associated with neural tissue at respective positions within the nasal cavity. The one or more properties may include, but are not limited to, physiological properties, bioelectric properties, and thermal properties.

In some embodiments, a subset of the plurality of electrodes is configured to deliver non-therapeutic stimulating energy to respective positions within the nasal cavity at a frequency for locating neural tissue and sense one or more properties associated with the neural tissue in response to the non-therapeutic stimulating energy, wherein the properties comprise at least one of physiological properties, bioelectric properties, and thermal properties. For example, the bioelectric properties may include, but are not limited to, complex impedance, resistance, reactance, capacitance, inductance, permittivity, conductivity, nerve firing voltage, nerve firing current, depolarization, hyperpolarization, magnetic field, and induced electromotive force.

In some embodiments, the energy delivered may cause vacuolar degeneration of the neural tissue. In some embodiments, energy delivered at each position by each of the plurality of electrodes may cause vacuolar degeneration of the neural tissue at each position. In some embodiments, the energy delivered may cause ionic agitation resulting in vacuolar degeneration of the neural tissue.

Yet still, in other embodiments, the energy delivered results in vacuolar degeneration of the neural tissue and/or necrosis or necrotic-like cell death of the neural tissue. Accordingly, the energy delivered causes ionic agitation by way of an osmotic potential change through which extracellular fluid to neural tissue passes into the nerve tubules, thereby resulting in vacuolar degeneration and/or necrosis or necrotic-like cell death of the neural tissue.

### Brief Description of the Drawings

FIGS. 1A and 1B are diagrammatic illustrations of a therapeutic neuromodulation system for treating a condition within a nasal cavity using a handheld device according to some embodiments of the present disclosure.
FIG. 2 is a diagrammatic illustration of the console coupled to the handheld neuromodulation device consistent with the present disclosure, further illustrating one embodiment of an end effector of the handheld device for delivering energy to tissue at the one or more target sites within the nasal cavity.
FIG. 3 is a diagrammatic illustration of the console coupled to the handheld neuromodulation device consistent with the present disclosure, further illustrating another embodiment of an end effector of the handheld device for delivering energy, via proximal and distal segments, to tissue at the one or more target sites within the nasal cavity.
FIG. 4A is a cut-away side view illustrating the anatomy of a lateral nasal wall.
FIG. 4B is an enlarged side view of the nerves of the lateral nasal wall of FIG. 1A.
FIG. 4C is a front view of a left palatine bone illustrating geometry of microforamina in the left palatine bone.
FIG. 5 is a side view of one embodiment of a handheld device for providing therapeutic nasal neuromodulation consistent with the present disclosure.
FIG. 6 is an enlarged, perspective view of the end effector of FIG. 2 in an expanded state.

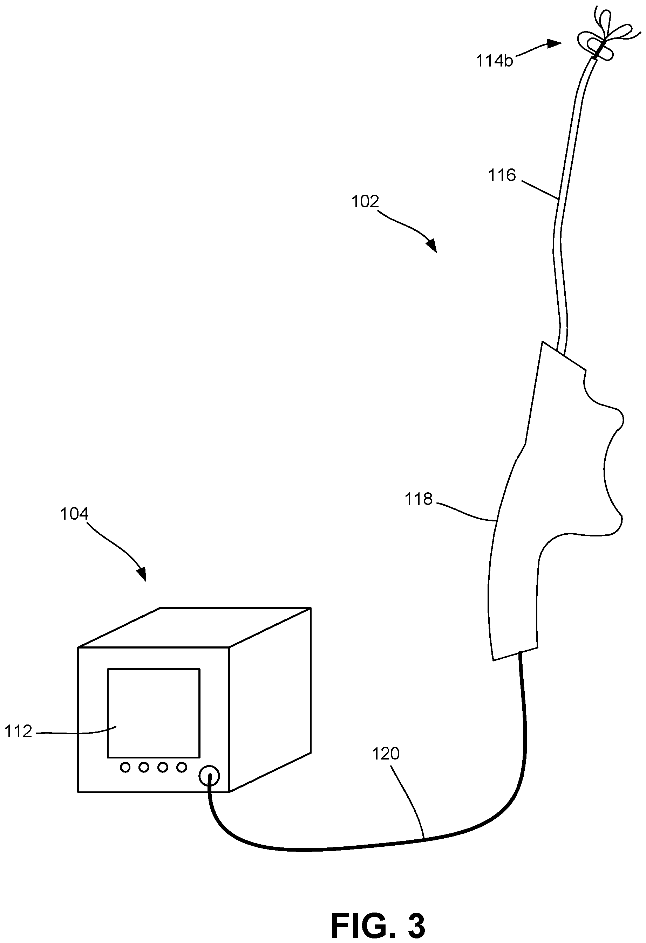

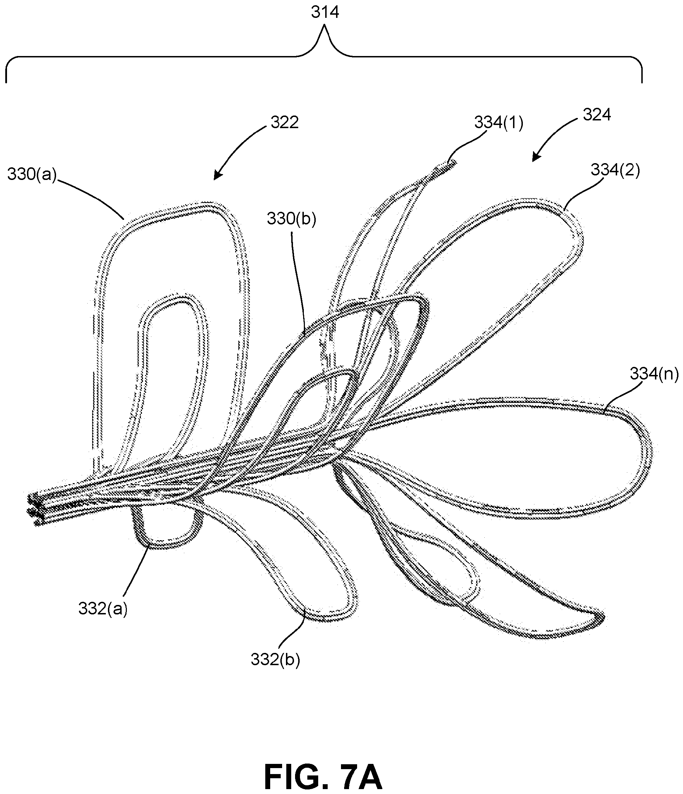

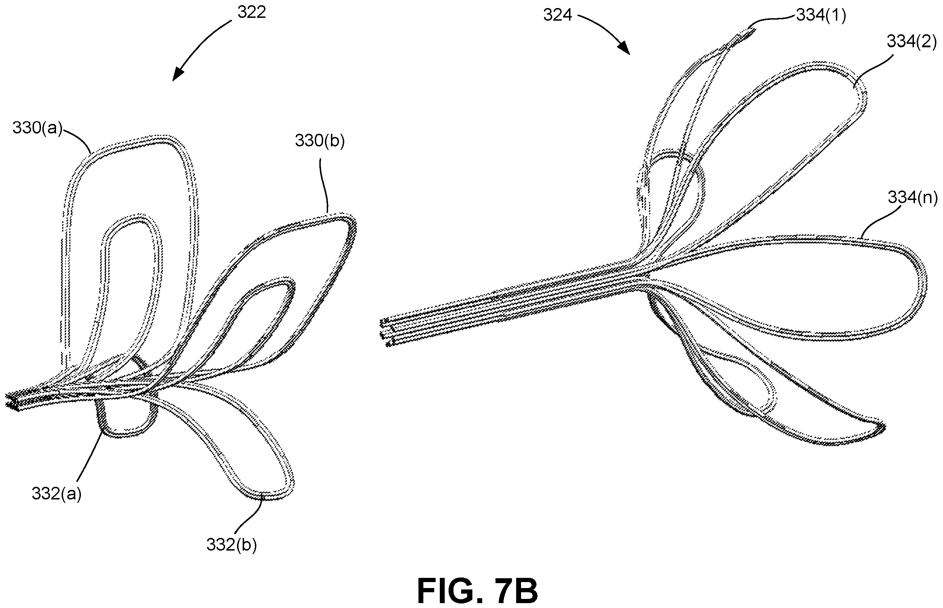

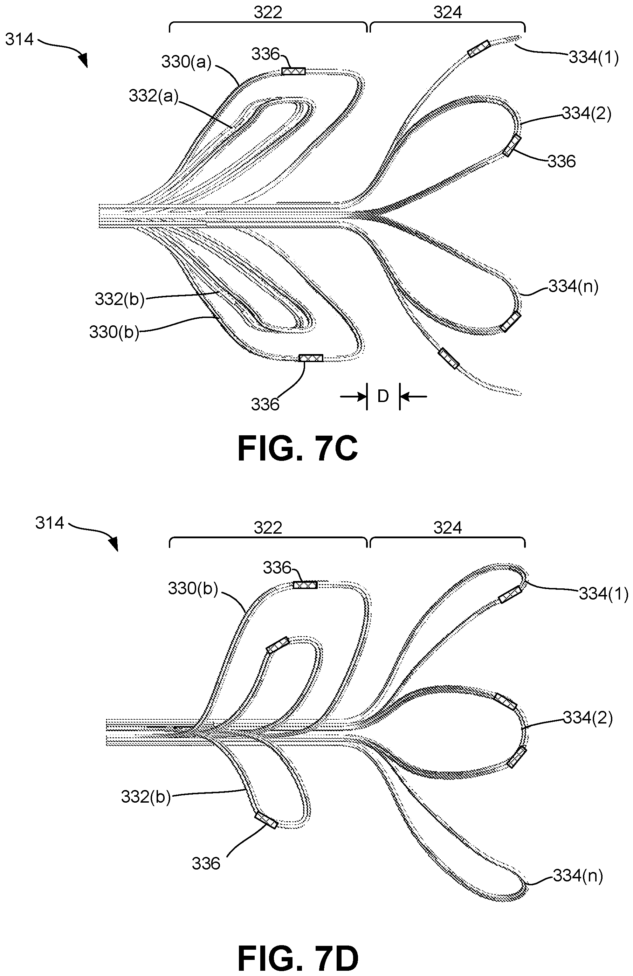

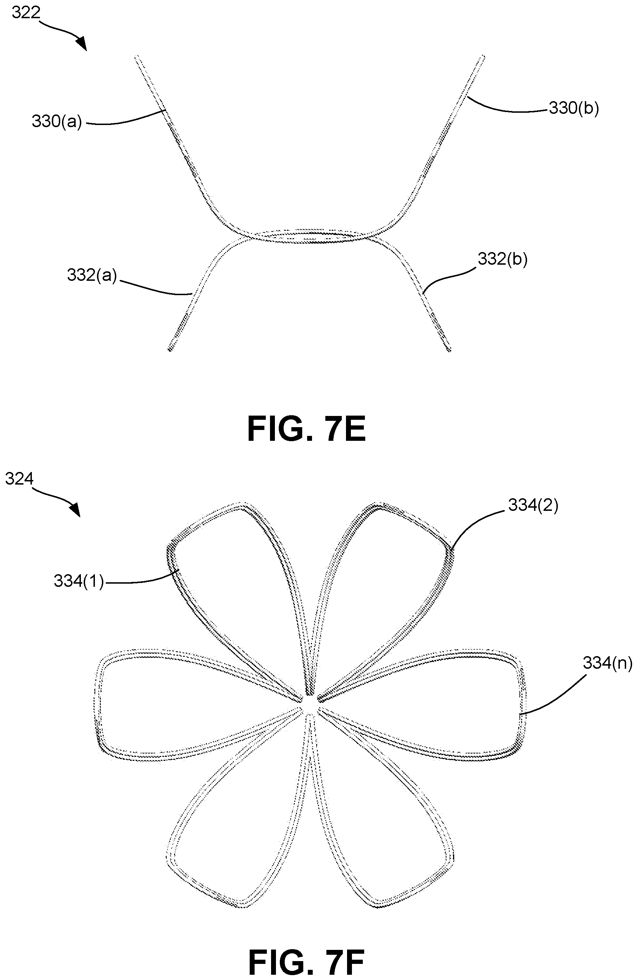

FIGS. 7A-7F are various views of the multi-segment end effector of FIG. 3. FIG. 7A is an enlarged, perspective view of the multi-segment end effector illustrating the first (proximal) segment and second (distal) segment. FIG. 7B is an exploded, perspective view of the multi-segment end effector. FIG. 7C is an enlarged, top view of the multi-segment end effector. FIG. 7D is an enlarged, side view of the multi-segment end effector. FIG. 7E is an enlarged, front (proximal facing) view of the first (proximal) segment of the multi-segment end effector. FIG. 7F is an enlarged, front (proximal facing) view of the second (distal) segment of the multi-segment end effector.
FIG. 8 is a perspective view, partly in section, of a portion of a support element illustrating an exposed conductive wire serving as an energy delivery element or electrode element.
FIG. 9 is a cross-sectional view of a portion of the shaft of the handheld device taken along lines 9-9 of FIG. 5.
FIG. 10A is a side view of the handle of the handheld device.
FIG. 10B is a side view of the handle illustrating internal components enclosed within.
FIG. 11 is a partial cut-away side view illustrating one approach for delivering an end effector a target site within a nasal region in accordance with embodiments of the present disclosure.
FIG. 12 is a block diagram illustrating communication of sensor data from the handheld device to the controller and subsequent tuning, via the controller, of energy output from each of the plurality of electrodes based on the sensor data for precision targeting and treatment of neural tissue.
FIG. 13A is a flow diagram illustrating one embodiment of a non-claimed method for treating a condition within a nasal cavity of a patient.
FIG. 13B is a flow diagram illustrating another embodiment of a non-claimed method for treating a condition within a nasal cavity of a patient.
FIG. 14A is a block diagram illustrating delivery of non-therapeutic energy from electrodes of the end effector at a frequency for locating neural tissue and sensing one or more properties associated with the neural tissue in response to the non-therapeutic energy.
FIG. 14B is a block diagram illustrating communication of sensor data from the handheld device to the controller and subsequent tuning, via the controller, of energy output based on the sensor data for precision targeting of neural tissue.
FIG. 14C is a block diagram illustrating delivery of energy (at diagnostic or therapeutic levels) to the target site specifically targeting neural tissue based on the tuned energy output from the controller.
FIG. 15 is a flow diagram illustrating one embodiment of a non-claimed method for treating a peripheral neurological condition.
FIG. 16 is a flow diagram illustrating one embodiment of a non-claimed method for diagnosing and/or treating a neurological condition of a patient.
FIG. 17A is a histological image of a tissue sample from a nasal cavity stained with Haemotoxylin and Eosin (H&E) having undergone a targeted therapeutic nasal neuromodulation in accordance with the systems and methods of the present invention.
FIG. 17B is a histological image of a tissue sample from a nasal cavity stained with H&E illustrating a deep-set nerve bundle with axonal vacuolar degeneration as a result of targeted therapeutic nasal neuromodulation in accordance with the systems and methods of the present invention.
FIG. 18A is a histological image of a tissue sample from a nasal cavity stained with H&E having undergone a targeted therapeutic nasal neuromodulation in accordance with the systems and methods of the present invention.
FIGS. 18B and 18C are magnified views of a portion of the histological image of FIG. 18A illustrating a deep-set nerve bundle with axonal vacuolar degeneration as a result of targeted therapeutic nasal neuromodulation in accordance with the systems and methods of the present invention.
FIGS. 19A-19D are additional histological images of tissue sample from a nasal cavity stained with H&E having undergone a targeted therapeutic nasal neuromodulation in accordance with the systems and methods of the present invention.
FIG. 20A is a histological image of a tissue sample from a nasal cavity stained with H&E having undergone a targeted therapeutic nasal neuromodulation in accordance with the systems and methods of the present invention.
FIG. 20B is a histological image of a tissue sample from a nasal cavity stained with H&E illustrating a deep-set nerve bundle with axonal vacuolar degeneration as a result of targeted therapeutic nasal neuromodulation in accordance with the systems and methods of the present invention.
FIGS. 21A-21D are additional histological images of tissue sample from a nasal cavity stained with H&E having undergone a targeted therapeutic nasal neuromodulation in accordance with the systems and methods of the present invention.
FIGS. 22A, 22B, 22C, and 22D are histological images of a tissue sample from a nasal cavity stained with H&E having undergone a targeted therapeutic nasal neuromodulation in accordance with the systems and methods of the present invention.
FIGS. 23A-23D, 24A-24C, and 25 are additional histological images of tissue sample from a nasal cavity stained with H&E having undergone a targeted therapeutic nasal neuromodulation in accordance with the systems and methods of the present invention.

### Detailed Description

There are various conditions related to the nasal cavity which may impact breathing and other functions of the nose. One of the more common conditions is rhinitis, which is defined as inflammation of the membranes lining the nose. The symptoms of rhinitis include nasal blockage, obstruction, congestion, nasal discharge (e.g., rhinorrhea and/or posterior nasal drip), facial pain, facial pressure, and/or reduction or complete loss of smell and/or taste. Sinusitis is another common condition, which involves an inflammation or swelling of the tissue lining the sinuses, which can lead to subsequent. Rhinitis and sinusitis are frequently associated with one another, as sinusitis is often preceded by rhinitis. Accordingly, the term rhinosinusitis is often used to describe both conditions.

Depending on the duration and type of systems, rhinosinusitis can fall within different subtypes, including allergic rhinitis, non-allergic rhinitis, chronic rhinitis, acute rhinitis, recurrent rhinitis, chronic sinusitis, acute sinusitis, recurrent sinusitis, and medical resistant rhinitis and/or sinusitis, in addition to combinations of one or more of the preceding conditions. It should be noted that an acute rhinosinusitis condition is one in which symptoms last for less than twelve weeks, whereas a chronic rhinosinusitis condition refers to symptoms lasting longer than twelve weeks.

A recurrent rhinosinusitis condition refers to four or more episodes of an acute rhinosinusitis condition within a twelve-month period, with resolution of symptoms between each episode. There are numerous environmental and biological causes of rhinosinusitis. Non-allergic rhinosinusitis, for example, can be caused by environmental irritants, medications, foods, hormonal changes, and/or nasal septum deviation. Triggers of allergic rhinitis can include exposure to seasonal allergens, perennial allergens that occur any time of year, and/or occupational allergens. Accordingly, rhinosinusitis affects millions of people and is a leading cause for patients to seek medical care.

The invention recognizes that knowing a depth, as well as specific location, of neural tissue within the nasal anatomy provides an ability to more precisely target neural tissue and minimize collateral damage to surrounding non-neural tissue (e.g., blood vessels). For example, the depth of neural tissues in the nasal anatomy varies depending on the nasal region and where in the nasal cavity treatment is being applied (e.g., there is up to a 6 times increase in depth of neural tissue on a nasal turbinate as compared to off of the nasal turbinate).

The invention provides systems with an ability to tune micro-lesion depths to accommodate location (e.g., depth) of the neural tissue within the nasal anatomy, which is important to prevent excessive damage and destruction to non-neural tissue when generating micro-lesions. Accordingly, the neuromodulation energy can be applied to the tissue in a highly targeted manner that forms micro-lesions to selectively modulate the targeted neural tissue, while avoiding non-targeted tissue (e.g., other neural tissue, blood vessels, etc.) and allowing the surrounding tissue structure to remain healthy for effective wound healing. In that manner, the invention solves the problem of causing collateral damage to non-neural tissue when generating micro-lesions within the nasal cavity and provides systems and methods for targeting neural tissue in a nasal region of a patient for the treatment of a rhinosinusitis condition that avoid or minimize creating collateral damage to surrounding or adjacent non-neural tissue, such as blood vessels as well as non-targeted neural tissue.

It should be noted that, although many of the embodiments are described with respect to systems for therapeutically modulating nerves in the nasal region for the treatment of rhinitis, other applications and other embodiments in addition to those described herein are within the scope of the present disclosure. For example, at least some embodiments of the present disclosure may be useful for the treatment of other indications, such as the treatment of chronic sinusitis and epistaxis. In particular, the embodiments described herein may be configured to treat allergic rhinitis, non-allergic rhinitis, chronic rhinitis, acute rhinitis, chronic sinusitis, acute sinusitis, chronic rhinosinusitis, acute rhinosinusitis, and/or medical resistant rhinitis.

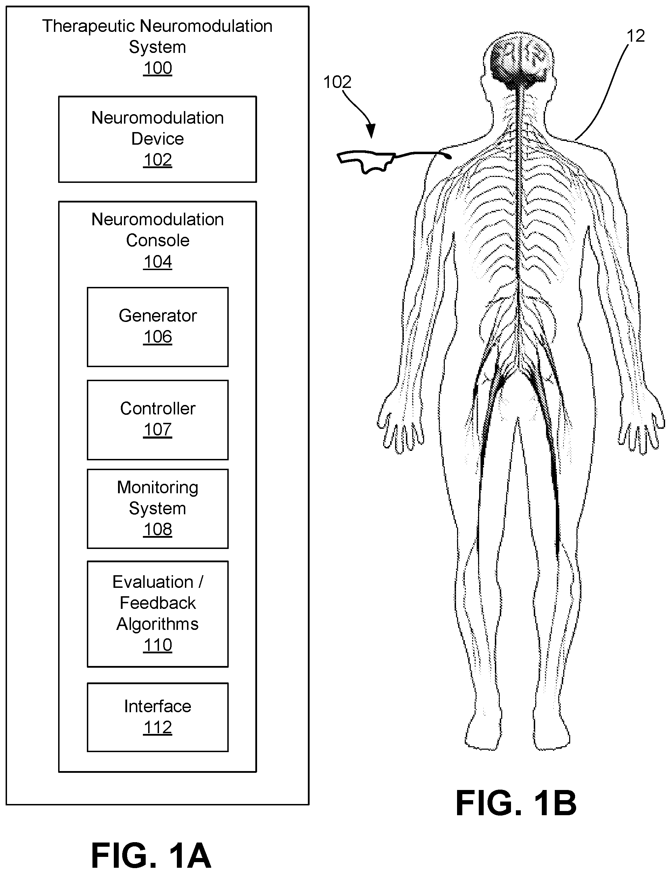

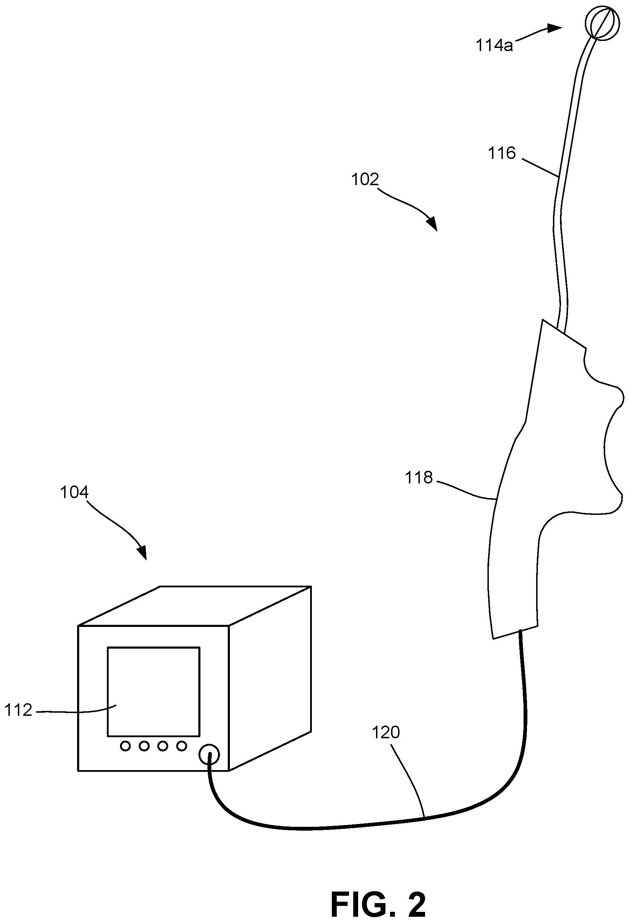

FIGS. 1A and 1B are diagrammatic illustrations of a therapeutic neuromodulation system 100 for treating a condition within a nasal cavity using a handheld device 102 according to some embodiments of the present disclosure. The system 100 generally includes a neuromodulation device 102 and a neuromodulation console 104 to which the device 102 is to be connected. FIGS. 2 and 3 are diagrammatic illustrations of the console 104 coupled to the handheld neuromodulation device 102 illustrating two different embodiments of an end effector (end effector 114a and end effector 114b) for delivering energy to tissue at the one or more target sites within the nasal cavity. For ease of description, the end effector embodiments 114a and 114b may be collectively referred to as "end effector 114" in the following description. As illustrated, the neuromodulation device 102 is a handheld device, which includes a collapsible/retractable and expandable end effector 114, a shaft 116 operably associated with the end effector 114, and a handle 118 operably associated with the shaft 116. The end effector 114 is configured to be advanced into the nasal cavity of a patient 12 and positioned at a location associated with one or more target sites to undergo therapeutic neuromodulation treatment. It should be noted that the terms "end effector" and "therapeutic assembly" may be used interchangeably throughout this disclosure.

For example, a surgeon or other medical professional performing a procedure can utilize the handle 118 to manipulate and advance the shaft 116 within the nasal cavity, wherein the shaft 116 is configured to locate at least a distal portion thereof intraluminally at a treatment or target site within a nasal region. The one or more target sites may generally be associated with postganglionic parasympathetic fibers that innervate the nasal mucosa. The target site may be a region, volume, or area in which the target nerves are located and may differ in size and shape depending upon the anatomy of the patient. Once positioned, the end effector 114 may be deployed and subsequently deliver energy to the one or more target sites to thereby therapeutically modulating nerves of interest, particularly nerves associated with a rhinosinusitis condition so as to treat such condition. For example, the end effector 114 may include at least one energy delivery element, such as an electrode, configured to therapeutically modulate the postganglionic parasympathetic nerves. For example, one or more electrodes may be provided by one or more portions of the end effector 114, wherein the electrodes may be configured to apply electromagnetic neuromodulation energy (e.g., radiofrequency (RF) energy) to target sites. In other embodiments, the end effector 114 may include other energy delivery elements configured to provide therapeutic neuromodulation using various other modalities, such as cryotherapeutic cooling, ultrasound energy (e.g., high intensity focused ultrasound ("HIFU") energy), microwave energy (e.g., via a microwave antenna), direct heating, high and/or low power laser energy, mechanical vibration, and/or optical power.

In some embodiments, the end effector 114 may include one or more sensors (not shown), such as one or more temperature sensors (e.g., thermocouples, thermistors, etc.), impedance sensors, and/or other sensors. The sensors and/or the electrodes may be connected to one or more wires extending through the shaft 116 and configured to transmit signals to and from the sensors and/or convey energy to the electrodes.

As shown, the device 102 is operatively coupled to the console 104 via a wired connection, such as cable 120. It should be noted, however, that the device 102 and console 104 may be operatively coupled to one another via a wireless connection. The console 104 is configured to provide various functions for the neuromodulation device 102, which may include, but is not limited to, controlling, monitoring, supplying, and/or otherwise supporting operation of the neuromodulation device 102. For example, when the neuromodulation device 102 is configured for electrode-based, heat-element-based, and/or transducer-based treatment, the console 104 may include an energy generator 106 configured to generate RF energy (e.g., monopolar, bipolar, or multi-polar RF energy), pulsed electrical energy, microwave energy, optical energy, ultrasound energy (e.g., intraluminally-delivered ultrasound and/or HIFU), direct heat energy, radiation (e.g., infrared, visible, and/or gamma radiation), and/or another suitable type of energy.

In some embodiments, the console 104 may include a controller 107 communicatively coupled to the neuromodulation device 102. However, in the embodiments described herein, the controller 107 may generally be carried by and provided within the handle 118 of the neuromodulation device 102. The controller 107 is configured to initiate, terminate, and/or adjust operation of one or more electrodes provided by the end effector 114 directly and/or via the console 104. For example, the controller 107 can be configured to execute an automated control algorithm and/or to receive control instructions from an operator (e.g., surgeon or other medical professional or clinician). For example, the controller 107 and/or other components of the console 104 (e.g., processors, memory, etc.) can include a computer-readable medium carrying instructions, which when executed by the controller 107, causes the device 102 to perform certain functions (e.g., apply energy in a specific manner, detect impedance, detect temperature, detect nerve locations or anatomical structures, etc.). A memory includes one or more of various hardware devices for volatile and non-volatile storage, and can include both read-only and writable memory. For example, a memory can comprise random access memory (RAM), CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory.

The console 104 may further be configured to provide feedback to an operator before, during, and/or after a treatment procedure via evaluation/feedback algorithms 110. For example, the evaluation/feedback algorithms 110 can be configured to provide information associated with the temperature of the tissue at the treatment site, the location of nerves at the treatment site, and/or the effect of the therapeutic neuromodulation on the nerves at the treatment site. In certain embodiments, the evaluation/feedback algorithm 110 can include features to confirm efficacy of the treatment and/or enhance the desired performance of the system 100. For example, the evaluation/feedback algorithm 110, in conjunction with the controller 107, can be configured to monitor temperature at the treatment site during therapy and automatically shut off the energy delivery when the temperature reaches a predetermined maximum (e.g., when applying RF energy) or predetermined minimum (e.g., when applying cryotherapy). In other embodiments, the evaluation/feedback algorithm 110, in conjunction with the controller 107, can be configured to automatically terminate treatment after a predetermined maximum time, a predetermined maximum impedance rise of the targeted tissue (i.e., in comparison to a baseline impedance measurement), a predetermined maximum impedance of the targeted tissue), and/or other threshold values for biomarkers associated with autonomic function. This and other information associated with the operation of the system 100 can be communicated to the operator via a graphical user interface (GUI) 112 provided via a display on the console 104 and/or a separate display (not shown) communicatively coupled to the console 104, such as a tablet or monitor. The GUI 112 may generally provide operational instructions for the procedure, such as directing the operator to select which nasal cavity to treat, indicating when the device 102 is primed and ready to perform treatment, and further providing status of therapy during the procedure, including indicating when the treatment is complete.

For example, in some embodiments, the end effector 114 and/or other portions of the system 100 can be configured to detect various parameters of the heterogeneous tissue at the target site to determine the anatomy at the target site (e.g., tissue types, tissue locations, vasculature, bone structures, foramen, sinuses, etc.), locate nerves and/or other structures, and allow for neural mapping. For example, the end effector 114 may be configured to detect impedance, dielectric properties, temperature, and/or other properties that indicate the presence of neural tissue or fibers in the target region.

As shown in FIG. 1A, the console 104 further includes a monitoring system 108 configured to receive data from the end effector 114 (i.e., detected electrical and/or thermal measurements of tissue at the target site), specifically sensed by appropriate sensors (e.g., temperature sensors and/or impedance sensors, or the like), and process this information to identify the presence of nerves, the location of nerves, neural activity at the target site, and/or other properties of the neural tissue, such a physiological properties (e.g., depth), bioelectric properties, and thermal properties. The nerve monitoring system 108 can be operably coupled to the electrodes and/or other features of the end effector 114 via signal wires (e.g., copper wires) that extend through the cable 120 and through the length of the shaft 116. In other embodiments, the end effector 114 can be communicatively coupled to the nerve monitoring system 108 using other suitable communication means.

The nerve monitoring system 108 can determine neural locations and activity before therapeutic neuromodulation to determine precise treatment regions corresponding to the positions of the desired nerves, during treatment to determine the effect of the therapeutic neuromodulation, and/or after treatment to evaluate whether the therapeutic neuromodulation treated the target nerves to a desired degree. This information can be used to make various determinations related to the nerves proximate to the target site, such as whether the target site is suitable for neuromodulation. In addition, the nerve monitoring system 108 can also compare the detected neural locations and/or activity before and after therapeutic neuromodulation, and compare the change in neural activity to a predetermined threshold to assess whether the application of therapeutic neuromodulation was effective across the treatment site. For example, the nerve monitoring system 108 can further determine electroneurogram (ENG) signals based on recordings of electrical activity of neurons taken by the end effector 114 before and after therapeutic neuromodulation. Statistically meaningful (e.g., measurable or noticeable) decreases in the ENG signal(s) taken after neuromodulation can serve as an indicator that the nerves were sufficiently ablated. Additional features and functions of the nerve monitoring system 108, as well as other functions of the various components of the console 104, including the evaluation/feedback algorithms 110 for providing real-time feedback capabilities for ensuring optimal therapy for a given treatment is administered, are described in at least U.S. Publication No. 2016/0331459 and U.S. Publication No. 2018/133460.

As will be described in greater detail herein, the neuromodulation device 102 provides access to target sites deep within the nasal region, such as at the immediate entrance of parasympathetic fibers into the nasal cavity to therapeutically modulate autonomic activity within the nasal cavity. In certain embodiments, for example, the neuromodulation device 102 can position the end effector 114 into contact with target sites within nasal cavity associated with postganglionic parasympathetic fibers that innervate the nasal mucosa.

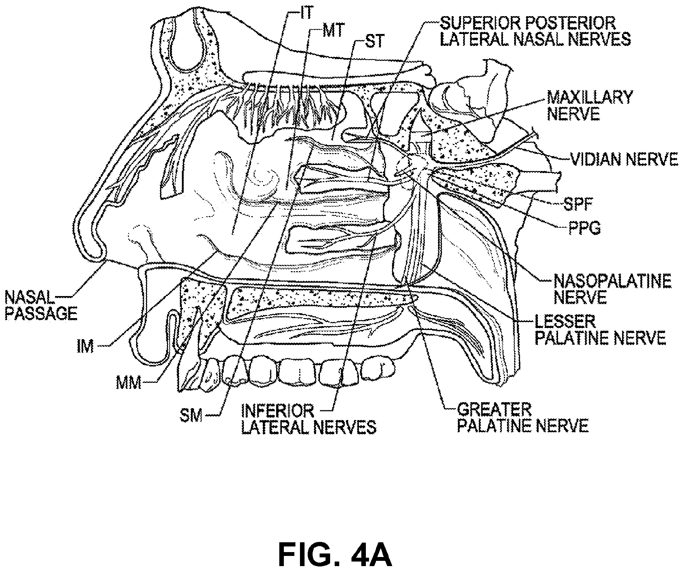

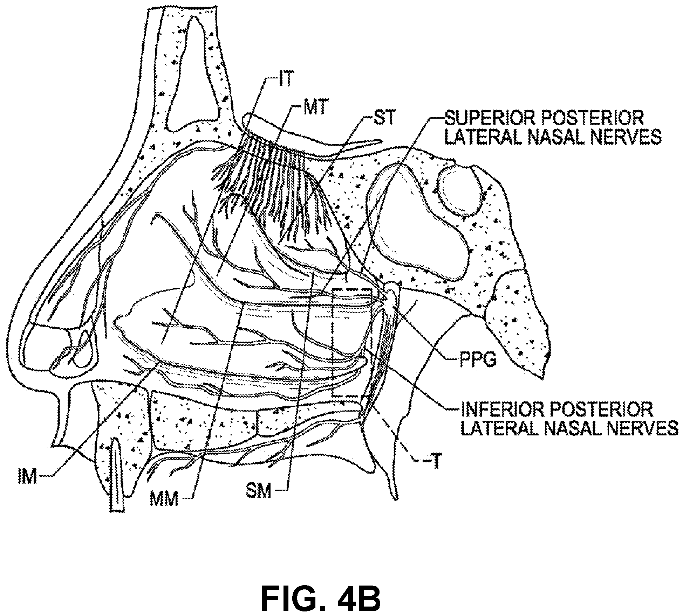

FIG. 4A is a cut-away side view illustrating the anatomy of a lateral nasal wall and FIG. 4B is an enlarged side view of the nerves of the lateral nasal wall of FIG. 3A. The sphenopalatine foramen (SPF) is an opening or conduit defined by the palatine bone and the sphenoid bone through which the sphenopalatine vessels and the posterior superior nasal nerves travel into the nasal cavity. More specifically, the orbital and sphenoidal processes of the perpendicular plate of the palatine bone define the sphenopalatine notch, which is converted into the SPF by the articulation with the surface of the body of the sphenoid bone.

The location of the SPF is highly variable within the posterior region of the lateral nasal cavity, which makes it difficult to visually locate the SPF. Typically, the SPF is located in the middle meatus (MM). However, anatomical variations also result in the SPF being located in the superior meatus (SM) or at the transition of the superior and middle meatuses. In certain individuals, for example, the inferior border of the SPF has been measured at about 19 mm above the horizontal plate of the palatine bone (i.e., the nasal sill), which is about 13 mm above the horizontal lamina of the inferior turbinate (IT) and the average distance from the nasal sill to the SPF is about 64.4 mm, resulting in an angle of approach from the nasal sill to the SPA of about 11.4°. However, studies to measure the precise location of the SPF are of limited practical application due to the wide variation of its location.

The anatomical variations of the SPF are expected to correspond to alterations of the autonomic and vascular pathways traversing into the nasal cavity. In general, it is thought that the posterior nasal nerves (also referred to as lateral posterior superior nasal nerves) branch from the pterygopalatine ganglion (PPG), which is also referred to as the sphenopalatine ganglion, through the SPF to enter the lateral nasal wall of the nasal cavity, and the sphenopalatine artery passes from the pterygopalatine fossa through the SPF on the lateral nasal wall. The sphenopalatine artery branches into two main portions: the posterior lateral nasal branch and the posterior septal branch. The main branch of the posterior lateral nasal artery travels inferiorly into the inferior turbinate IT (e.g., between about 1.0 mm and 1.5 mm from the posterior tip of the inferior turbinate IT), while another branch enters the middle turbinate MT and branches anteriorly and posteriorly.

Beyond the SPF, studies have shown that over 30% of human patients have one or more accessory foramen that also carries arteries and nerves into the nasal cavity. The accessory foramen are typically smaller than the SPF and positioned inferior to the SPF. For example, there can be one, two, three or more branches of the posterior nasal artery and posterior nasal nerves that extend through corresponding accessory foramen. The variability in location, size, and quantity associated with the accessory foramen and the associated branching arteries and nerves that travel through the accessory foramen gives rise to a great deal of uncertainty regarding the positions of the vasculature and nerves of the sphenopalatine region. Furthermore, the natural anatomy extending from the SPF often includes deep inferior and/or superior grooves that carry neural and arterial pathways, which make it difficult to locate arterial and neural branches. For example the grooves can extend more than 5 mm long, more than 2 mm wide, and more than 1 mm deep, thereby creating a path significant enough to carry both arteries and nerves. The variations caused by the grooves and the accessory foramen in the sphenopalatine region make locating and accessing the arteries and nerves (positioned posterior to the arteries) extremely difficult for surgeons.

Recent microanatomic dissection of the pterygopalatine fossa (PPF) have further evidenced the highly variable anatomy of the region surrounding the SPF, showing that a multiplicity of efferent rami that project from the pterygopalatine ganglion (PPG) to innervate the orbit and nasal mucosa via numerous groups of small nerve fascicles, rather than an individual postganglionic autonomic nerves (e.g., the posterior nasal nerve). Studies have shown that at least 87% of humans have microforamina and micro rami in the palatine bone.

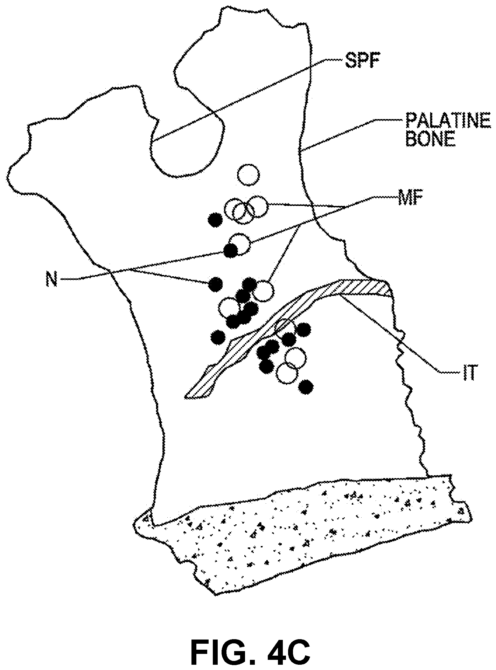

FIG. 4C, for example, is a front view of a left palatine bone illustrating geometry of microforamina and micro rami in a left palatine bone. In FIG. 34, the solid regions represent nerves traversing directly through the palatine bone, and the open circles represent nerves that were associated with distinct microforamina. As such, FIG. 4C illustrates that a medial portion of the palatine bone can include at least 25 accessory posterolateral nerves.

The respiratory portion of the nasal cavity mucosa is composed of a type of ciliated pseudo stratified columnar epithelium with a basement membrane. Nasal secretions (e.g., mucus) are secreted by goblet cells, submucosal glands, and transudate from plasma. Nasal seromucous glands and blood vessels are highly regulated by parasympathetic innervation deriving from the vidian and other nerves. Parasympathetic (cholinergic) stimulation through acetylcholine and vasoactive intestinal peptide generally results in mucus production. Accordingly, the parasympathetic innervation of the mucosa is primarily responsible submucosal gland activation/hyper activation, venous engorgement (e.g., congestion), and increased blood flow to the blood vessels lining the nose. Accordingly, severing or modulating the parasympathetic pathways that innervate the mucosa are expected to reduce or eliminate the hyper activation of the submucosal glands and engorgement of vessels that cause symptoms associated with rhinosinusitis and other indications.

As previously described herein, postganglionic parasympathetic fibers that innervate the nasal mucosa (i.e., posterior superior nasal nerves) were thought to travel exclusively through the SPF as a sphenopalatine neurovascular bundle. The posterior nasal nerves are branches of the maxillary nerve that innervate the nasal cavity via a number of smaller medial and lateral branches extending through the mucosa of the superior and middle turbinates ST, MT (i.e., nasal conchae) and to the nasal septum. The nasopalatine nerve is generally the largest of the medial posterior superior nasal nerves, and it passes anteroinferiorly in a groove on the vomer to the floor of the nasal cavity. From here, the nasopalatine nerve passes through the incisive fossa of the hard palate and communicates with the greater palatine nerve to supply the mucosa of the hard palate. The posterior superior nasal nerves pass through the pterygopalatine ganglion PPG without synapsing and onto the maxillary nerve via its ganglionic branches.

Based on the understanding that the posterior nasal nerves exclusively traverse the SPF to innervate the nasal mucosa, surgeries have been performed to selectively sever the posterior nasal nerve as it exits the SPF. However, as discussed above, the sinonasal parasympathetic pathway actually comprises individual rami project from the pterygopalatine ganglion (PPG) to innervate the nasal mucosa via multiple small nerve fascicles (i.e., accessory posterolateral nerves), not a single branch extending through the SPF. These rami are transmitted through multiple fissures, accessory foramina, and microforamina throughout the palatine bone and may demonstrate anastomotic loops with both the SPF and other accessory nerves. Thus, if only the parasympathetic nerves traversing the SPF were severed, almost all patients (e.g., 90% of patients or more) would retain intact accessory secretomotor fibers to the posterolateral mucosa, which would result in the persistence of symptoms the neurectomy was meant to alieve.

Accordingly, embodiments of the present disclosure are configured to therapeutically modulate nerves at precise and focused treatment sites corresponding to the sites of rami extending through fissures, accessory foramina, and microforamina throughout the palatine bone (e.g., target region T shown in FIG. 4B). In certain embodiments, the targeted nerves are postganglionic parasympathetic nerves that go on to innervate the nasal mucosa. This selective neural treatment is also expected to decrease the rate of postoperative nasal crusting and dryness because it allows a clinician to titrate the degree of anterior denervation through judicious sparing of the rami orbitonasal. Furthermore, embodiments of the present disclosure are also expected to maintain at least some sympathetic tone by preserving a portion of the sympathetic contributions from the deep petrosal nerve and internal maxillary periarterial plexus, leading to improved outcomes with respect to nasal obstruction. In addition, embodiments of the present disclosure are configured to target a multitude of parasympathetic neural entry locations (e.g., accessory foramen, fissures, and microforamina) to the nasal region to provide for a complete resection of all anastomotic loops, thereby reducing the rate of long-term re-innervation.

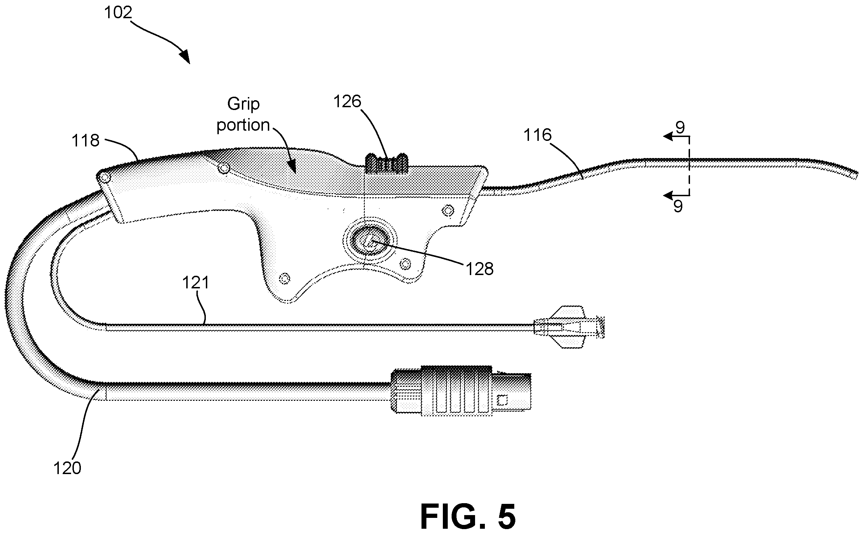

FIG. 5 is a side view of one embodiment of a handheld device 102 for providing therapeutic nasal neuromodulation consistent with the present disclosure. As previously described, the device 102 includes an end effector (not shown) transformable between a collapsed/retracted configuration and an expanded deployed configuration, a shaft 116 operably associated with the end effector, and a handle 118 operably associated with the shaft 116. The handle 118 includes at least a first mechanism 126 for deployment of the end effector from collapsed/retracted configuration to the expanded, deployed configuration, and a second mechanism 128, separate from the first mechanism 124, for control of energy output by the end effector, specifically electrodes or other energy elements provided by the end effector. The handheld device 102 may further include an auxiliary line 121, which may provide a fluid connection between a fluid source, for example, and the shaft 116 such that fluid may be provided to a target site via the distal end of the shaft 116. In some embodiments, the auxiliary line 121 may provide a connection between a vacuum source and the shaft 116, such that the device 102 may include suction capabilities (via the distal end of the shaft 116).

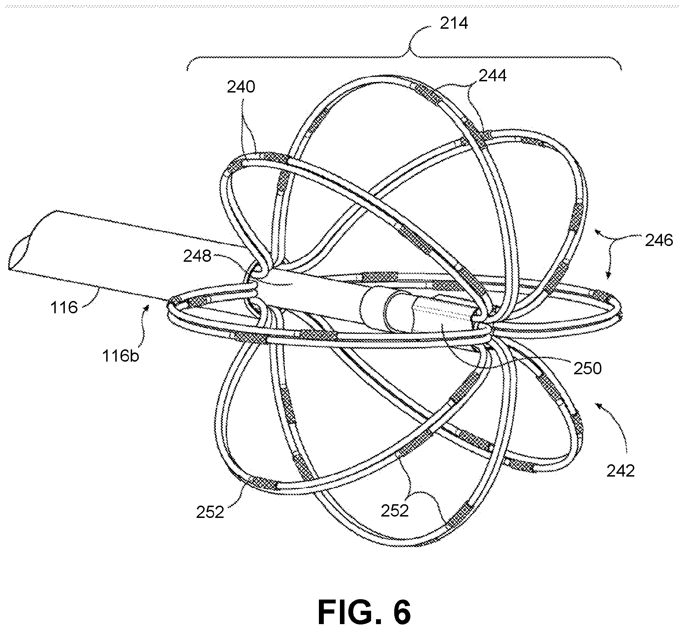

FIG. 6 is an enlarged, perspective view of one embodiment of an end effector 214 consistent with the present disclosure. As shown, the end effector 214 is generally positioned at a distal portion 116b of the shaft 116. The end effector 214 is transformable between a low-profile delivery state to facilitate intraluminal delivery of the end effector 214 to a treatment site within the nasal region and an expanded state, as shown. The end effector 214 includes a plurality of struts 240 that are spaced apart from each other to form a frame or basket 242 when the end effector 214 is in the expanded state. The struts 240 can carry one or more energy delivery elements, such as a plurality of electrodes 244. In the expanded state, the struts 240 can position at least two of the electrodes 244 against tissue at a target site within the nasal region (e.g., proximate to the palatine bone inferior to the SPF). The electrodes 244 can apply bipolar or multi-polar RF energy to the target site to therapeutically modulate postganglionic parasympathetic nerves that innervate the nasal mucosa proximate to the target site. In various embodiments, the electrodes 244 can be configured to apply pulsed RF energy with a desired duty cycle (e.g., 1 second on/0.5 seconds off) to regulate the temperature increase in the target tissue.

In the embodiment illustrated in FIG. 6, the basket 242 includes eight branches 246 spaced radially apart from each other to form at least a generally spherical structure, and each of the branches 246 includes two struts 240 positioned adjacent to each other. In other embodiments, however, the basket 242 can include fewer than eight branches 246 (e.g., two, three, four, five, six, or seven branches) or more than eight branches 246. In further embodiments, each branch 246 of the basket 242 can include a single strut 240, more than two struts 240, and/or the number of struts 240 per branch can vary. In still further embodiments, the branches 246 and struts 240 can form baskets or frames having other suitable shapes for placing the electrodes 244 in contact with tissue at the target site. For example, when in the expanded state, the struts 240 can form an ovoid shape, a hemispherical shape, a cylindrical structure, a pyramid structure, and/or other suitable shapes.

The end effector 214 can further include an internal or interior support member 248 that extends distally from the distal portion 116b of the shaft 116. A distal end portion 250 of the support member 248 can support the distal end portions of the struts 240 to form the desired basket shape. For example, the struts 240 can extend distally from the distal potion 116b of the shaft 116 and the distal end portions of the struts 240 can attach to the distal end portion 250 of the support member 248. In certain embodiments, the support member 248 can include an internal channel (not shown) through which electrical connectors (e.g., wires) coupled to the electrodes 244 and/or other electrical features of the end effector 214 can run. In various embodiments, the internal support member 248 can also carry an electrode (not shown) at the distal end portion 250 and/or along the length of the support member 248.

The basket 242 can transform from the low-profile delivery state to the expanded state (shown in FIG. 6) by either manually manipulating a handle of the device 102, interacting with the first mechanism 126 for deployment of the end effector 214 from collapsed/retracted configuration to the expanded, deployed configuration, and/or other feature at the proximal portion of the shaft 116 and operably coupled to the basket 242. For example, to move the basket 242 from the expanded state to the delivery state, an operator can push the support member 248 distally to bring the struts 240 inward toward the support member 248. An introducer or guide sheath (not shown) can be positioned over the low-profile end effector 214 to facilitate intraluminal delivery or removal of the end effector 214 from or to the target site. In other embodiments, the end effector 214 is transformed between the delivery state and the expanded state using other suitable means, such as the first mechanism 126, as will be described in greater detail herein.

The individual struts 240 can be made from a resilient material, such as a shape-memory material (e.g., Nitinol) that allows the struts 240 to self-expand into the desired shape of the basket 242 when in the expanded state. In other embodiments, the struts 240 can be made from other suitable materials and/or the end effector 214 can be mechanically expanded via a balloon or by proximal movement of the support member 248. The basket 242 and the associated struts 240 can have sufficient rigidity to support the electrodes 244 and position or press the electrodes 244 against tissue at the target site. In addition, the expanded basket 242 can press against surrounding anatomical structures proximate to the target site (e.g., the turbinates, the palatine bone, etc.) and the individual struts 240 can at least partially conform to the shape of the adjacent anatomical structures to anchor the end effector 214 at the treatment site during energy delivery. In addition, the expansion and conformability of the struts 240 can facilitate placing the electrodes 244 in contact with the surrounding tissue at the target site.

At least one electrode 244 is disposed on individual struts 240. In the illustrated embodiment, two electrodes 244 are positioned along the length of each strut 240. In other embodiments, the number of electrodes 244 on individual struts 240 be only one, more than two, zero, and/or the number of electrodes 244 on the different struts 240 can vary. The electrodes 244 can be made from platinum, iridium, gold, silver, stainless steel, platinum-iridium, cobalt chromium, iridium oxide, polyethylenedioxythiophene ("PEDOT"), titanium, titanium nitride, carbon, carbon nanotubes, platinum grey, Drawn Filled Tubing ("DFT") with a silver core made by Fort Wayne Metals of Fort Wayne, Ind., and/or other suitable materials for delivery RF energy to target tissue.

In certain embodiments, each electrode 444 can be operated independently of the other electrodes 244. For example, each electrode can be individually activated and the polarity and amplitude of each electrode can be selected by an operator or a control algorithm (e.g., executed by the controller 107 of FIG. 1A). Various embodiments of such independently controlled electrodes 244 are described in greater detail herein. The selective independent control of the electrodes 244 allows the end effector 214 to deliver RF energy to highly customized regions and to further create multiple micro-lesions to selectively modulate a target neural structure by effectively causing multi-point interruption of a neural signal due to the multiple micro-lesions. For example, a select portion of the electrodes 244 can be activated to target neural fibers in a specific region while the other electrodes 244 remain inactive. In certain embodiments, for example, electrodes 244 may be activated across the portion of the basket 242 that is adjacent to tissue at the target site, and the electrodes 244 that are not proximate to the target tissue can remain inactive to avoid applying energy to non-target tissue. Such configurations facilitate selective therapeutic modulation of nerves on the lateral nasal wall within one nostril without applying energy to structures in other portions of the nasal cavity.

The electrodes 244 can be electrically coupled to an RF generator (e.g., the generator 106 of FIG. 1A) via wires (not shown) that extend from the electrodes 244, through the shaft 116, and to the RF generator. When each of the electrodes 244 is independently controlled, each electrode 244 couples to a corresponding wire that extends through the shaft 116. In other embodiments, multiple electrodes 244 can be controlled together and, therefore, multiple electrodes 244 can be electrically coupled to the same wire extending through the shaft 116. The RF generator and/or components operably coupled (e.g., a control module) thereto can include custom algorithms to control the activation of the electrodes 244. For example, the RF generator can deliver RF power at about 200-300 W to the electrodes 244, and do so while activating the electrodes 244 in a predetermined pattern selected based on the position of the end effector 214 relative to the treatment site and/or the identified locations of the target nerves. In other embodiments, the RF generator delivers power at lower levels (e.g., less than 15 W, 15-50 W, 50-150 W, etc.) and/or higher power levels.

The end effector 214 can further include one or more sensors 252 (e.g., temperature sensors, impedance sensors, etc.) disposed on the struts 240 and/or other portions of the end effector 214 and configured to sense/detect one or more properties associated with neural tissue. For example, temperature sensors are configured to detect the temperature adjacent thereto. The sensors 252 can be electrically coupled to a console (e.g., the console 104 of FIG. 1A) via wires (not shown) that extend through the shaft 116. In various embodiments, the sensors 252 can be positioned proximate to the electrodes 244 to detect various properties of the neural tissue and/or the treatment associated therewith. As will be described in greater detail herein, the sensed data can be provided to the console 104, wherein such data is generally related to at least the presence of neural tissue at a given location (in which electrodes 244 and/or sensors 252 are present at the target site(s)) and depth of identified neural tissue, as well as other physiological, bioelectric, and/or thermal properties. In turn, the console 104 (via the controller 107, monitoring system 108, and evaluation/feedback algorithms 110) is configured to process such data and determine a level of therapeutic energy to be delivered by one or more the plurality of electrodes such that the energy delivered at each position by the one or more electrodes is sufficient to ablate neural tissue at each position and minimize and/or prevent damage to a surrounding or adjacent structure, such as an artery or arterial wall adjacent to the neural tissue at each position.

Such sensed data can further include feedback data associated with the effect of the therapeutic neuromodulation on neural tissue at any given location. For example, feedback data (sensed during therapeutic neuromodulation of neural tissue) may be associated with efficacy of ablation of the neural tissue at each position during and/or after delivery of initial energy from one or more of the plurality of electrodes. Accordingly, in certain embodiments, the console 104 (via the controller 107, monitoring system 108, and evaluation/feedback algorithms 110) is configured to process such feedback data to determine if certain properties of the neural tissue undergoing treatment (i.e., tissue temperature, tissue impedance, etc.) reach predetermined thresholds for irreversible tissue damage. The controller 107 can tune energy output individually for the one or more electrodes after an initial level of energy has been delivered based, at least in part, on feedback data. For example, once the threshold is reached, the application of therapeutic neuromodulation energy can be terminated to allow the tissue to remain intact. In certain embodiments, the energy delivery can automatically be tuned based on an evaluation/feedback algorithm (e.g., the evaluation/feedback algorithm 110 of FIG. 1A) stored on a console (e.g., the console 104 of FIG. 1A) operably coupled to the end effector 214.

FIGS. 7A-7F are various views of another embodiment of an end effector 314 consistent with the present disclosure. As generally illustrated, the end effector 314 is a multi-segmented end effector, which includes at least a first segment 322 and a second segment 324 spaced apart from one another. The first segment 322 is generally positioned closer to a distal portion of the shaft 116, and is thus sometimes referred to herein as the proximal segment 322, while the second segment 324 is generally positioned further from the distal portion of the shaft 116 and is thus sometimes referred to herein as the distal segment 324. Each of the first and second segments 322 and 324 is transformable between a retracted configuration, which includes a low-profile delivery state to facilitate intraluminal delivery of the end effector 314 to a treatment site within the nasal region, and a deployed configuration, which includes an expanded state, as shown in the figures.

FIG. 7A is an enlarged, perspective view of the multi-segment end effector illustrating the first (proximal) segment 322 and second (distal) segment 324. FIG. 7B is an exploded, perspective view of the multi-segment end effector 314. FIG. 7C is an enlarged, top view of the multi-segment end effector 314. FIG. 7D is an enlarged, side view of the multi-segment end effector 314. FIG. 7E is an enlarged, front (proximal facing) view of the first (proximal) segment 322 of the multi-segment end effector 314 and FIG. 7F is an enlarged, front (proximal facing) view of the second (distal) segment 324 of the multi-segment end effector 314.

As illustrated, the first segment 322 includes at least a first set of flexible support elements, generally in the form of wires, arranged in a first configuration, and the second segment 324 includes a second set of flexible support elements, also in the form of wires, arranged in a second configuration. The first and second sets of flexible support elements include composite wires having conductive and elastic properties. For example, in some embodiments, the composite wires include a shape memory material, such as nitinol. The flexible support elements may further include a highly lubricious coating, which may allow for desirable electrical insulation properties as well as desirable low friction surface finish. Each of the first and second segments 322, 324 is transformable between a retracted configuration and an expanded deployed configuration such that the first and second sets of flexible support elements are configured to position one or more electrodes provided on the respective segments (see electrodes 336 in FIGS. 7E and 7F) into contact with one or more target sites when in the deployed configuration.

As shown, when in the expanded deployed configuration, the first set of support elements of the first segment 322 includes at least a first pair of struts 330a, 330b, each comprising a loop (or leaflet) shape and extending in an upward direction and a second pair of struts 332a, 332b, each comprising a loop (or leaflet) shape and extending in a downward direction, generally in an opposite direction relative to at least the first pair of struts 330a, 330b. It should be noted that the terms upward and downward are used to describe the orientation of the first and second segments 322, 324 relative to one another. More specifically, the first pair of struts 330a, 330b generally extend in an outward inclination in a first direction relative to a longitudinal axis of the multi-segment end effector 314 and are spaced apart from one another. Similarly, the second pair of struts 332a, 332b extend in an outward inclination in a second direction substantially opposite the first direction relative to the longitudinal axis of the multi-segment end effector and spaced apart from one another.

The second set of support elements of the second segment 324, when in the expanded deployed configuration, includes a second set of struts 334(1), 334(2), 334(n) (approximately six struts), each comprising a loop shape extending outward to form an open-ended circumferential shape. As shown, the open-ended circumferential shape generally resembles a blooming flower, wherein each looped strut 334 may generally resemble a flower petal. It should be noted that the second set of struts 334 may include any number of individual struts and is not limited to six, as illustrated. For example, in some embodiments, the second segment 124 may include two, three, four, five, six, seven, eight, nine, ten, or more struts 334.

The first and second segments 322, 324, specifically struts 330, 332, and 334 include one or more energy delivery elements, such as a plurality of electrodes 336. It should be noted that any individual strut may include any number of electrodes 336 and is not limited to one electrode, as shown. In the expanded state, the struts 330, 332, and 334 can position any number of electrodes 336 against tissue at a target site within the nasal region (e.g., proximate to the palatine bone inferior to the SPF). The electrodes 336 can apply bipolar or multi-polar radiofrequency (RF) energy to the target site to therapeutically modulate postganglionic parasympathetic nerves that innervate the nasal mucosa proximate to the target site. In various embodiments, the electrodes 336 can be configured to apply pulsed RF energy with a desired duty cycle (e.g., 1 second on/0.5 seconds off) to regulate the temperature increase in the target tissue.

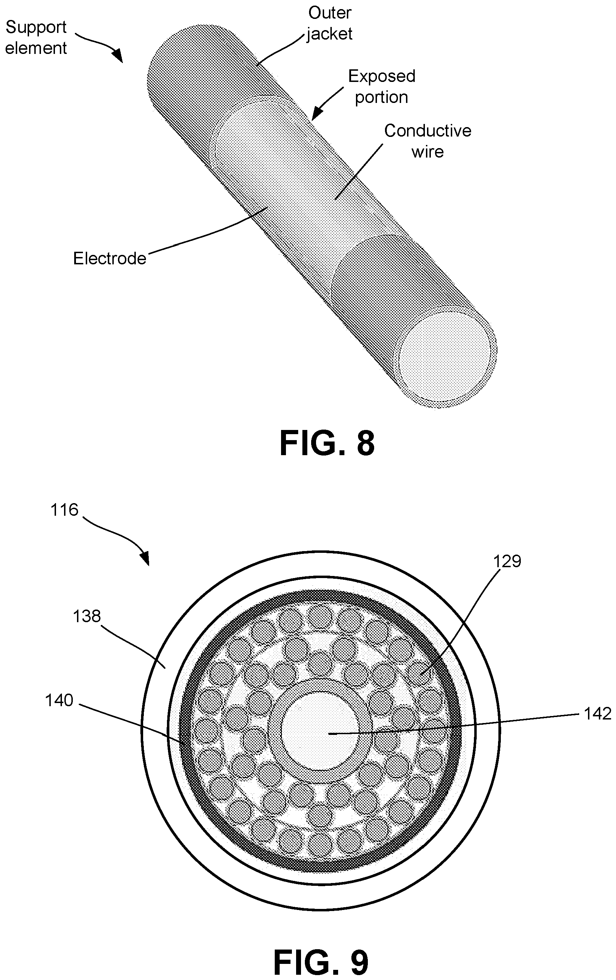

The first and second segments 322, 324 and the associated struts 330, 332, and 334 can have sufficient rigidity to support the electrodes 336 and position or press the electrodes 336 against tissue at the target site. In addition, each of the expanded first and second segments 322, 324 can press against surrounding anatomical structures proximate to the target site (e.g., the turbinates, the palatine bone, etc.) and the individual struts 330, 332, 334 can at least partially conform to the shape of the adjacent anatomical structures to anchor the end effector 314. In addition, the expansion and conformability of the struts 330, 332, 334 can facilitate placing the electrodes 336 in contact with the surrounding tissue at the target site. The electrodes 336 can be made from platinum, iridium, gold, silver, stainless steel, platinum-iridium, cobalt chromium, iridium oxide, polyethylenedioxythiophene (PEDOT), titanium, titanium nitride, carbon, carbon nanotubes, platinum grey, Drawn Filled Tubing (DFT) with a silver core, and/or other suitable materials for delivery RF energy to target tissue. In some embodiments, such as illustrated in FIG. 8, a strut may include an outer jacket surrounding a conductive wire, wherein portions of the outer jacket are selectively absent along a length of the strut, thereby exposing the underlying conductive wire so as to act as an energy delivering element (i.e., an electrode) and/or sensing element, as described in greater detail herein.

In certain embodiments, each electrode 336 can be operated independently of the other electrodes 336. For example, each electrode can be individually activated and the polarity and amplitude of each electrode can be selected by an operator or a control algorithm (e.g., executed by the controller 107 previously described herein). The selective independent control of the electrodes 336 allows the end effector 314 to deliver RF energy to highly customized regions. For example, a select portion of the electrodes 336 can be activated to target neural fibers in a specific region while the other electrodes 336 remain inactive. In certain embodiments, for example, electrodes 136 may be activated across the portion of the second segment 324 that is adjacent to tissue at the target site, and the electrodes 336 that are not proximate to the target tissue can remain inactive to avoid applying energy to non-target tissue. Such configurations facilitate selective therapeutic modulation of nerves on the lateral nasal wall within one nostril without applying energy to structures in other portions of the nasal cavity.

The electrodes 336 are electrically coupled to an RF generator (e.g., the generator 106 of FIG. 1A) via wires (not shown) that extend from the electrodes 336, through the shaft 116, and to the RF generator. When each of the electrodes 336 is independently controlled, each electrode 336 couples to a corresponding wire that extends through the shaft 116. In other embodiments, multiple electrodes 336 can be controlled together and, therefore, multiple electrodes 336 can be electrically coupled to the same wire extending through the shaft 116. As previously described, the RF generator and/or components operably coupled (e.g., a control module) thereto can include custom algorithms to control the activation of the electrodes 336. For example, the RF generator can deliver RF power at about 460-480 kHz (+ or - 5 kHz) to the electrodes 336, and do so while activating the electrodes 336 in a predetermined pattern selected based on the position of the end effector 314 relative to the treatment site and/or the identified locations of the target nerves. It should further be noted that the electrodes 336 may be individually activated and controlled (i.e., controlled level of energy output and delivery) based, at least in part, on feedback data. The RF generator is able to provide bipolar low power (10 watts with maximum setting of 50 watts) RF energy delivery, and further provide multiplexing capabilities (across a maximum of 30 channels).

Once deployed, the first and second segments 322, 324 contact and conform to a shape of the respective locations, including conforming to and complementing shapes of one or more anatomical structures at the respective locations. In turn, the first and second segments 322, 324 become accurately positioned within the nasal cavity to subsequently deliver, via one or more electrodes 336, precise and focused application of RF thermal energy to the one or more target sites to thereby therapeutically modulate associated neural tissue. More specifically, the first and second segments 322, 324 have shapes and sizes when in the expanded configuration that are specifically designed to place portions of the first and second segments 322, 324, and thus one or more electrodes associated therewith 336, into contact with target sites within nasal cavity associated with postganglionic parasympathetic fibers that innervate the nasal mucosa.

For example, the first set of flexible support elements of the first segment 322 conforms to and complements a shape of a first anatomical structure at the first location when the first segment 322 is in the deployed configuration and the second set of flexible support elements of the second segment 124 conforms to and complements a shape of a second anatomical structure at the second location when the second segment is in the deployed configuration. The first and second anatomical structures may include, but are not limited to, inferior turbinate, middle turbinate, superior turbinate, inferior meatus, middle meatus, superior meatus, pterygopalatine region, pterygopalatine fossa, sphenopalatine foramen, accessory sphenopalatine foramen(ae), and sphenopalatine micro-foramen(ae).

In some embodiments, the first segment 322 of the multi-segment end effector 314 is configured in a deployed configuration to fit around at least a portion of a middle turbinate at an anterior position relative to the middle turbinate and the second segment 324 of the multi-segment end effector is configured in a deployed configuration to contact a plurality of tissue locations in a cavity at a posterior position relative to the middle turbinate.

For example, the first set of flexible support elements of the first segment (i.e., struts 330 and 332) conforms to and complements a shape of a lateral attachment and posterior-inferior edge of the middle turbinate when the first segment 322 is in the deployed configuration and the second set of flexible support elements (i.e., struts 334) of the second segment 324 contact a plurality of tissue locations in a cavity at a posterior position relative to the lateral attachment and posterior-inferior edge of middle turbinate when the second segment 324 is in the deployed configuration. Accordingly, when in the deployed configuration, the first and second segments 322, 324 are configured to position one or more associated electrodes 336 at one or more target sites relative to either of the middle turbinate and the plurality of tissue locations in the cavity behind the middle turbinate. In turn, electrodes 336 are configured to deliver RF energy at a level sufficient to therapeutically modulate postganglionic parasympathetic nerves innervating nasal mucosa at an innervation pathway within the nasal cavity of the patient.

As illustrated in FIG. 7E, the first segment 322 comprises a bilateral geometry. In particular, the first segment 322 includes two identical sides, including a first side formed of struts 330a, 332a and a second side formed of struts 330b, 332b. This bilateral geometry allows at least one of the two sides to conform to and accommodate an anatomical structure within the nasal cavity when the first segment 322 is in an expanded state. For example, when in the expanded state, the plurality of struts 330a, 332a contact multiple locations along multiple portions of the anatomical structure and electrodes provided by the struts are configured to emit energy at a level sufficient to create multiple micro-lesions in tissue of the anatomical structure that interrupt neural signals to mucus producing and/or mucosal engorgement elements. In particular, struts 330a, 332a conform to and complement a shape of a lateral attachment and posterior-inferior edge of the middle turbinate when the first segment 322 is in the deployed configuration, thereby allowing for both sides of the anatomical structure to receive energy from the electrodes. By having this independence between first and second side (i.e., right and left side) configurations, the first segment 322 is a true bilateral device. By providing a bilateral geometry, the multi-segment end effector 314 does not require a repeat use configuration to treat the other side of the anatomical structure, as both sides of the structure are accounted at the same time due to the bilateral geometry. The resultant micro-lesion pattern can be repeatable and is predictable in both macro element (depth, volume, shape parameter, surface area) and can be controlled to establish low to high effects of each, as well as micro elements (the thresholding of effects within the range of the macro envelope can be controlled), as well be described in greater detail herein. The systems of the present invention are further able to establish gradients within allowing for control over neural effects without having widespread effect to other cellular bodies, as will be described in greater detail herein.

FIG. 8 is a cross-sectional view of a portion of the shaft 116 of the handheld device taken along lines 9-9 of FIG. 5. As illustrated, the shaft 116 may be constructed from multiple components so as to have the ability to constrain the end effector 314 in the retracted configuration (i.e., the low-profile delivery state) when the end effector 314 is retracted within the shaft 116, and to further provide an atraumatic, low profile and durable means to deliver the end effector 314 to the target site. The shaft 116 includes coaxial tubes which travel from the handle 118 to a distal end of the shaft 116. The shaft 116 assembly is low profile to ensure transnasal delivery of therapy. The shaft 116 includes an outer sheath 138, surrounding a hypotube 140, which is further assembled over electrode wires 129 which surround an inner lumen 142. The outer sheath 138 serves as the interface between the anatomy and the device 102. The outer sheath 138 may generally include a low friction PTFE liner to minimize friction between the outer sheath 138 and the hypotube 140 during deployment and retraction. In particular the outer sheath 138 may generally include an encapsulated braid along a length of the shaft 116 to provide flexibility while retaining kink resistance and further retaining column and/or tensile strength. For example, the outer sheath 138 may include a soft Pebax material, which is atraumatic and enables smooth delivery through the nasal passage.

The hypotube 140 is assembled over the electrode wires starting within the handle 118 and travelling to the proximal end of the end effector 314. The hypotube 140 generally acts to protect the wires during delivery and is malleable to enable flexibility without kinking to thereby improve trackability. The hypotube 140 provides stiffness and enables torqueability of the device 102 to ensure accurate placement of the end effector 314. The hypotube 140 also provides a low friction exterior surface which enables low forces when the outer sheath 138 moves relative to the hypotube 140 during deployment and retraction or constraint. The shaft 116 may be pre-shaped in such a manner so as to complement the nasal cavity. For example, the hypotube 140 may be annealed to create a bent shaft 116 with a pre-set curve. The hypotube 140 may include a stainless-steel tubing, for example, which interfaces with a liner in the outer sheath 138 for low friction movement.

The inner lumen 142 may generally provide a channel for fluid extraction during a treatment procedure. For example, the inner lumen 142 extends from the distal end of the shaft 116 through the hypotube 140 and to atmosphere via a fluid line (line 121 of FIG. 5). The inner lumen 142 materials are chosen to resist forces of external components acting thereon during a procedure.

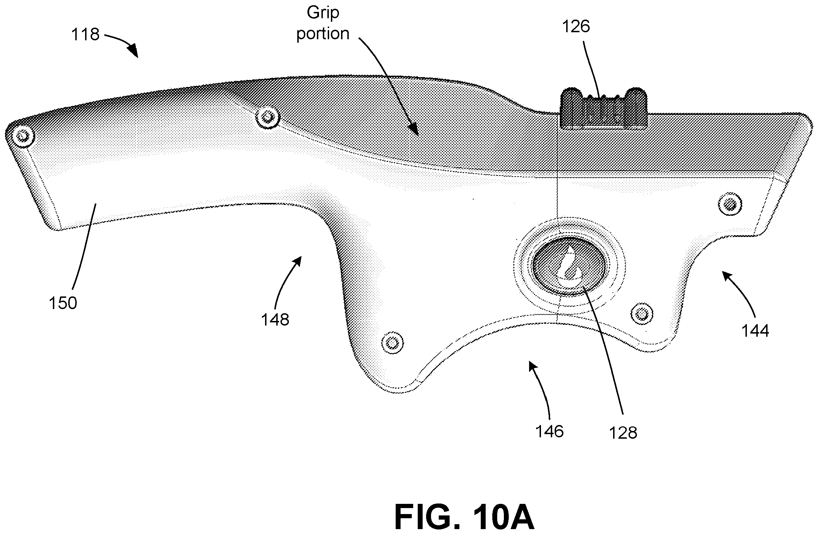

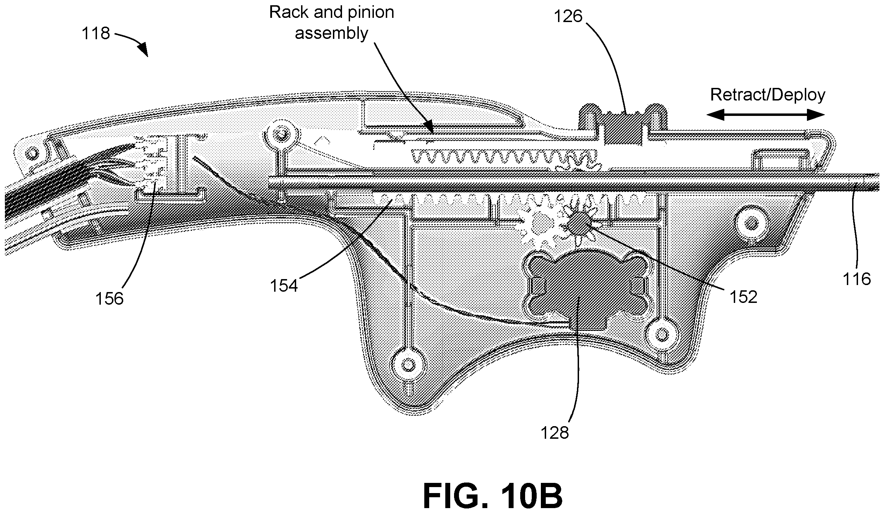

FIG. 10A is a side view of the handle of the handheld 118 and FIG. 10B is a side view of the handle 118 illustrating internal components enclosed within. The handle 118 generally includes an ergonomically-designed grip portion which provides ambidextrous use for both left and right handed use and conforms to hand anthropometrics to allow for at least one of an overhand grip style and an underhand grip style during use in a procedure. For example, the handle 118 may include specific contours, including recesses 144, 146, and 148 which are designed to naturally receive one or more of an operator's fingers in either of an overhand grip or underhand grip style and provide a comfortable feel for the operator. For example, in an underhand grip, recess 144 may naturally receive an operator's index finger, recess 146 may naturally receive an operator's middle finger, and recess 148 may naturally receive an operator's ring and little (pinkie or pinky) fingers which wrap around the proximal protrusion 150 and the operator's thumb naturally rests on a top portion of the handle 118 in a location adjacent to the first mechanism 126. In an overhand grip, the operator's index finger may naturally rest on the top portion of the handle 118, adjacent to the first mechanism 126, while recess 144 may naturally receive the operator's middle finger, recess 146 may naturally receive a portion of the operator's middle and/or ring fingers, and recess 148 may naturally receive and rest within the space (sometimes referred to as the purlicue) between the operator's thumb and index finger.

As previously described, the handle includes multiple user-operated mechanisms, including at least a first mechanism 126 for deployment of the end effector from the collapsed/retracted configuration to the expanded deployed configuration and a second mechanism 128 for controlling of energy output by the end effector, notably energy delivery from one or more electrodes. As shown, the user inputs for the first and second mechanisms 126, 128 are positioned a sufficient distance to one another to allow for simultaneous one-handed operation of both user inputs during a procedure. For example, user input for the first mechanism 126 is positioned on a top portion of the handle 118 adjacent the grip portion and user input for the second mechanism 128 is positioned on side portions of the handle 118 adjacent the grip portion. As such, in an underhand grip style, the operator's thumb rests on the top portion of the handle adjacent to the first mechanism 126 and at least their middle finger is positioned adjacent to the second mechanism 128, each of the first and second mechanisms 126, 128 accessible and able to be actuated. In an overhand grip system, the operator's index finger rests on the top portion of the handle adjacent to the first mechanism 126 and at least their thumb is positioned adjacent to the second mechanism 128, each of the first and second mechanisms 126, 128 accessible and able to be actuated. Accordingly, the handle accommodates various styles of grip and provides a degree of comfort for the surgeon, thereby further improving execution of the procedure and overall outcome.

Referring to FIG. 10B, the various components provided within the handle 118 are illustrated. As shown, the first mechanism 126 may generally include a rack and pinion assembly providing movement of end effector 314 between the retracted and deployed configurations in response to input from a user-operated controller. The rack and pinion assembly generally includes a set of gears 152 for receiving input from the user-operated controller and converting the input to linear motion of a rack member 154 operably associated with at least one of the shaft 116 and the end effector 314. The rack and pinion assembly comprises a gearing ratio sufficient to balance a stroke length and retraction and deployment forces, thereby improving control over the deployment of the end effector. As shown, the rack member 154 may be coupled to a portion of the shaft 116, for example, such that movement of the rack member 154 in a direction towards a proximal end of the handle 118 results in corresponding movement of the shaft 116 while the end effector 314 remains stationary, thereby exposing the end effector 314 and allowing the end effector 314 to transition from the constrained, retracted configuration to the expanded, deployed configuration. Similarly, upon movement of the rack member 154 in a direction towards a distal end of the handle 118 results in corresponding movement of the shaft 116 while the end effector 314 remains stationary, thereby enclosing the end effector 314 within the shaft 116. It should be noted that, in other embodiments, the rack member 154 may be directly coupled to a portion of the end effector 314 such that movement of the rack member 154 results in corresponding movement of the end effector 314 while the shaft 116 remains stationary, thereby transitioning the end effector 314 between the retracted and deployed configurations.

The user-operated controller associated with the first mechanism 126 may include a slider mechanism operably associated with the rack and pinion rail assembly. Movement of the slider mechanism in a rearward direction towards a proximal end of the handle results in transitioning of the end effector 314 to the deployed configuration and movement of the slider mechanism in a forward direction towards a distal end of the handle results in transitioning of the end effector to the retracted configuration. In other embodiment, the user-operated controller associated with the first mechanism 126 may include a scroll wheel mechanism operably associated with the rack and pinion rail assembly. Rotation of the wheel in a rearward direction towards a proximal end of the handle results in transitioning of the end effector to the deployed configuration and rotation of the wheel in a forward direction towards a distal end of the handle results in transitioning of the end effector to the retracted configuration.

The user-operated controller associated with the first mechanism 126 may generally provide a high degree of precision and control over the deployment (and retraction) of the first and second segments 322, 324. For example, in some instances, the operator may wish to only deploy the second segment 324 during the procedure, while the first segment 322 remains in the retracted configuration. The user-operated controller allows for an operator to provide a sufficient degree of input (i.e., slide the slider mechanism or scroll the scroll wheel to a specific position) which results in only the second segment 324 transitioning from the retracted configuration to the deployed configuration (while the first segment 322 remains enclosed within the shaft 116 and in the retracted configuration). For example, in some embodiments, the end effector 314 may further include a detent feature, such as a catch or similar element, positioned between the first and second segments 322, 324 and configured to provide a surgeon with feedback, such as tactile feedback, during deployment of the end effector segments, alerting the surgeon when at least the second segment 324 is fully deployed. In particular, as the surgeon slides the slider mechanism or scrolls the scroll wheel during deployment of the second segment 324, the detect feature (provided between the first and second segments 322, 324) may then reach a portion of the shaft 116 and cause an increase in resistance on the slider mechanism or scroll wheel, thereby indicating to the surgeon that the second segment 324 has been deployed and the first segment 322 remains in the retracted configuration. Accordingly, the surgeon can position and orient the second segment 324 as they desire without concern over the first segment 322 as it remains in the retracted configuration. In turn, one the second segment 324 is positioned at the desired target site, the surgeon may then deploy the first segment 322 to perform the procedure. Yet still, in some instances, only the second segment 324 may be used to perform a procedure (i.e., deliver energy to one or more target sites in contact with the second segment 324) and, as such, the first segment 322 may never be deployed.

The second mechanism 128 may generally include a user-operated controller configured to be actuated between at least an active position and an inactive position to thereby control delivery of energy from the end effector, notable delivery of energy from the electrodes. The user-operated controller may be multi-modal in that the user-operated controller may be actuated between multiple positions providing different functions/modes. For example, upon a single user input (i.e., single press of button associated within controller), the second mechanism may provide a baseline apposition / sensing check function prior to modulation. Upon pressing and holding the controller button for a pre-defined period of time, the energy output from the end effector may be activated. Further, upon double-tapping the controller button, energy output is deactivated.

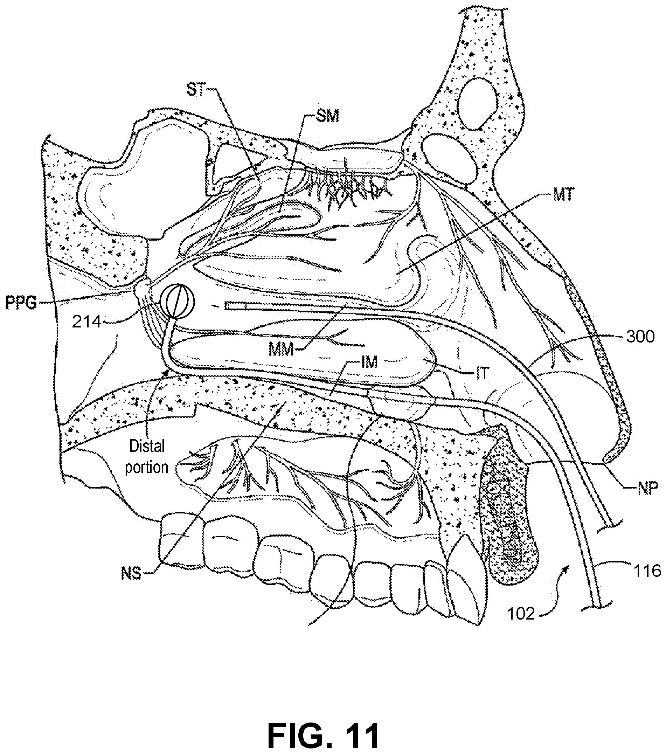

FIG. 11 is a partial cut-away side view illustrating one approach for delivering an end effector a target site within a nasal region in accordance with embodiments of the present disclosure. As shown, the end effector 214 is positioned within a nasal region. However, it should be noted that the following description related to the delivery and deployment of the end effector 214 also applies to end effector 314. The distal portion of the shaft 116 extends into the nasal passage NP, through the inferior meatus IM between the inferior turbinate IT and the nasal sill NS, and around the posterior portion of the inferior turbinate IT where the end effector 214 is deployed at a treatment site. The treatment site can be located proximate to the access point or points of postganglionic parasympathetic nerves (e.g., branches of the posterior nasal nerve and/or other parasympathetic neural fibers that innervate the nasal mucosa) into the nasal cavity. In other embodiments, the target site can be elsewhere within the nasal cavity depending on the location of the target nerves.

In various embodiments, the distal portion of the shaft 116 may be guided into position at the target site via a guidewire (not shown) using an over-the-wire (OTW) or a rapid exchange (RX) technique. For example, the end effector 214 can include a channel for engaging the guidewire. Intraluminal delivery of the end effector 214 can include inserting the guide wire into an orifice in communication with the nasal cavity (e.g., the nasal passage or mouth), and moving the shaft 116 and/or the end effector 214 along the guide wire until the end effector 214 reaches a target site (e.g., inferior to the SPF).

Yet still, in further embodiments, the neuromodulation device 102 can be configured for delivery via a guide catheter or introducer sheath (not shown) with or without using a guide wire. The introducer sheath can first be inserted intraluminally to the target site in the nasal region, and the distal portion of the shaft 116 can then be inserted through the introducer sheath. At the target site, the end effector 214 can be deployed through a distal end opening of the introducer sheath or a side port of the introducer sheath. In certain embodiments, the introducer sheath can include a straight portion and a pre-shaped portion with a fixed curve (e.g., a 5 mm curve, a 4 mm curve, a 3 mm curve, etc.) that can be deployed intraluminally to access the target site. In this embodiment, the introducer sheath may have a side port proximal to or along the pre-shaped curved portion through which the end effector 214 can be deployed. In other embodiments, the introducer sheath may be made from a rigid material, such as a metal material coated with an insulative or dielectric material. In this embodiment, the introducer sheath may be substantially straight and used to deliver the end effector 214 to the target site via a substantially straight pathway, such as through the middle meatus MM (FIG. 4A).

Image guidance may be used to aid the surgeon's positioning and manipulation of the distal portion of the shaft 116, as well as the deployment and manipulation of the end effector 214. For example, an endoscope 100 and/or other visualization device can be positioned to visualize the target site, the positioning of the end effector 214 at the target site, and/or the end effector 214 during therapeutic neuromodulation. The endoscope 100 may be delivered proximate to the target site by extending through the nasal passage NP and through the middle meatus MM between the inferior and middle turbinates IT and MT. From the visualization location within the middle meatus MM, the endoscope 100 can be used to visualize the treatment site, surrounding regions of the nasal anatomy, and the end effector 214.

In some embodiments, the distal portion of the shaft 116 may be delivered via a working channel extending through an endoscope, and therefore the endoscope can provide direct in-line visualization of the target site and the end effector 214. In other embodiments, an endoscope is incorporated with the end effector 114 and/or the distal portion of the shaft 116 to provide in-line visualization of the end effector 114 and/or the surrounding nasal anatomy. In other embodiments, image guidance can be provided with various other guidance modalities, such as image filtering in the infrared (IR) spectrum to visualize the vasculature and/or other anatomical structures, computed tomography (CT), fluoroscopy, ultrasound, optical coherence tomography (OCT), and/or combinations thereof. Yet still, in some embodiments, image guidance components may be integrated with the neuromodulation device 102 to provide image guidance during positioning of the end effector 214.

Once positioned at the target site, the therapeutic modulation may be applied via the one or more electrodes 244 and/or other features of the end effector 214 to precise, localized regions of tissue to induce one or more desired therapeutic neuromodulating effects to disrupt parasympathetic motor sensory function. The end effector 214 can selectively target postganglionic parasympathetic fibers that innervate the nasal mucosa at a target or treatment site proximate to or at their entrance into the nasal region. For example, the end effector 214 can be positioned to apply therapeutic neuromodulation at least proximate to the SPF (FIG. 4A) to therapeutically modulate nerves entering the nasal region via the SPF. The end effector 214 can also be positioned to inferior to the SPF to apply therapeutic neuromodulation energy across accessory foramen and microforamina (e.g., in the palatine bone) through which smaller medial and lateral branches of the posterior superior lateral nasal nerve enter the nasal region. The purposeful application of the energy at the target site may achieve therapeutic neuromodulation along all or at least a portion of posterior nasal neural fibers entering the nasal region. The therapeutic neuromodulating effects are generally a function of, at least in part, power, time, and contact between the energy delivery elements and the adjacent tissue. For example, in certain embodiments therapeutic neuromodulation of autonomic neural fibers are produced by applying RF energy at a power of about 2-20 W (e.g., 5 W, 7 W, 10 W, etc.) for a time period of about 1-20 sections (e.g., 5-10 seconds, 8-10 seconds, 10-12 seconds, etc.).

The therapeutic neuromodulating effects may include partial or complete denervation via thermal ablation and/or non-ablative thermal alteration or damage (e.g., via sustained heating and/or resistive heating). Desired thermal heating effects may include raising the temperature of target neural fibers above a desired threshold to achieve non-ablative thermal alteration, or above a higher temperature to achieve ablative thermal alteration. For example, the target temperature may be above body temperature (e.g., approximately 37° C.) but less than about 90° C. (e.g., 70-75° C.) for non-ablative thermal alteration, or the target temperature may be about 100° C. or higher (e.g., 110° C., 120° C., etc.) for the ablative thermal alteration. Desired non-thermal neuromodulation effects may include altering the electrical signals transmitted in a nerve.

Sufficiently modulating at least a portion of the parasympathetic nerves is expected to slow or potentially block conduction of autonomic neural signals to the nasal mucosa to produce a prolonged or permanent reduction in nasal parasympathetic activity. This is expected to reduce or eliminate activation or hyperactivation of the submucosal glands and venous engorgement and, thereby, reduce or eliminate the symptoms of rhinosinusitis. Further, because the device 102 applies therapeutic neuromodulation to the multitude of branches of the posterior nasal nerves rather than a single large branch of the posterior nasal nerve branch entering the nasal cavity at the SPF, the device 102 provides a more complete disruption of the parasympathetic neural pathway that affects the nasal mucosa and results in rhinosinusitis. Accordingly, the device 102 is expected to have enhanced therapeutic effects for the treatment of rhinosinusitis and reduced re-innervation of the treated mucosa.

In other embodiments, the device 102 can be configured to therapeutically modulate nerves and/or other structures to treat different indications. For example, the device 102 can be used to therapeutically modulate nerves that innervate the para-nasal sinuses to treat chronic sinusitis. In further embodiments, the system 100 and the device 102 disclosed herein can be configured therapeutically modulate the vasculature within the nasal anatomy to treat other indications, such as epistaxis (i.e., excessive bleeding from the nose). For example, the system 100 and the device 102 devices described herein can be used to apply therapeutically effective energy to arteries (e.g., the sphenopalatine artery and its branches) as they enter the nasal cavity (e.g., via the SPF, accessory foramen, etc.) to partially or completely coagulate or ligate the arteries. In other embodiments, the system 100 and the device 102 can be configured to partially or completely coagulate or ligate veins and/or other vessels. For such embodiments in which the end effector ligates or coagulates the vasculature, the system 100 and device 102 would be modified to deliver energy at significantly higher power (e.g., about 100 W) and/or longer times (e.g., 1 minute or longer) than would be required for therapeutic neuromodulation.

As previously described, the system 100 of the present invention provides for the targeting neural structures in a nasal region of a patient for the treatment of a rhinosinusitis condition while avoiding collateral damage to surrounding structures, such as surrounding tissue, blood vessels, as well as other neural structures.

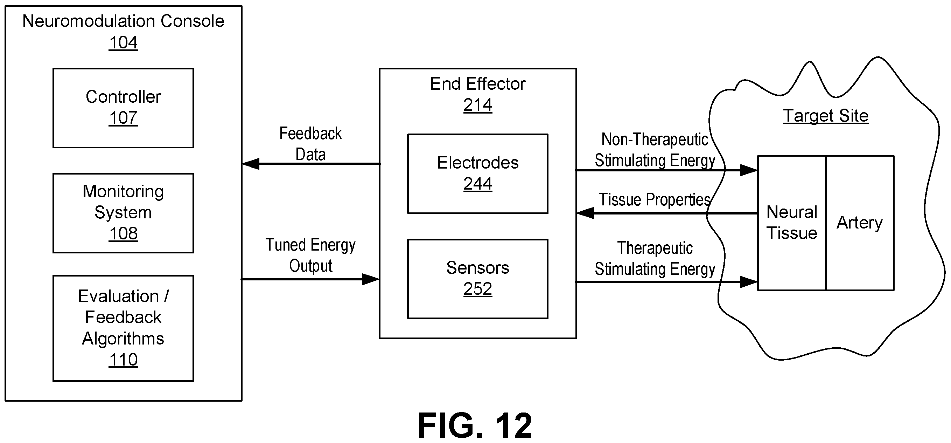

FIG. 12 is a block diagram illustrating the process of sensing, via an end effector, data associated with one or more target sites, notably the presence of neural tissue at the one more target sites, as well as other properties, including, for example, depth of neural tissue at multiple locations. FIG. 12 further illustrates the console processing such data (via the controller 107, monitoring system 108, and evaluation/feedback algorithms 110) and determining a level of therapeutic energy to be delivered by one or more of a plurality of electrodes of the end effector, wherein the energy delivered at each position by the one or more electrodes is sufficient to ablate neural tissue at each position and minimize and/or prevent damage to a surrounding or adjacent structure, such as an artery or arterial wall adjacent to the neural tissue at each position.

It should be noted that, while the block diagram of FIG. 12 includes reference to end effector 214, other end effector embodiments, including end effector 314, are similarly configured with respect to sensing data associated with at least the presence of neural tissue and other properties of the neural tissue, including neural tissue depth. Accordingly, the following process is not limited to end effector 214.

As previously described, the handheld treatment device includes an end effector comprising a micro-electrode array arranged about a plurality of struts. For example, end effector 214 includes a plurality of struts 240 that are spaced apart from each other to form a frame or basket 242 when the end effector 214 is in the expanded state. The struts 240 include a plurality of energy delivery elements, such as a plurality of electrodes 244. In the expanded state, each of the plurality of struts is able to conform to and accommodate an anatomical structure within the nasal cavity. When positioned within the nasal cavity, the struts contact multiple locations along multiple portions of a target site and thereby position one or more electrodes 244 against tissue at a target site within the nasal region (e.g., proximate to the palatine bone inferior to the SPF). At least a subset of electrodes is configured to sense the presence of neural tissue at a respective position of each of the electrodes, as well as a depth of the neural tissue, and further convey such data to the console 104.

For example, in one embodiment, a subset of the plurality of electrodes is configured to deliver non-therapeutic stimulating energy to respective positions within the nasal cavity at a frequency for locating neural tissue and further sense one or more properties, including at least the presence and/or depth of the neural tissue in response to the non-therapeutic stimulating energy. The properties may further include, but are not limited to, at least one of physiological properties, bioelectric properties, and thermal properties of the neural tissue, as well as other structures adjacent to the neural tissue.

For example, upon delivering non-therapeutic stimulating energy (via one or more electrodes 244) to respective positions within the nasal cavity, various properties of the tissue at the one or more target sites can be detected. This information can then be transmitted to the console 104, particularly the controller 107, monitoring system 108, and evaluation/feedback algorithms 110 to determine the anatomy at the target site (e.g., tissue types, tissue locations, vasculature, bone structures, foramen, sinuses, etc.), locate neural tissue, differentiate between different types of neural tissue, and map the anatomical and/or neural structure at the target site. For example, the end effector 214 can be used to detect resistance, complex electrical impedance, dielectric properties, temperature, and/or other properties that indicate the presence of neural fibers and/or other anatomical structures in the target region. In certain embodiments, the end effector 214, together with the console 104 components, can be used to determine resistance (rather than impedance) of the tissue (i.e., the load) to more accurately identify the characteristics of the tissue. For example, the evaluation/feedback algorithms 110 can determine resistance of the tissue by detecting the actual power and current of the load (e.g., via the electrodes 244).

In some embodiments, the system 100 provides resistance measurements with a high degree of accuracy and a very high degree of precision, such as precision measurements to the hundredths of an Ohm (e.g., 0.01Ω) for the range of 1-50Ω. The high degree of resistance detection accuracy provided by the system 100 allows for the detection sub-microscale structures, including the firing of neural tissue, differences between neural tissue and other anatomical structures (e.g., blood vessels), and even different types of neural tissue. This information can be analyzed by the evaluation/feedback algorithms 110 and/or the controller 107 and communicated to the operator via a high resolution spatial grid (e.g., on the display 112) and/or other type of display to identify neural tissue and other anatomy at the treatment site and/or indicate predicted neuromodulation regions based on the ablation pattern with respect to the mapped anatomy.

As previously described, in certain embodiments, each electrode 244 can be operated independently of the other electrodes 244. For example, each electrode can be individually activated and the polarity and amplitude of each electrode can be selected by an operator or a control algorithm executed by the controller 107. The selective independent control of the electrodes 244 allows the end effector 214 to detect information (i.e., the presence of neural tissue, depth of neural tissue, and other properties) and subsequently deliver RF energy to highly customized regions. For example, a select portion of the electrodes 244 can be activated to target specific neural fibers in a specific region while the other electrodes 244 remain inactive. In addition, the electrodes 244 can be individually activated to stimulate or therapeutically modulate certain regions in a specific pattern at different times (e.g., via multiplexing), which facilitates detection of anatomical parameters across a zone of interest and/or regulated therapeutic neuromodulation.

In certain embodiments, the system 100 can determine the locations and/or morphology of neural tissue and/or other anatomical structures before therapy such that the therapeutic neuromodulation can be applied to precise regions including target neural tissue, while avoiding negative effects on non-target structures, such as blood vessels. The system 100 can detect various bioelectrical parameters in an interest zone (e.g., within in the nasal cavity) to determine the location and morphology of various neural tissue (e.g., different types of neural tissue, neuronal directionality, etc.) and/or other tissue (e.g., glandular structures, vessels, bony regions, etc.). In some embodiments, the system 100 is configured to measure bioelectric potential.

To do so, one or more of the electrodes 244 is placed in contact with an epithelial surface at a region of interest (e.g., a treatment site). Electrical stimuli (e.g., constant or pulsed currents at one or more frequencies) are applied to the tissue by one or more electrodes 244 at or near the treatment site, and the voltage and/or current differences at various different frequencies between various pairs of electrodes 244 of the end effector 214 may be measured to produce a spectral profile or map of the detected bioelectric potential, which can be used to identify different types of tissues (e.g., vessels, neural tissue, and/or other types of tissue) in the region of interest. For example, current (i.e., direct or alternating current) can be applied to a pair of electrodes 244 adjacent to each other and the resultant voltages and/or currents between other pairs of adjacent electrodes 244 are measured. It will be appreciated that the current injection electrodes 244 and measurement electrodes 244 need not be adjacent, and that modifying the spacing between the two current injection electrodes 244 can affect the depth of the recorded signals. For example, closely-spaced current injection electrodes 244 provided recorded signals associated with tissue deeper from the surface of the tissue than further spaced apart current injection electrodes 244 that provide recorded signals associated with tissue at shallower depths. Recordings from electrode pairs with different spacings may be merged to provide additional information on depth and localization of anatomical structures.

Further, complex impedance and/or resistance measurements of the tissue at the region of interest can be detected directly from current-voltage data provided by the bioelectric potential measurements while differing levels of frequency currents are applied to the tissue (e.g., via the end effector 114), and this information can be used to map the neural and anatomical structures by the use of frequency differentiation reconstruction. Applying the stimuli at different frequencies will target different stratified layers or cellular bodies or clusters. At high signal frequencies (e.g., electrical injection or stimulation), for example, cell membranes of the neural tissue do not impede current flow, and the current passes directly through the cell membranes. In this case, the resultant measurement (e.g., impedance, resistance, capacitance, and/or induction) is a function of the intracellular and extracellular tissue and liquids. At low signal frequencies, the membranes impede current flow to provide different defining characteristics of the tissues, such as the shapes of the cells or cell spacing. The stimulation frequencies can be in the megahertz range, in the kilohertz range (e.g., 400-500 kHz, 450-480 kHz, etc.), and/or other frequencies attuned to the tissue being stimulated and the characteristics of the device being used. The detected complex impedance or resistances levels from the zone of interest can be displayed to the user (e.g., via the display 112) to visualize certain structures based on the stimulus frequency.

Further, the inherent morphology and composition of the anatomical structures in the nasal region react differently to different frequencies and, therefore, specific frequencies can be selected to identify very specific structures. For example, the morphology or composition of targeted structures for anatomical mapping may depend on whether the cells of tissue or other structure are membranonic, stratified, and/or annular. In various embodiments, the applied stimulation signals can have predetermined frequencies attuned to specific neural tissue, such as the level of myelination and/or morphology of the myelination. For example, second axonal parasympathetic structures are poorly myelinated than sympathetic nerves or other structures and, therefore, will have a distinguishable response (e.g., complex impedance, resistance, etc.) with respect to a selected frequency than sympathetic nerves. Accordingly, applying signals with different frequencies to the target site can distinguish the targeted parasympathetic nerves from the non-targeted sensory nerves, and therefore provide highly specific target sites for neural mapping before or after therapy and/or neural evaluation post-therapy.

In some embodiments, the neural and/or anatomical mapping includes measuring data at a region of interest with at least two different frequencies to identify certain anatomical structures such that the measurements are taken first based on a response to an injection signal having a first frequency and then again based on an injection signal having a second frequency different from the first. For example, there are two frequencies at which hypertrophied (i.e., disease-state characteristics) sub-mucosal targets have a different electrical conductivity or permittivity compared to "normal" (i.e., healthy) tissue. Complex conductivity may be determined based on one or more measured physiological parameters (e.g., complex impedance, resistance, dielectric measurements, dipole measurements, etc.) and/or observance of one or more confidently known attributes or signatures. Furthermore, the system 100 can also apply neuromodulation energy via the electrodes 244 at one or more predetermined frequencies attuned to a target neural structure to provide highly targeted ablation of the selected neural structure associated with the frequency(ies). This highly targeted neuromodulation also reduces the collateral effects of neuromodulation therapy to non-target sites/structures (e.g., blood vessels) because the targeted signal (having a frequency tuned to a target neural structure) will not have the same modulating effects on the non-target structures.

Accordingly, bioelectric properties, such as complex impedance and resistance, can be used by the system 100 before, during, and/or after neuromodulation therapy to guide one or more treatment parameters. For example, before, during, and/or after treatment, impedance or resistance measurements may be used to confirm and/or detect contact between one or more electrodes 244 and the adjacent tissue. The impedance or resistance measurements can also be used to detect whether the electrodes 244 are placed appropriately with respect to the targeted tissue type by determining whether the recorded spectra have a shape consistent with the expected tissue types and/or whether serially collected spectra were reproducible. In some embodiments, impedance or resistance measurements may be used to identify a boundary for the treatment zone (e.g., specific neural tissue that are to be disrupted), anatomical landmarks, anatomical structures to avoid (e.g., vascular structures or neural tissue that should not be disrupted), and other aspects of delivering energy to tissue.

The bioelectric information can be used to produce a spectral profile or map of the different anatomical features tissues at the target site, and the anatomical mapping can be visualized in a 3D or 2D image via the display 112 and/or other user interface to guide the selection of a suitable treatment site. This neural and anatomical mapping allows the system 100 to accurately detect and therapeutically modulate the postganglionic parasympathetic neural fibers that innervate the mucosa at the numerous neural entrance points into the nasal cavity. Further, because there are not any clear anatomical markers denoting the location of the SPF, accessory foramen, and microforamina, the neural mapping allows the operator to identify and therapeutically modulate nerves that would otherwise be unidentifiable without intricate dissection of the mucosa. In addition, anatomical mapping also allows the clinician to identify certain structures that the clinician may wish to avoid during therapeutic neural modulation (e.g., certain arteries). The neural and anatomical bioelectric properties detected by the system 100 can also be used during and after treatment to determine the real-time effect of the therapeutic neuromodulation on the treatment site. For example, the evaluation/feedback algorithms 110 can also compare the detected neural locations and/or activity before and after therapeutic neuromodulation, and compare the change in neural activity to a predetermined threshold to assess whether the application of therapeutic neuromodulation was effective across the treatment site.

The end effector 214 communicates the tissue data (i.e., detection of neural tissue, as well as the depth of neural tissue at respective locations at the target site(s)) to the console 104. In turn, console 104 (via the controller 107, monitoring system 108, and evaluation/feedback algorithms 110) is configured to process such data and determine a level of therapeutic energy to be delivered by one or more the plurality of electrodes 244 such that the energy delivered at each position by the one or more electrodes is sufficient to ablate neural tissue at each position and minimize and/or prevent damage to a surrounding or adjacent structure, such as an artery or arterial wall adjacent to the neural tissue at each position.

Such sensed data from the end effector 214 can further include feedback data associated with the effect of the therapeutic neuromodulation on neural tissue at any given location. For example, feedback data (sensed during therapeutic neuromodulation of neural tissue) may be associated with efficacy of ablation of the neural tissue at each position during and/or after delivery of initial energy from one or more of the plurality of electrodes 244. Accordingly, in certain embodiments, the console 104 (via the controller 107, monitoring system 108, and evaluation/feedback algorithms 110) is configured to process such feedback data to determine if certain properties of the neural tissue undergoing treatment (i.e., tissue temperature, tissue impedance, etc.) reach predetermined thresholds for irreversible tissue damage.

The electrodes 244 are configured to be independently controlled and activated by the controller 107 (in conjunction with the evaluation/feedback algorithms 110) to thereby deliver energy independent of one another. Accordingly, the controller 107 can tune energy output individually for the one or more electrodes 244 after an initial level of energy has been delivered based, at least in part, on feedback data. For example, once the threshold is reached, the application of therapeutic neuromodulation energy can be terminated to allow the tissue to remain intact. In other embodiments, if the threshold has not been reached, the controller can maintain, reduce, or increase energy output to a given electrode 244 until such threshold is reached. Accordingly, the energy delivery of any given electrode 244 can automatically be tuned based on an evaluation/feedback algorithm (e.g., the evaluation/feedback algorithm 110 of FIG. 1A) stored on a console (e.g., the console 104 of FIG. 1A) operably coupled to the end effector 214. For example, at least some of the electrodes 244 may have different levels of energy to be delivered at respective positions sufficient to ablate neural tissue at the respective positions based on the feedback data received for the respective locations.

For example, in some embodiments, the controller 107 is configured to tune energy output from each of the plurality of electrodes 244 after an initial level of energy has been delivered based, at least in part, on feedback data received. The feedback data may be associated with efficacy of ablation of the neural tissue at each position during and/or after delivery of initial energy from each of the plurality of electrodes. The feedback data includes one or more properties associated with neural tissue at respective positions within the nasal cavity. The one or more properties may include, but are not limited to, physiological properties, bioelectric properties, and thermal properties. For example, the bioelectric properties may include, but are not limited to, complex impedance, resistance, reactance, capacitance, inductance, permittivity, conductivity, nerve firing voltage, nerve firing current, depolarization, hyperpolarization, magnetic field, and induced electromotive force.

Accordingly, the neuromodulation energy can be applied to the tissue in a highly targeted manner and form micro-lesions to selectively modulate the target structure, while avoiding non-targeted structures (e.g., other neural tissue, blood vessels, etc.) and allowing the surrounding tissue structure to remain healthy for effective wound healing. In that manner, the present invention provides a treatment device that is capable of highly conforming to anatomical variations within a nasal cavity and further provides a controller providing unprecedented control to an operator so that an operator can perform an accurate, minimally invasive, and localized application of energy to target sites within the nasal cavity to cause multi-point interruption of neural signal without causing collateral damage or disruption to non-neural tissue (e.g., blood vessels, bone, muscle, etc.) or non-targeted neural tissue. More specifically, based on feedback data from the treatment device, the controller allows for the tuning of micro-lesion depths at a given position to accommodate variations in neural tissue, including depth of neural tissue as well as other physiological, bioelectric, and/or thermal properties, which is critical in preventing excessive damage and destruction to surrounding structures during a treatment procedure. For example, the depth of neural tissue can vary greatly depending on the region and position within the nasal cavity. A depth of neural tissue within a turbinate location can be six times greater than a non-turbinate location.

Therefore, the system of the present invention provides a user-friendly, non-invasive means of treating rhinosinusitis conditions, including precise and focused application of energy to the intended target sites for therapeutic modulation of intended neural tissue, specifically the creation of multiple micro-lesion resulting in multi-point interruption of neural signals, without causing collateral damage or disruption to non-neural tissue (e.g., blood vessels, etc.) or non-targeted neural tissue. The creation of micro-lesions along a neural tissue is critical for various reasons. The creation of multiple micro-lesions, and resulting multi-point interruption, accounts for the various entry points and pathways associated with certain nerves, including the microforamina. The multiple micro-lesions further creates more than a single break along a nerve pathway, which is vital for longevity of the effect of treatment as it prevents functional alignment and regeneration of the nerve tissue. Furthermore, the controller is able to tailor the size and shape of any given micro-lesion at a respective location along a neural tissue by way of independent control of electrodes of the electrode array, thereby further enhancing the effectiveness of treatment by accounting for variations in neural tissue depth, for example, at any given location. The creation of multiple micro-lesions is much more effective than a single, continuous lesion, which presents further potential risks, such as an acute and latent risk of hemorrhage, which carries a high mortality risk due to the arteries and blood supply present in the nasal cavity.

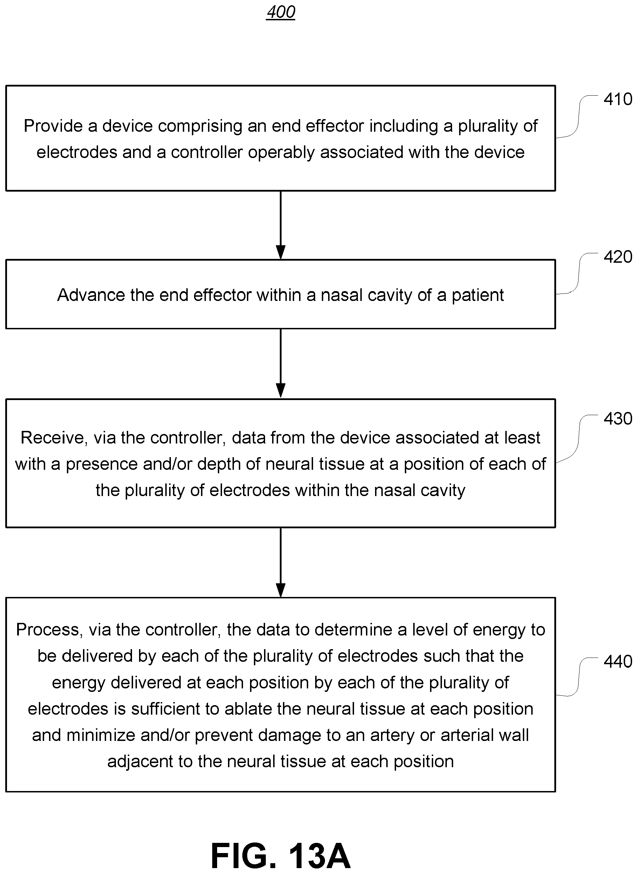

FIG. 13A is a flow diagram illustrating one embodiment of a non-claimed method 400 for treating a condition within a nasal cavity of a patient. The method 400 includes providing a device comprising an end effector including a plurality of electrodes and a controller operably associated with the device (operation 410) and advancing the end effector within a nasal cavity of a patient (operation 420). The end effector generally includes a micro-electrode array arranged about a plurality of struts, wherein each of the plurality of struts is able to conform to and accommodate an anatomical structure within the nasal cavity. When positioned within the nasal cavity, the struts contact multiple locations along multiple portions of a target site.

The method 400 further includes receiving, via the controller, data from the device associated at least with a presence and/or depth of neural tissue at a position of each of the plurality of electrodes within the nasal cavity (operation 430). The micro-electrode array includes at least a subset of electrodes configured to sense the presence of neural tissue at a respective position of each of the electrodes, as well as a depth of the neural tissue, and further convey such data to the controller. For example, in some embodiments, a subset of the plurality of electrodes is configured to deliver non-therapeutic stimulating energy to respective positions within the nasal cavity at a frequency for locating neural tissue and further sense one or more properties, including at least the presence and/or depth of the neural tissue in response to the non-therapeutic stimulating energy. The delivery of non-therapeutic stimulating energy may further allow for the sensing of other properties, such as physiological properties, bioelectric properties, and thermal properties of neural tissue at respective locations.

The method 400 further includes processing, via the controller, the data to determine a level of energy to be delivered by each of the plurality of electrodes such that the energy delivered at each position by each of the plurality of electrodes is sufficient to ablate the neural tissue at each position and minimize and/or prevent damage to an artery or arterial wall adjacent to the neural tissue at each position (operation 440). The electrodes are configured to be independently controlled and activated by the controller to thereby deliver energy independent of one another. Accordingly, the neuromodulation energy can be applied to the tissue via individual electrodes at respective locations in a highly targeted manner and form micro-lesions to selectively modulate the target structure, while avoiding non-targeted structures (e.g., other neural tissue, blood vessels, etc.) and allowing the surrounding tissue structure to remain healthy for effective wound healing. The controller is further configured to tune energy output from each of the plurality of electrodes after an initial level of energy has been delivered based, at least in part, on feedback data received from the device. The feedback data may be associated with efficacy of ablation of the neural tissue at each position during and/or after delivery of initial energy from each of the plurality of electrodes. The feedback data includes physiological properties, bioelectric properties, and thermal properties associated with the neural tissue.

In that manner, the invention solves the problem of causing collateral damage to non-neural tissue when generating micro-lesions within the nasal cavity and provides systems and methods for targeting neural tissue in a nasal region of a patient for the treatment of a rhinosinusitis condition that avoid or minimize creating collateral damage to surrounding or adjacent non-neural tissue, such as blood vessels as well as non-targeted neural tissue.

### SYSTEMS AND METHODS FOR TREATING AT LEAST ONE OF RHINITIS, CONGESTION, AND RHINORRHEA

### Field of the Invention

The invention further generally relates to systems for treating at least one of rhinitis, congestion, and/or rhinorrhea via thrombus formation.

### Background

Rhinitis is an inflammatory disease of the nose and is reported to affect up to 40% of the population. It is the fifth most common chronic disease in the United States. The most common and impactful symptoms of rhinitis are congestion and rhinorrhea. Nasal congestion, or "stuffiness", occurs when nasal tissues and blood vessels that line the passages inside the nasal cavity become swollen with excess fluid, thereby causing a "stuffy" feeling. More specifically, certain mucus producing and/or mucosal engorgement elements within the nasal cavity are responsible for causing nasal congestion. Such elements include the nasal mucosa, particularly mucus glands responsible for producing mucus in response to neural signals, as well as associated blood vessels which may become engorged and swollen as a result of increased blood flow due to irritation or inflammation of nasal tissues. Rhinorrhea is a condition where the nasal cavity is filled with a significant amount of mucus fluid. The condition, commonly known as a runny nose, occurs relatively frequently as a symptom of allergies (hay fever) or certain viral infections, such as the common cold.

### Summary

The invention further recognizes that intentional thrombotic formation within vasculature associated with certain mucus producing and/or mucosal engorgement elements within the nasal cavity provides a major clinical benefit in treating rhinitis, congestion and/or rhinorrhea. In particular, the invention provides systems and methods with an ability to deliver energy to tissue within the nasal cavity at a level sufficient to cause thrombus formation in blood vessels associated with (i.e., providing blood supply to) nasal mucosa, including mucus-producing glands. The thrombus formation within the blood vessels will cause occlusion (obstruction) within the lumen of the blood vessels, thereby resulting in starvation of the underlying nasal mucosa, specifically the mucus glands, as the blood supply will effectively be cut off. In turn, the mucus glands will experience hypoxia, a condition in which the mucus glands will become deprived of adequate oxygen supply. As a result, the mucus glands will undergo necrosis, thereby reducing or entirely eliminating mucus production from such glands. In that manner, the systems and methods address the most common and impactful symptoms or rhinitis, namely nasal congestion and rhinorrhea.

Aspects of the invention are accomplished with systems previously described herein, including a treatment device including an end effector to be placed within the nasal cavity of patient for the treatment of at least one of rhinitis, congestion, and rhinorrhea. The treatment device comprises an end effector, including a plurality of electrodes configured to delivery energy to one or more target sites within the nasal cavity. For example, the end effector may include a micro-electrode array, which may be arranged about a plurality of struts. Each of the plurality of struts is able to conform to and accommodate an anatomical structure within the nasal cavity. When positioned within the nasal cavity, the struts contact multiple locations along multiple portions of one or more target sites. Once in position, therapeutic energy is delivered by each of the plurality of electrodes sufficient to cause thrombus formation within one or more blood vessels associated with mucus producing and/or mucosal engorgement elements, namely nasal mucus and the associated mucus-producing glands. In particular, the energy is delivered at a level sufficient to trigger detachment of endothelial cells from blood vessel walls, thereby initiating a thrombotic cascade within the lumens of the blood vessels for thrombus formation, while minimizing and/or preventing damage to the walls themselves so as to maintain integrity of the walls and prevent or reduce the risk of rupture. For example, in some instances, the systems and methods may further include a controller configured to determine a level of energy to be delivered that is sufficient to cause the intentional thrombus formation (i.e., endothelial detachment) but below a certain threshold level that would otherwise damage the blood vessel wall. The thrombus formation effectively occludes the lumen of the blood vessels, resulting in local hypoxia and eventual necrosis of the mucus producing and/or mucosal engorgement elements, thereby decreasing production of mucus and/or mucosal engorgement in the nasal cavity.

In that manner, the treatment device that is capable of highly conforming to anatomical variations within a nasal cavity and further allows an operator to perform an accurate, minimally invasive, and localized application of energy to target sites within the nasal cavity to cause the desired effect, namely thrombus formation within targeted blood vessels for subsequent hypoxia and necrosis of nasal mucosa, particularly mucus glands, to reduce or eliminate mucus production. Accordingly, the present invention provides a user-friendly, minimally-invasive means of treating rhinitis symptoms, most notably nasal congestion and rhinorrhea.

One non-claimed aspect provides a method for treating at least one of rhinitis, congestion, and rhinorrhea within a nasal cavity of a patient. The method includes causing thrombus formation within one or more blood vessels associated with mucus producing and/or mucosal engorgement elements, resulting in local hypoxia of the mucus producing and/or mucosal engorgement elements, thereby decreasing production of mucus and/or mucosal engorgement. The mucus producing elements may include nasal mucosa and related mucus glands, for example.

In some embodiments, the causing of thrombus formation is accomplished via delivery of energy from a plurality of electrodes of an end effector advanced within a nasal cavity of a patient, wherein energy is delivered to one or more target sites associated with the mucus producing and/or mucosal engorgement elements at a level sufficient to cause the thrombus formation. The energy may be delivered at a level sufficient to trigger detachment of endothelial cells from blood vessel walls while minimizing and/or preventing damage to blood vessel walls. The energy may be delivered at a level sufficient to maintain integrity of the blood vessel walls. The endothelial detachment initiates thrombotic cascade within the lumen of the one or more blood vessels. The thrombotic cascade and subsequent thrombi formation may include, for example, an accumulation of fibrin admixed with blood. The delivery of energy may result in ablation of targeted tissue at the one or more target sites. The ablation may include thermal ablation. The ablation may be caused by delivery of radio-frequency energy, for example.

Another aspect of the invention provides a system for treating at least one of rhinitis, congestion, and rhinorrhea within a nasal cavity of a patient. The system includes a device comprising an end effector including a plurality of electrodes and a controller operably associated with the device. The controller is configured to receive data from the device associated at least with presence and/or a depth/location of blood vessels associated with mucus producing and/or mucosal engorgement elements at a position of each of the plurality of electrodes within the nasal cavity. The device is further configured to process the data to determine a level of energy to be delivered by each of the plurality of electrodes such that the energy delivered at each position by each of the plurality of electrodes is sufficient to cause thrombus formation within one or more blood vessels associated with the mucus producing and/or mucosal engorgement elements, resulting in local hypoxia of the mucus producing and/or mucosal engorgement elements, thereby decreasing production of mucus and/or mucosal engorgement. The mucus producing elements may include nasal mucosa and related mucus glands, for example.

In some embodiments, the energy is delivered at a level sufficient to trigger detachment of endothelial cells from blood vessel walls while minimizing and/or preventing damage to blood vessel walls. The energy may be delivered at a level sufficient to maintain integrity of the blood vessel walls. The endothelial detachment may initiate thrombotic cascade within the lumen of the one or more blood vessels. The thrombotic cascade and subsequent thrombi formation includes an accumulation of fibrin admixed with blood. The delivery of energy results in ablation of targeted tissue at one or more target sites associated with the mucus producing and/or mucosal engorgement elements. The ablation may include thermal ablation. The ablation may be caused by delivery of radio-frequency energy.

In some embodiments, the energy delivered may further cause vacuolar degeneration and/or necrosis or necrotic-like cell death of neural tissue associated with the mucus producing and/or mucosal engorgement elements.

The most common and impactful symptoms of rhinitis are congestion and rhinorrhea. Nasal congestion, or "stuffiness", occurs when nasal tissues and blood vessels that line the passages inside the nasal cavity become swollen with excess fluid, thereby causing a "stuffy" feeling. More specifically, certain mucus producing and/or mucosal engorgement elements within the nasal cavity are responsible for causing nasal congestion. Such elements include the nasal mucosa, particularly mucus glands responsible for producing mucus in response to neural signals, as well as associated blood vessels which may become engorged and swollen as a result of increased blood flow due to irritation or inflammation of nasal tissues. Rhinorrhea is a condition where the nasal cavity is filled with a significant amount of mucus fluid. The condition, commonly known as a runny nose, occurs relatively frequently as a symptom of allergies (hay fever) or certain viral infections, such as the common cold.

The invention recognizes that intentional thrombotic formation within vasculature associated with certain mucus producing and/or mucosal engorgement elements within the nasal cavity provides a major clinical benefit in treating rhinitis, congestion and/or rhinorrhea. In particular, the invention provides systems and methods with an ability to deliver energy to tissue within the nasal cavity at a level sufficient to cause thrombus formation in blood vessels associated with (i.e., providing blood supply to) nasal mucosa, including mucus-producing glands. The thrombus formation within the blood vessels will cause occlusion (obstruction) within the lumen of the blood vessels, thereby resulting in starvation of the underlying nasal mucosa, specifically the mucus glands, as the blood supply will effectively be cut off. In turn, the mucus glands will experience hypoxia, a condition in which the mucus glands will become deprived of adequate oxygen supply. As a result, the mucus glands will undergo necrosis, thereby reducing or entirely eliminating mucus production from such glands. In that manner, the systems and methods address the most common and impactful symptoms or rhinitis, namely nasal congestion and rhinorrhea.

It should be noted that, although many of the embodiments are described with respect to systems for therapeutically modulating tissue (neural and/or non-neural tissue) in the nasal region for the treatment of rhinitis, congestion, and/or rhinorrhea, other applications and other embodiments in addition to those described herein are within the scope of the present disclosure. For example, at least some embodiments of the present disclosure may be useful for the treatment of other indications, such as the treatment of chronic sinusitis and epistaxis. In particular, the embodiments described herein may be configured to treat allergic rhinitis, non-allergic rhinitis, chronic rhinitis, acute rhinitis, chronic sinusitis, acute sinusitis, chronic rhinosinusitis, acute rhinosinusitis, and/or medical resistant rhinitis.

In addition to therapeutically modulating nerves at precise and focused treatment sites corresponding to the sites of rami extending through fissures, accessory foramina, and microforamina throughout the palatine bone, wherein the targeted nerves are postganglionic parasympathetic nerves that go on to innervate the nasal mucosa, while maintaining at least some sympathetic tone by preserving a portion of the sympathetic contributions from the deep petrosal nerve and internal maxillary periarterial plexus, leading to improved outcomes with respect to nasal obstruction, embodiments of the present disclosure are configured to therapeutically modulate non-neural tissue at precise and focused treatment sites corresponding to targeted structures associated with mucus producing and/or mucosal engorgement elements, including, but not limited to, nasal mucosa and related mucus glands. For example, nasal congestion, or "stuffiness", occurs when nasal tissues and blood vessels that line the passages inside the nasal cavity become swollen with excess fluid, thereby causing a "stuffy" feeling. More specifically, certain mucus producing and/or mucosal engorgement elements within the nasal cavity are responsible for causing nasal congestion. As previously described, such elements include nasal mucosa and related mucus glands, which are responsible for producing mucus in response to neural signals, as well as associated blood vessels which may become engorged and swollen as a result of increased blood flow as a result of irritation or inflammation of nasal tissues. In certain embodiments, that targeted structures to receive therapeutic modulation (i.e., receive delivery of energy) include, for example, blood vessels associated with mucus glands, as will be described in greater detail herein.

The invention recognizes that intentional thrombotic formation within vasculature associated with certain mucus producing and/or mucosal engorgement elements within the nasal cavity provides a major clinical benefit in treating rhinitis, congestion and/or rhinorrhea. In particular, the invention provides systems and methods with an ability to deliver energy to tissue within the nasal cavity at a level sufficient to cause thrombus formation in blood vessels associated with (i.e., providing blood supply to) nasal mucosa, including mucus-producing glands. The thrombus formation within the blood vessels will cause occlusion (obstruction) within the lumen of the blood vessels, thereby resulting in starvation of the underlying nasal mucosa, specifically the mucus glands, as the blood supply will effectively be cut off. In turn, the mucus glands will experience hypoxia, a condition in which the mucus glands will become deprived of adequate oxygen supply. As a result, the mucus glands will undergo necrosis, thereby reducing or entirely eliminating mucus production from such glands. In that manner, the systems and methods address the most common and impactful symptoms or rhinitis, namely nasal congestion and rhinorrhea.

Aspects of the invention are accomplished with systems that include a treatment device including an end effector to be placed within the nasal cavity of patient for the treatment of at least one of rhinitis, congestion, and rhinorrhea. The treatment device comprises an end effector, including a plurality of electrodes configured to delivery energy to one or more target sites within the nasal cavity. For example, the end effector may include a micro-electrode array, which may be arranged about a plurality of struts. Each of the plurality of struts is able to conform to and accommodate an anatomical structure within the nasal cavity. When positioned within the nasal cavity, the struts contact multiple locations along multiple portions of one or more target sites. Once in position, therapeutic energy is delivered by each of the plurality of electrodes sufficient to cause thrombus formation within one or more blood vessels associated with mucus producing and/or mucosal engorgement elements, namely nasal mucus and the associated mucus-producing glands. In particular, the energy is delivered at a level sufficient to trigger detachment of endothelial cells from blood vessel walls, thereby initiating a thrombotic cascade within the lumens of the blood vessels for thrombus formation, while minimizing and/or preventing damage to the walls themselves so as to maintain integrity of the walls and prevent or reduce the risk of rupture. For example, in some instances, the systems and methods may further include a controller configured to determine a level of energy to be delivered that is sufficient to cause the intentional thrombus formation (i.e., endothelial detachment) but below a certain threshold level that would otherwise damage the blood vessel wall. The thrombus formation effectively occludes the lumen of the blood vessels, resulting in local hypoxia and eventual necrosis of the mucus producing and/or mucosal engorgement elements, thereby decreasing production of mucus and/or mucosal engorgement in the nasal cavity.

In that manner, the treatment device that is capable of highly conforming to anatomical variations within a nasal cavity and further allows an operator to perform an accurate, minimally invasive, and localized application of energy to target sites within the nasal cavity to cause the desired effect, namely thrombus formation within targeted blood vessels for subsequent hypoxia and necrosis of nasal mucosa, particularly mucus glands, to reduce or eliminate mucus production. Accordingly, the present invention provides a user-friendly, minimally-invasive means of treating rhinitis symptoms, most notably nasal congestion and rhinorrhea.

As such, the invention recognizes that, in some instances, knowing the presence, location, as well as depth of various tissue (neural tissue and non-neural tissue) within the nasal anatomy provides an ability to precisely target vasculature associated with mucus producing and/or mucosal engorgement elements to provide the desired effect (i.e., thrombus formation within such vasculature), while minimizing unintended damage. For example, the depth of neural tissues as well as non-neural tissues (i.e., nasal mucosa, mucus-producing glands, seromucous glands, blood vessels, etc.) in the nasal anatomy varies depending on the nasal region and where in the nasal cavity treatment is being applied. The systems and methods of the present invention may further include an ability to tune energy delivery to accommodate the precise location (e.g., depth) of targeted blood vessels associated with the functioning of certain mucus producing and/or mucosal engorgement elements, which is important to prevent excessive damage and destruction to non-targeted tissue when performing a treatment procedure within the nasal cavity. In particular, the invention allows for tuning energy delivery to a level sufficient to cause thrombus formation within one or more blood vessels associated with mucus glands, thereby resulting in local hypoxia and eventual necrosis of the mucus glands for subsequent decrease or complete elimination of mucus production from such glands, while preventing excessive damage to the blood vessels themselves (i.e., maintain integrity of blood vessel walls) and further minimizing or preventing damage to surrounding non-targeted tissue. In that manner, the invention solves the problem of causing unnecessary collateral damage to non-targeted tissue during a procedure for the treatment of a rhinosinusitis condition, most notably the treatment of nasal congestion.

For example, the micro-electrode array may include at least a subset of electrodes configured to sense the presence and a depth/location of blood vessels associated with mucus producing and/or mucosal engorgement elements at a respective position of each of the electrodes. In turn, the controller processes such data and determines a level of therapeutic energy to be delivered by each of the plurality of electrodes sufficient to cause thrombus formation within one or more blood, wherein the energy is delivered at a level sufficient to trigger detachment of endothelial cells from blood vessel walls, thereby initiating a thrombotic cascade within the lumens of the blood vessels for thrombus formation, while minimizing and/or preventing damage to the blood vessel walls so as to maintain integrity of the blood vessel walls (i.e., prevent rupture). More specifically, based on feedback data from the end effector, the controller allows for the tuning of energy delivery at a given position to accommodate variations in tissue, including depth of targeted blood vessels, as well as other physiological, bioelectric, and/or thermal properties, which is critical in preventing excessive damage and destruction to surrounding structures during a treatment procedure.

Furthermore, embodiments of the present disclosure are configured to therapeutically modulate non-neural tissue at precise and focused treatment sites corresponding to blood vessels associated with mucus producing and/or mucosal engorgement elements, including, but not limited to, nasal mucosa and related mucus glands.

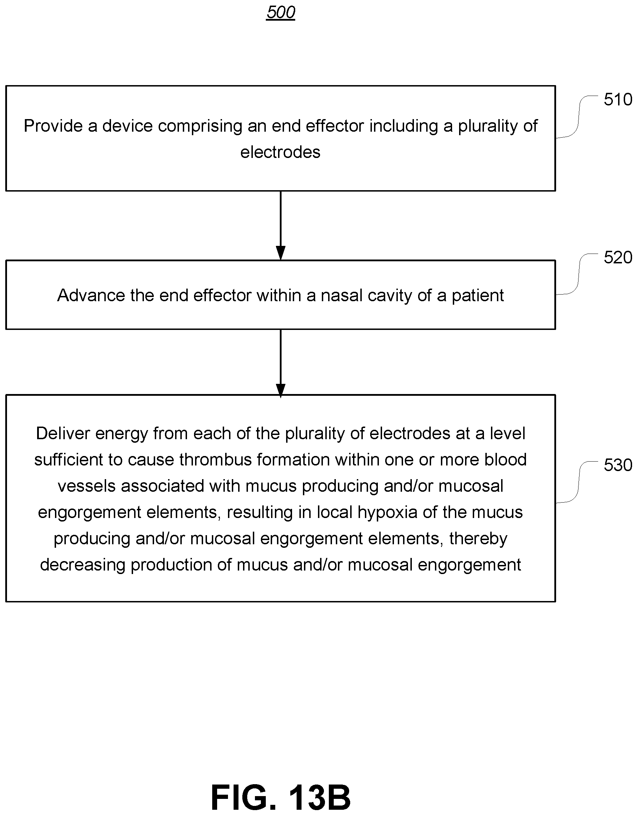

FIG. 13B is a flow diagram illustrating one embodiment of a non-claimed method 500 for treating a condition within a nasal cavity of a patient. The method 500 includes providing a device comprising an end effector including a plurality of electrodes (operation 510) and advancing the end effector within a nasal cavity of a patient (operation 520). The end effector generally includes a micro-electrode array arranged about a plurality of struts, wherein each of the plurality of struts is able to conform to and accommodate an anatomical structure within the nasal cavity. When positioned within the nasal cavity, the struts contact multiple locations along multiple portions of a target site. The end effector may generally be advanced to one or more target sites associated with mucus producing and/or mucosal engorgement elements, such as submucosal glands, for example.

The method 500 further includes delivering energy from each of the plurality of electrodes at a level sufficient to cause thrombus formation within one or more blood vessels associated with mucus producing and/or mucosal engorgement elements, resulting in local hypoxia of the mucus producing and/or mucosal engorgement elements, thereby decreasing production of mucus and/or mucosal engorgement. In particular, the energy is delivered at a level sufficient to trigger detachment of endothelial cells from blood vessel walls, thereby initiating a thrombotic cascade within the lumens of the blood vessels for thrombus formation, while minimizing and/or preventing damage to the walls themselves so as to maintain integrity of the walls and prevent or reduce the risk of rupture. For example, in some instances, the level of energy delivered is sufficient to cause the intentional thrombus formation (i.e., endothelial detachment) but below a certain threshold level that would otherwise damage the blood vessel wall, thereby maintaining integrity of the walls (i.e., prevent rupture).. The thrombus formation effectively occludes the lumen of the blood vessels, resulting in local hypoxia and eventual necrosis of the mucus producing and/or mucosal engorgement elements, thereby decreasing production of mucus and/or mucosal engorgement in the nasal cavity.

In that manner, the treatment device that is capable of highly conforming to anatomical variations within a nasal cavity and further allows an operator to perform an accurate, minimally invasive, and localized application of energy to target sites within the nasal cavity to cause the desired effect, namely thrombus formation within targeted blood vessels for subsequent hypoxia and necrosis of nasal mucosa, particularly mucus glands, to reduce or eliminate mucus production. Accordingly, the present invention provides a user-friendly, minimally-invasive means of treating rhinitis symptoms, most notably nasal congestion and rhinorrhea.

For example, mucus is produced by, and covers, mucous membranes. Mucus is typically produced from cells found in mucous glands, although it may also originate from mixed glands, which contain both serous and mucous cells. It is a viscous colloid containing inorganic salts, antiseptic enzymes (such as lysozymes), immunoglobulins, and glycoproteins such as lactoferrin and mucins, which are produced by goblet cells in the mucous membranes and submucosal glands.

Nasal mucus is produced by the nasal mucosa. Small particles such as dust, particulate pollutants, and allergens, as well as infectious agents and bacteria are caught in the viscous nasal mucus and prevented from entering the system. This event along with the continual movement of the respiratory mucus layer toward the oropharynx (mucociliary clearance), helps prevent foreign objects from entering the lungs during breathing. This explains why coughing often occurs in those who smoke cigarettes. The body's natural reaction is to increase mucus production. In addition, mucus aids in moisturizing the inhaled air and prevents tissues such as the nasal and airway epithelia from drying out. Nasal and airway mucus is produced continuously, with most of it swallowed subconsciously, even when it is dried.

Increased mucus production in the respiratory tract is a symptom of many common illnesses, such as the common cold and influenza. Mucus hypersecretion can occur in inflammatory respiratory diseases such as respiratory allergies, asthma, and chronic bronchitis. The presence of mucus in the nose and throat is normal, but increased quantities can impede comfortable breathing and must be cleared by blowing the nose or expectorating it as sputum from the throat.

The nasal mucosa, or respiratory mucosa, is the mucous membrane lining the nasal cavities, and is intimately adherent to the periosteum or perichondrium of the nasal conchae. It is continuous with the skin through the nostrils, and with the mucous membrane of the nasal part of the pharynx through the choanae. From the nasal cavity, it's continuity with the conjunctiva may be traced, through the nasolacrimal and lacrimal ducts, and with the frontal, ethmoidal, sphenoidal, and maxillary sinuses, through the several openings in the meatuses. The mucous membrane is thickest, and most vascular, over the nasal conchae. It is also thick over the septum, but it is very thin in the meatuses on the floor of the nasal cavities, and in the various sinuses. It is one of the most commonly infected tissues in adults and children. Inflammation of this tissue may cause significant impairment of daily activities, with symptoms such as stuffy nose, headache, mouth breathing, etc.

The nasal mucosa relies on associated blood vessels for blood supply. Accordingly, by targeting and modulating such blood vessels (i.e., delivering a specific level of energy thereto), associated nasal mucosa and related mucus glands relying on blood supplied by the blood vessels can be targeted due to secondary effects as a result. For example, the energy can be applied to the tissue in a highly targeted manner to selectively modulate the targeted blood vessels for the desired thrombus formation effect while maintaining integrity of the blood vessel walls, and further avoiding non-targeted structures (e.g., other blood vessels, neural tissue, etc.) and allowing the surrounding tissue structure to remain healthy for effective wound healing.

In particular, energy can be delivered at a level sufficient to cause thrombus formation within the one or more blood vessels. The energy may be delivered at a level sufficient to trigger detachment of endothelial cells from blood vessel walls which, in turn, initiates thrombotic cascade within the lumen of the one or more blood vessels. The thrombotic cascade and subsequent thrombi formation may include, for example, an accumulation of fibrin admixed with blood. A thrombus, colloquially called a blood clot, is the final product of the blood coagulation step in hemostasis. There are generally two components to a thrombus: aggregated platelets and red blood cells that form a plug, and a mesh of cross-linked fibrin protein. The substance making up a thrombus is sometimes called cruor. A thrombus occurs when the hemostatic process, which normally occurs in response to injury, becomes activated in an uninjured or slightly injured vessel. A thrombus in a large blood vessel will decrease blood flow through that vessel (termed a mural thrombus). In a small blood vessel, blood flow may be completely cut off (termed an occlusive thrombus), resulting in death of tissue supplied by that vessel.

A thrombus is a healthy response to injury intended to prevent bleeding, but can be harmful in thrombosis, when clots obstruct blood flow through healthy blood vessels. However, in this instance, the thrombotic formation within the vasculature is intentional, as there is a major clinical benefit. In particular, thrombus formation within the blood vessels will cause occlusion (obstruction) within the lumen of the blood vessels, thereby resulting in starvation of the underlying nasal mucosa, specifically the mucus glands, as the blood supply will effectively be cut off. In turn, the mucus glands will experience hypoxia, a condition in which the nasal mucosa, particularly the mucus glands, will become deprived of adequate oxygen supply. In turn, the mucus glands will undergo necrosis, thereby impacting their ability to produce mucus.

### SYSTEMS AND METHODS FOR NEURAL TISSUE IDENFICATION AND PRECISION THERAPEUTIC NEUROMODULATION

### Field of the Invention

The invention further generally relates to systems for providing detection, identification, and precision targeting of neural tissue for the treatment of a neurological condition while minimizing or avoiding collateral damage to surrounding or adjacent non-neural tissue, such as blood vessels and bone, as well as non-targeted neural tissue.

### Background

Neuromodulation refers to the alteration, or modulation, of nerve activity by delivering electrical (or sometimes pharmaceutical) agents directly to a target area. The delivery of electrical stimulation can result in partial or complete incapacitation, or other effective disruption, of neural activity. Therapeutic neuromodulation, for example, can include partially or completely inhibiting, reducing, and/or blocking neural communication along neural fibers for the treatment of certain conditions and disorders, specifically for pain relief and/or restoration of function. Some conditions and disorders that may be treated via neuromodulation include, but are not limited to, epilepsy, migraine headaches, spinal cord injuries, Parkinson's disease, and urinary incontinence, to name a few.

Neuromodulation treatment procedures involve the application of electrodes to the brain, the spinal cord, or peripheral nerves for subsequent treatment of conditions or disorders associated therewith. The electrodes are coupled, via an extension cable, to a pulse generator and power source, which generates the necessary electrical stimulation. An electrical current passes from the generator to the nerve, and can either inhibit pain signals or stimulate neural impulses where they were previously absent. Importantly, electrodes must be precisely placed and the level of electrical stimulation must be controlled so as to avoid or minimize creating collateral damage to surrounding or adjacent non-neural structures, such as bone and blood vessels, as well as non-targeted neural tissue.

Peripheral nerve stimulation is a commonly used approach to treat peripheral neurological conditions and conditions, including chronic pain. In order to establish accurate placement of electrodes and level of electrical stimulation to the targeted peripheral nerve, peripheral nerve stimulation treatment typically requires an initial testing or trial period. For example, a small electrical device (a wire-like electrode) is surgically implanted and placed next to one of the peripheral nerves. The electrode delivers rapid electrical pulses during the initial testing period (trial) to determine whether the electrical pulses result in the desired effect. Once the desired effect is established (via repositioning and/or adjusting of electrical stimulation levels) a more permanent electrode may be implanted into a patient's body. Accordingly, a drawback to current neuromodulation procedures, notably neuromodulation of peripheral nerves, is that such procedures cannot precisely target neural tissue, thereby presenting risk of causing significant collateral damage to surrounding non-neural tissue (such as blood vessels), and/or other non-targeted neural tissue.

### Summary

The invention recognizes that knowing specific properties of neural tissue, including the morphology of the neural tissue and the specific nerve type, at a given target site prior to neuromodulation treatment provides an ability to more precisely target that neural tissue and minimize collateral damage to surrounding non-neural tissue (e.g., blood vessels). For example, the specific nerve type of a given neural tissue dictates the level of electrical stimulation required to elicit a desired effect. Furthermore, physical properties of the neural tissue, including depth of the neural tissue, further impacts the level of electrical stimulation necessary to result in effective therapeutic neuromodulation. For example, types of neural tissue may vary at certain target sites and neural tissue depth may vary depending on the specific region of the patient's body upon which treatment is to be applied. The invention provides systems and methods with an ability to determine, prior to neuromodulation treatment, the type of neural tissue at a target site and tune energy output based on such determination such that resulting micro-lesions accommodate the specific neural tissue to be treated and the precise location (e.g., depth) of the neural tissue at a given site, which is important to prevent excessive damage and destruction to non-neural tissue when generating micro-lesions. In that manner, the invention solves the problem of causing unnecessary collateral damage to non-neural tissue when generating micro-lesions during a neuromodulation procedure, as the systems and methods are able to detect and identify nerve type, location, and other properties of intended neural tissue prior to neuromodulation treatment.

Aspects of the invention are accomplished with systems previously described herein, including a treatment device and a controller for providing precise control and targeting of energy output from the treatment device. The treatment device includes an end effector comprising an electrode array composed of a plurality of electrodes. The electrode array includes at least a subset of electrodes configured to sense the presence of neural tissue at a respective position of each of the electrodes, as well as morphology of the neural tissue, and further convey such data to the controller. In turn, the controller processes such data and determines a type of neural tissue at the target site. The controller further determines a level of energy to be delivered by one or more of the plurality of electrodes based, at least in part, on the type of neural tissue. In one example, the level of energy output from the end effector may be at a diagnostic energy level useful for diagnosing a neurological condition based on feedback data captured by electrodes and/or sensors of the end effector. In another example, the level of energy output from the end effector may be at a therapeutic energy level sufficient to therapeutically modulate (e.g. ablate) the neural tissue while minimizing and/or preventing damage to a surrounding or adjacent structure, such as an artery or arterial wall adjacent to the neural tissue.

The plurality electrodes of the electrode array may be configured to be independently controlled and activated by the controller to thereby deliver energy independent of one another. Accordingly, the neuromodulation energy can be applied to the tissue in a highly targeted manner and form micro-lesions to selectively modulate the target structure, while avoiding non-targeted structures (e.g., other neural tissue, blood vessels, etc.) and allowing the surrounding tissue structure to remain healthy for effective wound healing. In that manner, the present invention provides a neuromodulation treatment system providing unprecedented control to an operator so that an operator can perform an accurate, minimally invasive, and localized application of energy to target sites to cause multi-point interruption of neural signal without causing collateral damage or disruption to other, non-targeted tissue (e.g., blood vessels, other non-targeted neural tissue, etc.). More specifically, based on an initial feedback data from the treatment device prior to delivery of therapeutic energy, the specific type of neural tissue is identified as well as other properties of the neural tissue. In turn, the controller allows for the tuning of energy output such that resulting micro-lesions accommodate variations in neural tissue, including the specific type of neural tissue, depth of neural tissue, as well as other physiological, bioelectric, and/or thermal properties, which is critical in preventing excessive damage and destruction to surrounding structures during a treatment procedure.

Accordingly, the system of the present invention provides a user-friendly, minimally-invasive means of treating a neurological condition or disorder, including precise and focused application of energy to the intended target sites for therapeutic modulation of intended neural tissue, specifically the creation of multiple micro-lesion resulting in multi-point interruption of neural signals, without causing collateral damage or disruption to non-neural tissue (e.g., blood vessels) or non-targeted neural tissue. The creation of micro-lesions along a neural tissue is critical for various reasons. The creation of multiple micro-lesions, and resulting multi-point interruption, accounts for the various entry points and pathways associated with certain nerves. The multiple micro-lesions further creates more than a single break along a nerve pathway, which is vital for longevity of the effect of treatment as it prevents functional alignment and regeneration of the nerve tissue. Furthermore, the controller is able to tailor the size and shape of any given micro-lesion at a respective location along a neural tissue by way of independent control of electrodes of the electrode array, thereby further enhancing the effectiveness of treatment by accounting for nerve type and variations in neural tissue depth, for example, at any given location. The creation of multiple micro-lesions is much more effective than a single, continuous lesion, which presents further potential risks.

One aspect of the invention provides a system for treating a peripheral neurological condition. The system includes a device comprising an end effector including a plurality of electrodes and a controller operably associated with the device. The controller is configured to receive data from the device associated at least with presence and/or a depth of neural tissue at a position of each of the plurality of electrodes relative to one or more target sites. The controller is further configured to process the data to determine a level of energy to be delivered by each of the plurality of electrodes such that the energy delivered at each position by each of the plurality of electrodes is sufficient to ablate the neural tissue at each position and minimize and/or prevent damage to an artery or arterial wall adjacent to the neural tissue at each position.

In some embodiments, a subset of the plurality of electrodes is configured to sense at least the presence and/or depth of neural tissue at the position of each of the plurality of electrodes and deliver data associated with the depths to the controller. For example, a subset of the plurality of electrodes may be configured to deliver non-therapeutic stimulating energy to respective positions at the one or more target sites at a frequency for locating neural tissue and further sense one or more properties, including at least the presence and/or depth of the neural tissue in response to the non-therapeutic stimulating energy.

In some embodiments, the controller is configured to tune energy output from each of the plurality of electrodes after an initial level of energy has been delivered based, at least in part, on feedback data received from the device. The feedback data may be associated with efficacy of ablation of the neural tissue at each position during and/or after delivery of initial energy from each of the plurality of electrodes. The feedback data may include, for example, one or more properties associated with neural tissue at respective positions at the one or more target sites, the one or more properties comprising at least one of physiological properties, bioelectric properties, and thermal properties. In some embodiments, a subset of the plurality of electrodes is configured to deliver non-therapeutic stimulating energy to respective positions at the one or more target sites at a frequency for locating neural tissue and sense one or more properties associated with the neural tissue in response to the non-therapeutic stimulating energy. The properties may include at least one of physiological properties, bioelectric properties, and thermal properties. The bioelectric properties may include, but are not limited to, complex impedance, resistance, reactance, capacitance, inductance, permittivity, conductivity, nerve firing voltage, nerve firing current, depolarization, hyperpolarization, magnetic field, and induced electromotive force.

In some embodiments, at least some of the electrodes are configured to be independently controlled and activated by the controller to thereby deliver energy independent of one another. For example, at least some of the electrodes have different levels of energy to be delivered at respective positions sufficient to ablate neural tissue at the respective positions.

Another non-claimed aspect provides a method for treating a peripheral neurological condition. The method includes providing a device comprising an end effector including a plurality of electrodes and a controller operably associated with the device and positioning the end effector at one or more target sites associated with a patient. The method further includes receiving, via the controller, data from the device associated at least with a presence and/or depth of neural tissue at a position of each of the plurality of electrodes at the one or more target sites. The method further includes processing, via the controller, the data to determine a level of energy to be delivered by each of the plurality of electrodes such that the energy delivered at each position by each of the plurality of electrodes is sufficient to ablate the neural tissue at each position and minimize and/or prevent damage to an artery or arterial wall adjacent to the neural tissue at each position.

In some embodiments, a subset of the plurality of electrodes is configured to sense at least the presence and/or depth of neural tissue at the position of each of the plurality of electrodes and deliver data associated with the depths to the controller. For example, a subset of the plurality of electrodes may be configured to deliver non-therapeutic stimulating energy to respective positions at the one or more target sites at a frequency for locating neural tissue and further sense one or more properties, including at least the presence and/or depth of the neural tissue in response to the non-therapeutic stimulating energy.

In some embodiments, the controller is configured to tune energy output from each of the plurality of electrodes after an initial level of energy has been delivered based, at least in part, on feedback data received from the device. The feedback data may be associated with efficacy of ablation of the neural tissue at each position during and/or after delivery of initial energy from each of the plurality of electrodes. For example, the feedback data may include one or more properties associated with neural tissue at respective positions at the one or more target sites. The one or more properties may include at least one of physiological properties, bioelectric properties, and thermal properties. In some embodiments, a subset of the plurality of electrodes is configured to deliver non-therapeutic stimulating energy to respective positions at the one or more target sites at a frequency for locating neural tissue and sense one or more properties associated with the neural tissue in response to the non-therapeutic stimulating energy. The properties may include at least one of physiological properties, bioelectric properties, and thermal properties. In some embodiments, the bioelectric properties may include at least one of complex impedance, resistance, reactance, capacitance, inductance, permittivity, conductivity, nerve firing voltage, nerve firing current, depolarization, hyperpolarization, magnetic field, and induced electromotive force.

In some embodiments, energy delivered may cause vacuolar degeneration of the neural tissue. For example, in some embodiments, the energy delivered by each of the plurality of electrodes causes vacuolar degeneration of the neural tissue at each position. In some embodiments, the energy delivered causes ionic agitation resulting in vacuolar degeneration of the neural tissue. Yet still, in some embodiments, the energy delivered results in vacuolar degeneration of the neural tissue and/or necrosis or necrotic-like cell death of the neural tissue. Further still, in some embodiments, the energy delivered causes ionic agitation by way of an osmotic potential change through which extracellular fluid to neural tissue passes into the nerve tubules, thereby resulting in vacuolar degeneration and/or necrosis or necrotic-like cell death of the neural tissue.

Another aspect of the invention provides a system for diagnosing and/or treating a neurological condition of a patient. The system includes a device comprising an end effector including a plurality of electrodes and a controller operably associated with the device. The controller is configured to receive data from the device that is associated with neural tissue at a target site, process the data to determine a type of neural tissue at the target site, and determine energy output from the end effector based on the type of neural tissue at the target site.

In some embodiments, the energy output from the end effector may be at a diagnostic energy level or a therapeutic energy level. As such, in one embodiment, a subset of the plurality of electrodes may be configured to deliver non-therapeutic energy to the target site at a level sufficient to modulate the neural tissue for diagnostic feedback and minimize and/or prevent damage to non-target neural tissue and/or non-target anatomical structures at the target site. In turn, a subset of the plurality of electrodes may be configured to sense one or more properties of the neural tissue in response to the non-therapeutic energy and deliver data associated with the one or more properties of the neural tissue to the controller for subsequent processing for diagnosing a neurological condition. In other embodiments, a subset of the plurality of electrodes is configured to deliver therapeutic energy to the target site at a level sufficient to therapeutically modulate the neural tissue and minimize and/or prevent damage to non-target neural tissue and/or non-target anatomical structures at the target site.

In some embodiments, a subset of the plurality of electrodes is configured to sense one or more properties associated with the neural tissue at the target site and deliver data associated with the one or more properties to the controller. A subset of the plurality of electrodes may be configured to deliver non-therapeutic stimulating energy to the target site at a frequency for locating neural tissue and further sense one or more properties, including at least the presence and morphology of the neural tissue in response to the non-therapeutic stimulating energy. The morphology may include at least one of physiological properties and bioelectric properties of the neural tissue. The bioelectric properties may include at least one of complex impedance, resistance, reactance, capacitance, inductance, permittivity, conductivity, nerve firing voltage, nerve firing current, depolarization, hyperpolarization, magnetic field, and induced electromotive force. The controller may be configured to determine neural tissue type based, at least in part, on the morphology.

Another non-claimed aspect provides a method for diagnosing and/or treating a neurological condition of a patient. The method includes providing a device comprising an end effector including a plurality of electrodes and a controller operably associated with the device. The method further includes positioning the end effector at a target site associated with a patient. The method includes receiving, via the controller, data from the device associated with neural tissue at the target site and processing, via the controller, the data to determine a type of neural tissue at the target site. The method further includes determining, via the controller, energy output from the end effector based on the type of neural tissue at the target site.

In some embodiments, the energy output from the end effector is at a diagnostic energy level or a therapeutic energy level. In some embodiments, the method further includes delivering, via a subset of the plurality of electrodes, non-therapeutic energy to the target site at a level sufficient to modulate the neural tissue for diagnostic feedback and minimize and/or prevent damage to non-target neural tissue and/or non-target anatomical structures at the target site. As such, in some embodiments, the method includes sensing, via a subset of the plurality of electrodes, one or more properties of the neural tissue in response to the non-therapeutic energy and delivering data associated with the one or more properties of the neural tissue to the controller for subsequent processing for diagnosing a neurological condition. In other embodiments, the method includes delivering, via a subset of the plurality of electrodes, therapeutic energy to the target site at a level sufficient to therapeutically modulate the neural tissue and minimize and/or prevent damage to non-target neural tissue and/or non-target anatomical structures at the target site.

In some embodiments, the method includes sensing, via a subset of the plurality of electrodes, one or more properties associated with the neural tissue at the target site and delivering data associated with the one or more properties to the controller. The method further includes delivering, via a subset of the plurality of electrodes, non-therapeutic stimulating energy to the target site at a frequency for locating neural tissue and further sense one or more properties, including at least the presence and morphology of the neural tissue in response to the non-therapeutic stimulating energy. The morphology may include at least one of physiological properties and bioelectric properties of the neural tissue. The bioelectric properties may include, but are not limited to, complex impedance, resistance, reactance, capacitance, inductance, permittivity, conductivity, nerve firing voltage, nerve firing current, depolarization, hyperpolarization, magnetic field, and induced electromotive force. The controller is configured to determine neural tissue type based, at least in part, on the morphology.

### Detailed Description

Neuromodulation is technology that acts directly upon nerves. It is the alteration, or modulation, of nerve activity by delivering electrical or pharmaceutical agents directly to a target area. Neuromodulation devices and treatments have been shown to be highly effective at treating a variety of conditions and disorders. The most common indication for neuromodulation is treatment of chronic pain. However, the number of neuromodulation applications over the years has increased to include more than just the treatment of chronic pain, such as deep brain stimulation (DBS) treatment for Parkinson's disease, sacral nerve stimulation for pelvic disorders and incontinence, and spinal cord stimulation for ischemic disorders (angina, peripheral vascular disease).

Neuromodulation is particularly useful in the treatment of peripheral neurological disorders. There are currently over 100 kinds of peripheral nerve disorders, which can affect one nerve or many nerves. Some are the result of other diseases, like diabetic nerve problems. Others, like Guillain-Barre syndrome, happen after a virus infection. Still others are from nerve compression, like carpal tunnel syndrome or thoracic outlet syndrome. In some cases, like complex regional pain syndrome and brachial plexus injuries, the problem begins after an injury. However, some people are born with peripheral neurological disorders.

Peripheral nerve stimulation has become established for very specific clinical indications, including certain complex regional pain syndromes, pain due to peripheral nerve injuries, and the like. Some of the common applications of peripheral nerve stimulation include treatment of back pain, occipital nerve stimulation for treatment of migraine headaches, and pudendal nerve stimulation that is being investigated for use in urinary bladder incontinence.

The invention recognizes that knowing specific properties of neural tissue, including the morphology of the neural tissue and the specific nerve type, at a given target site prior to neuromodulation treatment provides an ability to more precisely target that neural tissue and minimize collateral damage to surrounding non-neural tissue (e.g., blood vessels). For example, the specific nerve type of a given neural tissue dictates the level of electrical stimulation required to elicit a desired effect. Furthermore, physical properties of the neural tissue, including location and depth of the neural tissue, further impacts the level of electrical stimulation necessary to result in effective therapeutic neuromodulation. For example, types of neural tissue may vary at certain target sites and neural tissue depth may vary depending on the specific region of the patient's body upon which treatment is to be applied.

The invention provides systems with an ability to determine, prior to neuromodulation treatment, the type of neural tissue at a target site and tune energy output based on such determination such that resulting micro-lesions accommodate the specific neural tissue to be treated and the precise location (e.g., depth) of the neural tissue at a given site, which is important to prevent excessive damage and destruction to non-neural tissue when generating micro-lesions. In that manner, the invention solves the problem of causing unnecessary collateral damage to non-neural tissue when generating micro-lesions during a neuromodulation procedure, as the systems and methods are able to detect and identify nerve type, location, and other properties of intended neural tissue prior to neuromodulation treatment.

It should be noted that, although many of the embodiments are described with respect to systems for therapeutically modulating nerves associated with the peripheral nervous system (PNS) and thus the treatment of peripheral neurological conditions or disorders, other applications and other embodiments in addition to those described herein are within the scope of the present disclosure. For example, at least some embodiments of the present disclosure may be useful for the treatment of other disorders, such as the treatment of disorders associated with the central nervous system.

Referring to FIGS. 1A and 1B, the therapeutic neuromodulation system 100 can be used for treating a neurological condition of a patient by using a handheld device 102 according to some embodiments of the present disclosure. As previously described, the system 100 generally includes a neuromodulation device 102 and a neuromodulation console 104 to which the device 102 is to be connected. Referring back to FIG. 2, diagrammatic illustrations of the console 104 coupled to the handheld neuromodulation device 102 illustrate an exemplary embodiment of an end effector 114 for delivering energy to tissue at the one or more target sites of a patient for the treatment of a neurological disorder. As illustrated, the neuromodulation device 102 is a handheld device, which includes end effector 114, a shaft 116 operably associated with the end effector 114, and a handle 118 operably associated with the shaft 116. The end effector 114 may be collapsible/retractable and expandable, thereby allowing for the end effector 114 to be minimally invasive (i.e., in a collapsed or retracted state) upon delivery to one or more target sites within a patient and then expanded once positioned at the target site. It should be noted that the terms "end effector" and "therapeutic assembly" may be used interchangeably throughout this disclosure.

For example, a surgeon or other medical professional performing a procedure can utilize the handle 118 to manipulate and advance the shaft 116 to a desired target site, wherein the shaft 116 is configured to locate at least a distal portion thereof intraluminally at a treatment or target site within a portion of the patient associated with neural tissue to undergo neuromodulation for subsequent treatment of an associated neurological condition or disorder. The one or more target sites may generally be associated with peripheral nerve fibers. The target site may be a region, volume, or area in which the target nerves are located and may differ in size and shape depending upon the anatomy of the patient. Once positioned, the end effector 114 may be deployed and subsequently deliver energy to the one or more target sites. The energy delivered may be non-therapeutic stimulating energy at a frequency for locating neural tissue and further sensing one or more properties of the neural tissue. For example, the end effector 114 may include an electrode array, which includes at least a subset of electrodes configured to sense the presence of neural tissue at a respective position of each of the electrodes, as well as morphology of the neural tissue, wherein such data may be used for determining, via the console 104, the type of neural tissue. Based on the identification of the neural tissue type, the energy delivered from the end effector 114 upon the target site may be therapeutic stimulating energy (tuned via the controller 104 and based on the neural tissue type) at a level sufficient to therapeutically modulate the neural tissue and minimize and/or prevent damage to non-target neural tissue and/or non-target anatomical structures at the target site, such as blood vessels and/or bone. Accordingly, the end effector 114 is able to therapeutically modulating nerves of interest, particularly nerves associated with a peripheral neurological conditional or disorder so as to treat such condition or disorder, while minimizing and/or preventing collateral damage.

For example, the end effector 114 may include at least one energy delivery element, such as an electrode, configured to delivery energy to the target tissue which may be used for sensing presence and/or specific properties of neural tissue or for therapeutically modulating neural tissue. For example, one or more electrodes may be provided by one or more portions of the end effector 114, wherein the electrodes may be configured to apply electromagnetic neuromodulation energy (e.g., radiofrequency (RF) energy) to target sites. In other embodiments, the end effector 114 may include other energy delivery elements configured to provide therapeutic neuromodulation using various other modalities, such as cryotherapeutic cooling, ultrasound energy (e.g., high intensity focused ultrasound ("HIFU") energy), microwave energy (e.g., via a microwave antenna), direct heating, high and/or low power laser energy, mechanical vibration, and/or optical power.

In some embodiments, the end effector 114 may include one or more sensors (not shown), such as one or more temperature sensors (e.g., thermocouples, thermistors, etc.), impedance sensors, and/or other sensors. The sensors and/or the electrodes may be connected to one or more wires extending through the shaft 116 and configured to transmit signals to and from the sensors and/or convey energy to the electrodes.

As shown, the device 102 is operatively coupled to the console 104 via a wired connection, such as cable 120. It should be noted, however, that the device 102 and console 104 may be operatively coupled to one another via a wireless connection. The console 104 is configured to provide various functions for the neuromodulation device 102, which may include, but is not limited to, controlling, monitoring, supplying, and/or otherwise supporting operation of the neuromodulation device 102. For example, when the neuromodulation device 102 is configured for electrode-based, heat-element-based, and/or transducer-based treatment, the console 104 may include an energy generator 106 configured to generate RF energy (e.g., monopolar, bipolar, or multi-polar RF energy), pulsed electrical energy, microwave energy, optical energy, ultrasound energy (e.g., intraluminally-delivered ultrasound and/or HIFU), direct heat energy, radiation (e.g., infrared, visible, and/or gamma radiation), and/or another suitable type of energy.

In some embodiments, the console 104 may include a controller 107 communicatively coupled to the neuromodulation device 102. However, in the embodiments described herein, the controller 107 may generally be carried by and provided within the handle 118 of the neuromodulation device 102. The controller 107 is configured to initiate, terminate, and/or adjust operation of one or more electrodes provided by the end effector 114 directly and/or via the console 104. For example, the controller 107 can be configured to execute an automated control algorithm and/or to receive control instructions from an operator (e.g., surgeon or other medical professional or clinician). For example, the controller 107 and/or other components of the console 104 (e.g., processors, memory, etc.) can include a computer-readable medium carrying instructions, which when executed by the controller 107, causes the device 102 to perform certain functions (e.g., apply energy in a specific manner, detect impedance, detect temperature, detect nerve locations or anatomical structures, etc.). A memory includes one or more of various hardware devices for volatile and non-volatile storage, and can include both read-only and writable memory. For example, a memory can comprise random access memory (RAM), CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory.

The console 104 may further be configured to provide feedback to an operator before, during, and/or after a treatment procedure via evaluation/feedback algorithms 110. For example, the evaluation/feedback algorithms 110 can be configured to provide information associated with the location of nerves at the treatment site, the temperature of the tissue at the treatment site, and/or the effect of the therapeutic neuromodulation on the nerves at the treatment site. In certain embodiments, the evaluation/feedback algorithm 110 can include features to confirm efficacy of the treatment and/or enhance the desired performance of the system 100. For example, the evaluation/feedback algorithm 110, in conjunction with the controller 107, can be configured to monitor temperature at the treatment site during therapy and automatically shut off the energy delivery when the temperature reaches a predetermined maximum (e.g., when applying RF energy) or predetermined minimum (e.g., when applying cryotherapy). In other embodiments, the evaluation/feedback algorithm 110, in conjunction with the controller 107, can be configured to automatically terminate treatment after a predetermined maximum time, a predetermined maximum impedance rise of the targeted tissue (i.e., in comparison to a baseline impedance measurement), a predetermined maximum impedance of the targeted tissue), and/or other threshold values for biomarkers associated with autonomic function. This and other information associated with the operation of the system 100 can be communicated to the operator via a graphical user interface (GUI) 112 provided via a display on the console 104 and/or a separate display (not shown) communicatively coupled to the console 104, such as a tablet or monitor. The GUI 112 may generally provide operational instructions for the procedure, such as indicating when the device 102 is primed and ready to perform the treatment, and further providing status of therapy during the procedure, including indicating when the treatment is complete.

For example, as previously described, the end effector 114 and/or other portions of the system 100 can be configured to detect various parameters of the heterogeneous tissue at the target site to determine the anatomy at the target site (e.g., tissue types, tissue locations, vasculature, bone structures, foramen, sinuses, etc.), locate nerves and/or other structures, and allow for neural mapping. For example, the end effector 114 may be configured to detect impedance, dielectric properties, temperature, and/or other properties that indicate the presence of neural tissue or fibers in the target region, as described in greater detail herein.

As shown in FIG. 1A, the console 104 further includes a monitoring system 108 configured to receive data from the end effector 114 (i.e., detected electrical and/or thermal measurements of tissue at the target site), specifically sensed by appropriate sensors (e.g., temperature sensors and/or impedance sensors, or the like), and process this information to identify the presence of nerves, the location of nerves, neural activity at the target site, and/or other properties of the neural tissue, such a physiological properties (e.g., depth), bioelectric properties, and thermal properties. The nerve monitoring system 108 can be operably coupled to the electrodes and/or other features of the end effector 114 via signal wires (e.g., copper wires) that extend through the cable 120 and through the length of the shaft 116. In other embodiments, the end effector 114 can be communicatively coupled to the nerve monitoring system 108 using other suitable communication means.

The nerve monitoring system 108 can determine neural locations and activity before therapeutic neuromodulation to determine precise treatment regions corresponding to the positions of the desired nerves. The nerve monitoring system 108 can further be used during treatment to determine the effect of the therapeutic neuromodulation, and/or after treatment to evaluate whether the therapeutic neuromodulation treated the target nerves to a desired degree. This information can be used to make various determinations related to the nerves proximate to the target site, such as whether the target site is suitable for neuromodulation. In addition, the nerve monitoring system 108 can also compare the detected neural locations and/or activity before and after therapeutic neuromodulation, and compare the change in neural activity to a predetermined threshold to assess whether the application of therapeutic neuromodulation was effective across the treatment site. For example, the nerve monitoring system 108 can further determine electroneurogram (ENG) signals based on recordings of electrical activity of neurons taken by the end effector 114 before and after therapeutic neuromodulation. Statistically meaningful (e.g., measurable or noticeable) decreases in the ENG signal(s) taken after neuromodulation can serve as an indicator that the nerves were sufficiently ablated. Additional features and functions of the nerve monitoring system 108, as well as other functions of the various components of the console 104, including the evaluation/feedback algorithms 110 for providing real-time feedback capabilities for ensuring optimal therapy for a given treatment is administered, are described in at least U.S. Publication No. 2016/0331459 and U.S. Publication No. 2018/133460.

The neuromodulation device 102 provides access to target sites associated with peripheral nerves for the subsequent neuromodulation of such nerves and treatment of a corresponding peripheral neurological condition or disorder. The peripheral nervous system is one of two components that make up the nervous system of bilateral animals, with the other part being the central nervous system (CNS). The PNS consists of the nerves and ganglia outside the brain and spinal cord. The main function of the PNS is to connect the CNS to the limbs and organs, essentially serving as a relay between the brain and spinal cord and the rest of the body. The peripheral nervous system is divided into the somatic nervous system and the autonomic nervous system. In the somatic nervous system, the cranial nerves are part of the PNS with the exception of the optic nerve (cranial nerve II), along with the retina. The second cranial nerve is not a true peripheral nerve but a tract of the diencephalon. Cranial nerve ganglia originated in the CNS. However, the remaining ten cranial nerve axons extend beyond the brain and are therefore considered part of the PNS. The autonomic nervous system exerts involuntary control over smooth muscle and glands. The connection between CNS and organs allows the system to be in two different functional states: sympathetic and parasympathetic. Accordingly, the devices, systems, and methods of the present invention are useful in detecting, identifying, and precision targeting nerves associated with the peripheral nervous system for treatment of corresponding peripheral neurological conditions or disorders.

The peripheral neurological conditions or disorders may include, but are not limited to, chronic pain, movement disorders, epilepsy, psychiatric disorders, cardiovascular disorders, gastrointestinal disorders, genitourinary disorders, to name a few. For example, chronic pain may include headaches, complex regional pain syndrome, neuropathy, peripheral neuralgia, ischemic pain, failed back surgery syndrome, and trigeminal neuralgia. The movement disorders may include spasticity, Parkinson's disease, tremor, dystonia, Tourette syndrome, camptocormia, hemifacial spasm, and Meige syndrome. The psychiatric disorders may include depression, obsessive compulsive disorder, drug addiction, and anorexia/eating disorders. The functional restoration may include restoration of certain functions post traumatic brain injury, hearing impairment, and blindness. The cardiovascular disorders may include angina, heart failure, hypertension, peripheral vascular disorders, and stroke. The gastrointestinal disorders may include dysmotility and obesity. The genitourinary disorders may include painful bladder syndrome, interstitial cystitis, and voiding dysfunction.

For example, the system 100 may be used for the treatment of a cardiovascular disorder, such as arrhythmias or heart rhythm disorders, including, but not limited to, atrial fibrillation (AF or A-fib). Atrial fibrillation is an irregular and often rapid heart rate that can increase one's risk of stroke, heart failure, and other heart-related complications. Atrial fibrillation occurs when regions of cardiac tissue abnormally conduct electric signals to adjacent tissue, thereby disrupting the normal cardiac cycle and causing asynchronous rhythm. Atrial fibrillation symptoms often include heart palpitations, shortness of breath, and weakness. While episodes of atrial fibrillation can come and go, a person may develop atrial fibrillation that doesn't go away and thus will require treatment. Although atrial fibrillation itself usually isn't life-threatening, it is a serious medical condition that sometimes requires emergency treatment, as it may lead to complications. For example, atrial fibrillation is associated with an increased risk of heart failure, dementia, and stroke.

The normal electrical conduction system of the heart allows the impulse that is generated by the sinoatrial node (SA node) of the heart to be propagated to and stimulate the myocardium (muscular layer of the heart). When the myocardium is stimulated, it contracts. It is the ordered stimulation of the myocardium that allows efficient contraction of the heart, thereby allowing blood to be pumped to the body. In AF, the normal regular electrical impulses generated by the sinoatrial node in the right atrium of the heart are overwhelmed by disorganized electrical impulses usually originating in the roots of the pulmonary veins. This leads to irregular conduction of ventricular impulses that generate the heartbeat. In particular, during AF, the heart's two upper chambers (the atria) beat chaotically and irregularly, out of coordination with the two lower chambers (the ventricles) of the heart.

During atrial fibrillation, the regular impulses produced by the sinus node for a normal heartbeat are overwhelmed by rapid electrical discharges produced in the atria and adjacent parts of the pulmonary veins. Sources of these disturbances are either automatic foci, often localized at one of the pulmonary veins, or a small number of localized sources in the form of either a reentrant leading circle, or electrical spiral waves (rotors). These localized sources may be found in the left atrium near the pulmonary veins or in a variety of other locations through both the left or right atrium. There are three fundamental components that favor the establishment of a leading circle or a rotor: 1) slow conduction velocity of cardiac action potential; 2) short refractory period; and 3) small wavelength. Wavelength is the product of velocity and refractory period. If the action potential has fast conduction, with a long refractory period and/or conduction pathway shorter than the wavelength, an AF focus would not be established. In multiple wavelet theory, a wavefront will break into smaller daughter wavelets when encountering an obstacle, through a process called vortex shedding; but under proper conditions, such wavelets can reform and spin around a center, forming an AF focus.

The system 100 provides for the treatment of AF, in which the neuromodulation device 102 may provide access to and provide treatment of one or more target sites associated with nerves that correspond to, or are otherwise associated with, treating AF. For example, the device 102, in conjunction with the console 104, may detect, identify, and precision target cardiac tissue and subsequently deliver energy at a level or frequency sufficient to therapeutically modulate nerves associated with such cardiac tissue. The therapeutic modulation of such nerves is sufficient to disrupt the origin of the signals causing the AF and/or disrupt the conducting pathway for such signals.

Similar to the conduction system of the heart, a neural network exists which surrounds the heart and plays an important role in formation of the substrate of AF and when a trigger is originated, usually from pulmonary vein sleeves, AF occurs. This neural network includes ganglionated plexi (GP) located adjacent to pulmonary vein ostia which are under control of higher centers in normal people. For example, the heart is richly innervated by the autonomic nerves. The ganglion cells of the autonomic nerves are located either outside the heart (extrinsic) or inside the heart (intrinsic). Both extrinsic and intrinsic nervous systems are important for cardiac function and arrhythmogenesis. The vagal nerves include axons that come from various nuclei in the medulla. The extrinsic sympathetic nerves come from the paravertebral ganglia, including the superior cervical ganglion, middle cervical ganglion, the cervicothoracic (stellate) ganglion and the thoracic ganglia. The intrinsic cardiac nerves are found mostly in the atria, and are intimately involved in atrial arrhythmogenesis cardiovascular disorder, such as arrhythmias or heart rhythm disorders, including, but not limited to, atrial fibrillation. When GP become hyperactive owing to loss of inhibition from higher centers (e.g., in elderly), AF can occur.

The system 100 can be used to control hyperactive GP either by stimulating higher centers and their connections, such as vagus nerve stimulation, or simply by ablating GP. Accordingly, the device 102, in conjunction with the console 104, may detect and identify ganglionated plexus (GP) and further determine an energy level sufficient to therapeutically modulate or treat (i.e., ablate) the GP for the treatment of AF (i.e., surgically disrupting the origin of the signals causing the AF and disrupting the conducting pathway for such signals) while minimizing and/or preventing collateral damage to surrounding or adjacent non-neural tissue including bloods vessels and bone and non-targeted neural tissue. It should be noted that other nerves and/or cardiac tissue, or other structures, known to have an impact on or cause AF, may be targeted by the system 100, including, but not limited to, pulmonary veins (e.g., pulmonary vein isolation upon creation of lesions around PV ostia to prevent triggers from reaching atrial substrate).

In addition to treating arrhythmias, the system 100 may also be used for the treatment of other cardiovascular-related conditions, particularly those involving the kidney. The kidneys play a significant role in the progression of CHF, as well as in Chronic Renal Failure (CRF), End-Stage Renal Disease (ESRD), hypertension (pathologically high blood pressure), and other cardio-renal diseases.

The functions of the kidney can be summarized under three broad categories: filtering blood and excreting waste products generated by the body's metabolism; regulating salt, water, electrolyte and acid-base balance; and secreting hormones to maintain vital organ blood flow. Without properly functioning kidneys, a patient will suffer water retention, reduced urine flow and an accumulation of waste toxins in the blood and body. These conditions resulting from reduced renal function or renal failure (kidney failure) are believed to increase the workload of the heart.

For example, in a CHF patient, renal failure will cause the heart to further deteriorate as the water build-up and blood toxins accumulate due to the poorly functioning kidneys and, in turn, cause the heart further harm. CHF is a condition that occurs when the heart becomes damaged and reduces blood flow to the organs of the body. If blood flow decreases sufficiently, kidney function becomes impaired and results in fluid retention, abnormal hormone secretions and increased constriction of blood vessels. These results increase the workload of the heart and further decrease the capacity of the heart to pump blood through the kidney and circulatory system. This reduced capacity further reduces blood flow to the kidney. It is believed that progressively decreasing perfusion of the kidney is a principal non-cardiac cause perpetuating the downward spiral of CHF. Moreover, the fluid overload and associated clinical symptoms resulting from these physiologic changes are predominant causes for excessive hospital admissions, reduced quality of life, and overwhelming costs to the health care system due to CHF.

End-stage renal disease is another condition at least partially controlled by renal neural activity. There has been a dramatic increase in patients with ESRD due to diabetic nephropathy, chronic glomerulonephritis and uncontrolled hypertension. Chronic renal failure (CRF) slowly progresses to ESRD. CRF represents a critical period in the evolution of ESRD. The signs and symptoms of CRF are initially minor, but over the course of 2-5 years, become progressive and irreversible. While some progress has been made in combating the progression to, and complications of, ESRD, the clinical benefits of existing interventions remain limited.

Arterial hypertension is a major health problem worldwide. Treatment-resistant hypertension is defined as the failure to achieve target blood pressure despite the concomitant use of maximally tolerated doses of three different antihypertensive medications, including a diuretic. Treatment-resistant hypertension is associated with considerable morbidity and mortality. Patients with treatment-resistant hypertension have markedly increased cardiovascular morbidity and mortality, facing an increase in the risk of myocardial infarction (MI), stroke, and death compared to patients whose hypertension is adequately controlled.

The autonomic nervous system is recognized as an important pathway for control signals that are responsible for the regulation of body functions critical for maintaining vascular fluid balance and blood pressure. The autonomic nervous system conducts information in the form of signals from the body's biologic sensors such as baroreceptors (responding to pressure and volume of blood) and chemoreceptors (responding to chemical composition of blood) to the central nervous system via its sensory fibers. It also conducts command signals from the central nervous system that control the various innervated components of the vascular system via its motor fibers.

It is known from clinical experience and research that an increase in renal sympathetic nerve activity leads to vasoconstriction of blood vessels supplying the kidney, decreased renal blood flow, decreased removal of water and sodium from the body, and increased renin secretion. It is also known that reduction of sympathetic renal nerve activity, e.g., via denervation, may reverse these processes.

The renal sympathetic nervous system plays a critical influence in the pathophysiology of hypertension. The adventitia of the renal arteries has efferent and afferent sympathetic nerves. Renal sympathetic activation via the efferent nerves initiates a cascade resulting in elevated blood pressure. Efferent sympathetic outflow leads to vasoconstriction with a subsequent reduction in glomerular blood flow, a lowering of the glomerular filtration rate, release of renin by the juxtaglomerular cells, and the subsequent activation of the renin-angiotensin-aldosterone axis leading to increased tubular reabsorption of sodium and water. Decreased glomerular filtration rate also prompts additional systemic sympathetic release of catecholamines. As a consequence, blood pressure increases by a rise in total blood volume and increased peripheral vascular resistance.

The system 100 can be used for the treatment of cardio-renal diseases, including hypertension, by providing renal neuromodulation and/or denervation. For example, the device 102 may be placed at one or more target sites associated with renal nerves other neural fibers that contribute to renal neural function, or other neural features. For example, the device 102, in conjunction with the console 104, may detect, identify, and precision target renal nerve tissue and subsequently deliver energy at a level or frequency sufficient to therapeutically modulate nerves associated with such renal tissue. The therapeutic modulation of such renal nerves and/or renal tissue is sufficient to completely block or denervate the target neural structures and/or disrupt renal nervous activity, while minimizing and/or preventing collateral damage to surrounding or adjacent non-neural tissue including bloods vessels and bone and non-targeted neural tissue.

FIG. 5 is a side view of one embodiment of a handheld device for providing therapeutic neuromodulation consistent with the present disclosure. As previously described, the device 102 includes an end effector (not shown) transformable between a collapsed/retracted configuration and an expanded deployed configuration, a shaft 116 operably associated with the end effector, and a handle 118 operably associated with the shaft 116. The handle 118 includes at least a first mechanism 126 for deployment of the end effector from collapsed/retracted configuration to the expanded, deployed configuration, and a second mechanism 128, separate from the first mechanism 124, for control of energy output by the end effector, specifically electrodes or other energy elements provided by the end effector. The handheld device 102 may further include an auxiliary line 121, which may provide a fluid connection between a fluid source, for example, and the shaft 116 such that fluid may be provided to a target site via the distal end of the shaft 116. In some embodiments, the auxiliary line 121 may provide a connection between a vacuum source and the shaft 116, such that the device 102 may include suction capabilities (via the distal end of the shaft 116).

FIG. 6 is an enlarged, perspective view of one embodiment of an end effector 214 consistent with the present disclosure. As shown, the end effector 214 is generally positioned at a distal portion 116b of the shaft 116. The end effector 214 is transformable between a low-profile delivery state to facilitate intraluminal delivery of the end effector 214 to a treatment site and an expanded state, as shown. The end effector 214 includes a plurality of struts 240 that are spaced apart from each other to form a frame or basket 242 when the end effector 214 is in the expanded state. The struts 240 can carry one or more energy delivery elements, such as a plurality of electrodes 244. In the expanded state, the struts 240 can position at least two of the electrodes 244 against tissue at a target site within a particular region. The electrodes 244 can apply bipolar or multi-polar RF energy to the target site to therapeutically modulate nerves associated with a peripheral neurological condition or disorder. In various embodiments, the electrodes 244 can be configured to apply pulsed RF energy with a desired duty cycle (e.g., 1 second on/0.5 seconds off) to regulate the temperature increase in the target tissue.

In the embodiment illustrated in FIG. 6, the basket 242 includes eight branches 246 spaced radially apart from each other to form at least a generally spherical structure, and each of the branches 246 includes two struts 240 positioned adjacent to each other. In other embodiments, however, the basket 242 can include fewer than eight branches 246 (e.g., two, three, four, five, six, or seven branches) or more than eight branches 246. In further embodiments, each branch 246 of the basket 242 can include a single strut 240, more than two struts 240, and/or the number of struts 240 per branch can vary. In still further embodiments, the branches 246 and struts 240 can form baskets or frames having other suitable shapes for placing the electrodes 244 in contact with tissue at the target site. For example, when in the expanded state, the struts 240 can form an ovoid shape, a hemispherical shape, a cylindrical structure, a pyramid structure, and/or other suitable shapes.

The end effector 214 can further include an internal or interior support member 248 that extends distally from the distal portion 116b of the shaft 116. A distal end portion 250 of the support member 248 can support the distal end portions of the struts 240 to form the desired basket shape. For example, the struts 240 can extend distally from the distal potion 116b of the shaft 116 and the distal end portions of the struts 240 can attach to the distal end portion 250 of the support member 248. In certain embodiments, the support member 248 can include an internal channel (not shown) through which electrical connectors (e.g., wires) coupled to the electrodes 244 and/or other electrical features of the end effector 214 can run. In various embodiments, the internal support member 248 can also carry an electrode (not shown) at the distal end portion 250 and/or along the length of the support member 248.

The basket 242 can transform from the low-profile delivery state to the expanded state (shown in FIG. 6) by either manually manipulating a handle of the device 102, interacting with the first mechanism 126 for deployment of the end effector 214 from collapsed/retracted configuration to the expanded, deployed configuration, and/or other feature at the proximal portion of the shaft 116 and operably coupled to the basket 242. For example, to move the basket 242 from the expanded state to the delivery state, an operator can push the support member 248 distally to bring the struts 240 inward toward the support member 248. An introducer or guide sheath (not shown) can be positioned over the low-profile end effector 214 to facilitate intraluminal delivery or removal of the end effector 214 from or to the target site. In other embodiments, the end effector 214 is transformed between the delivery state and the expanded state using other suitable means, such as the first mechanism 126, as will be described in greater detail herein.

The individual struts 240 can be made from a resilient material, such as a shape-memory material (e.g., Nitinol) that allows the struts 240 to self-expand into the desired shape of the basket 242 when in the expanded state. In other embodiments, the struts 240 can be made from other suitable materials and/or the end effector 214 can be mechanically expanded via a balloon or by proximal movement of the support member 248. The basket 242 and the associated struts 240 can have sufficient rigidity to support the electrodes 244 and position or press the electrodes 244 against tissue at the target site. In addition, the expanded basket 242 can press against surrounding anatomical structures proximate to the target site and the individual struts 240 can at least partially conform to the shape of the adjacent anatomical structures to anchor the end effector 214 at the treatment site during energy delivery. In addition, the expansion and conformability of the struts 240 can facilitate placing the electrodes 244 in contact with the surrounding tissue at the target site.

At least one electrode 244 is disposed on individual struts 240. In the illustrated embodiment, two electrodes 244 are positioned along the length of each strut 240. In other embodiments, the number of electrodes 244 on individual struts 240 be only one, more than two, zero, and/or the number of electrodes 244 on the different struts 240 can vary. The electrodes 244 can be made from platinum, iridium, gold, silver, stainless steel, platinum-iridium, cobalt chromium, iridium oxide, polyethylenedioxythiophene ("PEDOT"), titanium, titanium nitride, carbon, carbon nanotubes, platinum grey, Drawn Filled Tubing ("DFT") with a silver core made by Fort Wayne Metals of Fort Wayne, Ind., and/or other suitable materials for delivery RF energy to target tissue.

In certain embodiments, each electrode 444 can be operated independently of the other electrodes 244. For example, each electrode can be individually activated and the polarity and amplitude of each electrode can be selected by an operator or a control algorithm (e.g., executed by the controller 107 of FIG. 1A). Various embodiments of such independently controlled electrodes 244 are described in greater detail herein. The selective independent control of the electrodes 244 allows the end effector 214 to deliver RF energy to highly customized regions and to further create multiple micro-lesions to selectively modulate a target neural structure by effectively causing multi-point interruption of a neural signal due to the multiple micro-lesions. For example, a select portion of the electrodes 244 can be activated to target neural fibers in a specific region while the other electrodes 244 remain inactive. In certain embodiments, for example, electrodes 244 may be activated across the portion of the basket 242 that is adjacent to tissue at the target site, and the electrodes 244 that are not proximate to the target tissue can remain inactive to avoid applying energy to non-target tissue. Such configurations facilitate selective therapeutic modulation of nerves along a portion of a target site without applying energy to structures in other portions of the target site.

The electrodes 244 can be electrically coupled to an RF generator (e.g., the generator 106 of FIG. 1A) via wires (not shown) that extend from the electrodes 244, through the shaft 116, and to the RF generator. When each of the electrodes 244 is independently controlled, each electrode 244 couples to a corresponding wire that extends through the shaft 116. In other embodiments, multiple electrodes 244 can be controlled together and, therefore, multiple electrodes 244 can be electrically coupled to the same wire extending through the shaft 116. The RF generator and/or components operably coupled (e.g., a control module) thereto can include custom algorithms to control the activation of the electrodes 244. For example, the RF generator can deliver RF power at about 200-300 W to the electrodes 244, and do so while activating the electrodes 244 in a predetermined pattern selected based on the position of the end effector 214 relative to the treatment site and/or the identified locations of the target nerves. In other embodiments, the RF generator delivers power at lower levels (e.g., less than 15 W, 15-50 W, 50-150 W, etc.) and/or higher power levels.

The end effector 214 can further include one or more sensors 252 (e.g., temperature sensors, impedance sensors, etc.) disposed on the struts 240 and/or other portions of the end effector 214 and configured to sense/detect one or more properties associated with neural tissue. For example, temperature sensors are configured to detect the temperature adjacent thereto. The sensors 252 can be electrically coupled to a console (e.g., the console 104 of FIG. 1A) via wires (not shown) that extend through the shaft 116. In various embodiments, the sensors 252 can be positioned proximate to the electrodes 244 to detect various properties of the neural tissue and/or the treatment associated therewith. As will be described in greater detail herein, the sensed data can be provided to the console 104, wherein such data is generally related to at least the presence of neural tissue at a given location (in which electrodes 244 and/or sensors 252 are present at the target site(s)) and morphology of the neural tissue, including physiological and bioelectric properties. In turn, the console 104 (via the controller 107, monitoring system 108, and evaluation/feedback algorithms 110) is configured to process such data and determine the presence of neural tissue and the type of neural tissue, as well as other properties, such as depth, for example. The console (via the controller 107, monitoring system 108, and evaluation/feedback algorithms 110) is further configured to determine the level of therapeutic energy to be delivered by one or more the plurality of electrodes such that the energy delivered at each position by the one or more electrodes is sufficient to ablate neural tissue at each position and minimize and/or prevent damage to a surrounding or adjacent structure, such as an artery or arterial wall adjacent to the neural tissue at each position.

Such sensed data can further include feedback data associated with the effect of the therapeutic neuromodulation on neural tissue at any given location. For example, feedback data (sensed during therapeutic neuromodulation of neural tissue) may be associated with efficacy of ablation of the neural tissue at each position during and/or after delivery of initial energy from one or more of the plurality of electrodes. Accordingly, in certain embodiments, the console 104 (via the controller 107, monitoring system 108, and evaluation/feedback algorithms 110) is configured to process such feedback data to determine if certain properties of the neural tissue undergoing treatment (i.e., tissue temperature, tissue impedance, etc.) reach predetermined thresholds for irreversible tissue damage. The controller 107 can tune energy output individually for the one or more electrodes after an initial level of energy has been delivered based, at least in part, on feedback data. For example, once the threshold is reached, the application of therapeutic neuromodulation energy can be terminated to allow the tissue to remain intact. In certain embodiments, the energy delivery can automatically be tuned based on an evaluation/feedback algorithm (e.g., the evaluation/feedback algorithm 110 of FIG. 1A) stored on a console (e.g., the console 104 of FIG. 1A) operably coupled to the end effector 214.

FIGS. 7A-7F are various views of another embodiment of an end effector 314 consistent with the present disclosure. As generally illustrated, the end effector 314 is a multi-segmented end effector, which includes at least a first segment 322 and a second segment 324 spaced apart from one another. The first segment 322 is generally positioned closer to a distal portion of the shaft 116, and is thus sometimes referred to herein as the proximal segment 322, while the second segment 324 is generally positioned further from the distal portion of the shaft 116 and is thus sometimes referred to herein as the distal segment 324. Each of the first and second segments 322 and 324 is transformable between a retracted configuration, which includes a low-profile delivery state and a deployed configuration, which includes an expanded state, as shown in the figures. The end effector 314 is generally designed to be positioned within a nasal region of the patient for the treatment of a rhinosinusitis condition while minimizing or avoiding collateral damage to surrounding tissue, such as blood vessels. In particular, the end effector 314 is configured to be advanced within the nasal cavity and be positioned at one or more target sites generally associated with postganglionic parasympathetic fibers that innervate the nasal mucosa. In turn, the end effector 314 is configured to therapeutically modulate the postganglionic parasympathetic nerves.

It should be noted, however, that an end effector consistent with the present disclosure may be multi-segmented in a similar fashion as end effector 314 and may be used to provide treatment in other regions of the patient outside of the nasal cavity and thus is not limited to the particular design/configuration as the end effector 314 nor the intended treatment site (e.g., nasal cavity). Rather, other multi-segmented designs are contemplated for use in particular regions of a patient, particularly regions in which the use of multiple and distinct segments would be advantageous, as is the case with the end effector 314 design due to the anatomy of the nasal cavity.

FIG. 7A is an enlarged, perspective view of the multi-segment end effector illustrating the first (proximal) segment 322 and second (distal) segment 324. FIG. 7B is an exploded, perspective view of the multi-segment end effector 314. FIG. 7C is an enlarged, top view of the multi-segment end effector 314. FIG. 7D is an enlarged, side view of the multi-segment end effector 314. FIG. 7E is an enlarged, front (proximal facing) view of the first (proximal) segment 322 of the multi-segment end effector 314 and FIG. 7F is an enlarged, front (proximal facing) view of the second (distal) segment 324 of the multi-segment end effector 314.

As illustrated, the first segment 322 includes at least a first set of flexible support elements, generally in the form of wires, arranged in a first configuration, and the second segment 324 includes a second set of flexible support elements, also in the form of wires, arranged in a second configuration. The first and second sets of flexible support elements include composite wires having conductive and elastic properties. For example, in some embodiments, the composite wires include a shape memory material, such as Nitinol. The flexible support elements may further include a highly lubricious coating, which may allow for desirable electrical insulation properties as well as desirable low friction surface finish. Each of the first and second segments 322, 324 is transformable between a retracted configuration and an expanded deployed configuration such that the first and second sets of flexible support elements are configured to position one or more electrodes provided on the respective segments (see electrodes 336 in FIGS. 7E and 7F) into contact with one or more target sites when in the deployed configuration.

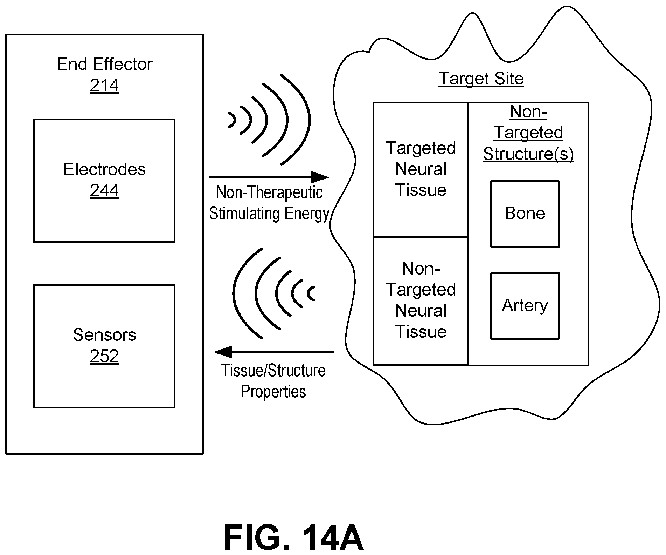

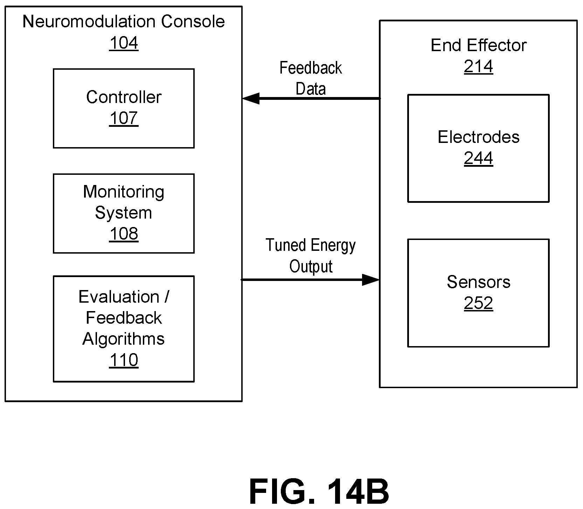

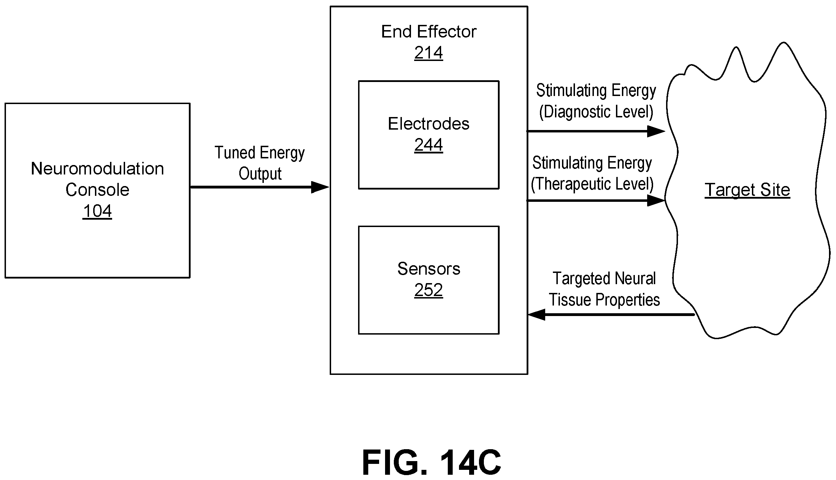

FIGS. 14A, 14B, and 14C are block diagrams illustrating the process of sensing, via an end effector, data associated with one or more target sites, notably the presence of neural tissue at the one more target sites, as well as the morphology of neural tissue at multiple locations and the subsequent processing of such data (via the controller 107, monitoring system 108, and evaluation/feedback algorithms 110) to determining the type of neural tissue at the target site, as well as other properties, such as depth of the target tissue, and further determining a level of therapeutic energy to be delivered by one or more of a plurality of electrodes of the end effector based on the determined type of neural tissue. The tuned energy output results in delivery of energy at each position by the one or more electrodes sufficient to ablate neural tissue at each position and minimize and/or prevent damage to a surrounding or adjacent structure, such as an artery or arterial wall adjacent to the neural tissue at each position.

It should be noted that, while the block diagrams of FIGS. 14A, 14B, and 14C include reference to end effector 214, other end effector embodiments, including end effector 314, are similarly configured with respect to sensing data associated with at least the presence of neural tissue and other properties of the neural tissue, including neural tissue depth. Accordingly, the following process is not limited to end effector 214.

FIG. 14A is a block diagram illustrating delivery of non-therapeutic energy from electrodes 244 of the end effector at a frequency for locating neural tissue and sensing one or more properties associated with the neural tissue in response to the non-therapeutic energy.

As previously described, the handheld treatment device includes an end effector comprising a micro-electrode array arranged about a plurality of struts. For example, end effector 214 includes a plurality of struts 240 that are spaced apart from each other to form a frame or basket 242 when the end effector 214 is in the expanded state. The struts 240 include a plurality of energy delivery elements, such as a plurality of electrodes 244. In the expanded state, each of the plurality of struts is able to conform to and accommodate an anatomical structure at a target site. When positioned, the struts may contact multiple locations along multiple portions of a target site and thereby position one or more electrodes 244 against tissue at a target site. At least a subset of electrodes is configured to sense the presence of neural tissue at a respective position of each of the electrodes, as well as morphology of the neural tissue, and further convey such data to the console 104. For example, in one embodiment, a subset of the plurality of electrodes is configured to deliver non-therapeutic stimulating energy to respective positions at a frequency for locating neural tissue and further sensing one or more properties, including at least the presence and/or depth of the neural tissue in response to the non-therapeutic stimulating energy. The properties may further include, but are not limited to, at least one of physiological properties, bioelectric properties, and thermal properties of the neural tissue, as well as other structures adjacent to the neural tissue.

For example, upon delivering non-therapeutic stimulating energy (via one or more electrodes 244) to respective positions, various properties of the tissue at the one or more target sites can be detected. This information can then be transmitted to the console 104, particularly the controller 107, monitoring system 108, and evaluation/feedback algorithms 110 to determine the anatomy at the target site (e.g., tissue types, tissue locations, vasculature, bone structures, foramen, sinuses, etc.), locate neural tissue, differentiate between different types of neural tissue, and map the anatomical and/or neural structure at the target site. For example, the end effector 214 can be used to detect resistance, complex electrical impedance, dielectric properties, temperature, and/or other properties that indicate the presence of neural fibers and/or other anatomical structures in the target region. In certain embodiments, the end effector 214, together with the console 104 components, can be used to determine resistance (rather than impedance) of the tissue (i.e., the load) to more accurately identify the characteristics of the tissue. For example, the evaluation/feedback algorithms 110 can determine resistance of the tissue by detecting the actual power and current of the load (e.g., via the electrodes 244).

In some embodiments, the system 100 provides resistance measurements with a high degree of accuracy and a very high degree of precision, such as precision measurements to the hundredths of an Ohm (e.g., 0.01Ω) for the range of 1-50Q. The high degree of resistance detection accuracy provided by the system 100 allows for the detection sub-microscale structures, including the firing of neural tissue, differences between neural tissue and other anatomical structures (e.g., blood vessels), and even different types of neural tissue. This information can be analyzed by the evaluation/feedback algorithms 110 and/or the controller 107 and communicated to the operator via a high resolution spatial grid (e.g., on the display 112) and/or other type of display to identify neural tissue and other anatomy at the treatment site and/or indicate predicted neuromodulation regions based on the ablation pattern with respect to the mapped anatomy.

As previously described, in certain embodiments, each electrode 244 can be operated independently of the other electrodes 244. For example, each electrode can be individually activated and the polarity and amplitude of each electrode can be selected by an operator or a control algorithm executed by the controller 107. The selective independent control of the electrodes 244 allows the end effector 214 to detect information (i.e., the presence of neural tissue, depth of neural tissue, and other physiological and bioelectrical properties) and subsequently deliver RF energy to highly customized regions. For example, a select portion of the electrodes 244 can be activated to target specific neural fibers in a specific region while the other electrodes 244 remain inactive. In addition, the electrodes 244 can be individually activated to stimulate or therapeutically modulate certain regions in a specific pattern at different times (e.g., via multiplexing), which facilitates detection of anatomical parameters across a zone of interest and/or regulated therapeutic neuromodulation.

As previously described, the system 100 can determine the locations and/or morphology of neural tissue and/or other anatomical structures prior to therapy such that the therapeutic neuromodulation can be applied to precise regions including target neural tissue, while avoiding negative effects on non-target structures, such as non-neural tissue (e.g., blood vessels) as well as and non-target neural tissue. The system 100 can detect various bioelectrical parameters in an interest zone to determine the location and morphology of various neural tissue (e.g., different types of neural tissue, neuronal directionality, etc.) and/or other tissue (e.g., glandular structures, vessels, bony regions, etc.). In some embodiments, the system 100 is configured to measure bioelectric potential.

To do so, one or more of the electrodes 244 is placed in contact with an epithelial surface at a region of interest (e.g., a treatment site). Electrical stimuli (e.g., constant or pulsed currents at one or more frequencies) are applied to the tissue by one or more electrodes 244 at or near the treatment site, and the voltage and/or current differences at various different frequencies between various pairs of electrodes 244 of the end effector 214 may be measured to produce a spectral profile or map of the detected bioelectric potential, which can be used to identify different types of tissues (e.g., vessels, neural tissue, and/or other types of tissue) in the region of interest. For example, current (i.e., direct or alternating current) can be applied to a pair of electrodes 244 adjacent to each other and the resultant voltages and/or currents between other pairs of adjacent electrodes 244 are measured. It will be appreciated that the current injection electrodes 244 and measurement electrodes 244 need not be adjacent, and that modifying the spacing between the two current injection electrodes 244 can affect the depth of the recorded signals. For example, closely-spaced current injection electrodes 244 provided recorded signals associated with tissue deeper from the surface of the tissue than further spaced apart current injection electrodes 244 that provide recorded signals associated with tissue at shallower depths. Recordings from electrode pairs with different spacings may be merged to provide additional information on depth and localization of anatomical structures.

Further, complex impedance and/or resistance measurements of the tissue at the region of interest can be detected directly from current-voltage data provided by the bioelectric potential measurements while differing levels of frequency currents are applied to the tissue (e.g., via the end effector 114), and this information can be used to map the neural and anatomical structures by the use of frequency differentiation reconstruction. Applying the stimuli at different frequencies will target different stratified layers or cellular bodies or clusters. At high signal frequencies (e.g., electrical injection or stimulation), for example, cell membranes of the neural tissue do not impede current flow, and the current passes directly through the cell membranes. In this case, the resultant measurement (e.g., impedance, resistance, capacitance, and/or induction) is a function of the intracellular and extracellular tissue and liquids. At low signal frequencies, the membranes impede current flow to provide different defining characteristics of the tissues, such as the shapes of the cells or cell spacing. The stimulation frequencies can be in the megahertz range, in the kilohertz range (e.g., 400-500 kHz, 450-480 kHz, etc.), and/or other frequencies attuned to the tissue being stimulated and the characteristics of the device being used. The detected complex impedance or resistances levels from the zone of interest can be displayed to the user (e.g., via the display 112) to visualize certain structures based on the stimulus frequency.

Further, the inherent morphology and composition of the anatomical structures within a given region or zone of a patient's body react differently to different frequencies and, therefore, specific frequencies can be selected to identify very specific structures. For example, the morphology or composition of targeted structures for anatomical mapping may depend on whether the cells of tissue or other structure are membranonic, stratified, and/or annular. In various embodiments, the applied stimulation signals can have predetermined frequencies attuned to specific neural tissue, such as the level of myelination and/or morphology of the myelination. For example, second axonal parasympathetic structures are poorly myelinated than sympathetic nerves or other structures and, therefore, will have a distinguishable response (e.g., complex impedance, resistance, etc.) with respect to a selected frequency than sympathetic nerves. Accordingly, applying signals with different frequencies to the target site can distinguish the targeted parasympathetic nerves from the non-targeted sensory nerves, and therefore provide highly specific target sites for neural mapping before or after therapy and/or neural evaluation post-therapy.

In some embodiments, the neural and/or anatomical mapping includes measuring data at a region of interest with at least two different frequencies to identify certain anatomical structures such that the measurements are taken first based on a response to an injection signal having a first frequency and then again based on an injection signal having a second frequency different from the first. For example, there are two frequencies at which hypertrophied (i.e., disease-state characteristics) sub-mucosal targets have a different electrical conductivity or permittivity compared to "normal" (i.e., healthy) tissue. Complex conductivity may be determined based on one or more measured physiological parameters (e.g., complex impedance, resistance, dielectric measurements, dipole measurements, etc.) and/or observance of one or more confidently known attributes or signatures. Furthermore, the system 100 can also apply neuromodulation energy via the electrodes 244 at one or more predetermined frequencies attuned to a target neural structure to provide highly targeted ablation of the selected neural structure associated with the frequency(ies). This highly targeted neuromodulation also reduces the collateral effects of neuromodulation therapy to non-target sites/structures (e.g., blood vessels) because the targeted signal (having a frequency tuned to a target neural structure) will not have the same modulating effects on the non-target structures.

Accordingly, bioelectric properties, such as complex impedance and resistance, can be used by the system 100 before, during, and/or after neuromodulation therapy to guide one or more treatment parameters. For example, before, during, and/or after treatment, impedance or resistance measurements may be used to confirm and/or detect contact between one or more electrodes 244 and the adjacent tissue. The impedance or resistance measurements can also be used to detect whether the electrodes 244 are placed appropriately with respect to the targeted tissue type by determining whether the recorded spectra have a shape consistent with the expected tissue types and/or whether serially collected spectra were reproducible. In some embodiments, impedance or resistance measurements may be used to identify a boundary for the treatment zone (e.g., specific neural tissue that are to be disrupted), anatomical landmarks, anatomical structures to avoid (e.g., vascular structures or neural tissue that should not be disrupted), and other aspects of delivering energy to tissue.

The bioelectric information can be used to produce a spectral profile or map of the different anatomical features tissues at the target site, and the anatomical mapping can be visualized in a 3D or 2D image via the display 112 and/or other user interface to guide the selection of a suitable treatment site. This neural and anatomical mapping allows the system 100 to accurately detect and therapeutically modulate neural fibers associated with certain neurological conditions or disorders to be treated. In addition, anatomical mapping also allows the clinician to identify certain structures that the clinician may wish to avoid during therapeutic neural modulation (e.g., certain arteries). The neural and anatomical bioelectric properties detected by the system 100 can also be used during and after treatment to determine the real-time effect of the therapeutic neuromodulation on the treatment site. For example, the evaluation/feedback algorithms 110 can also compare the detected neural locations and/or activity before and after therapeutic neuromodulation, and compare the change in neural activity to a predetermined threshold to assess whether the application of therapeutic neuromodulation was effective across the treatment site.

FIG. 14B is a block diagram illustrating communication of sensor data from the handheld device 102 to the controller and subsequent tuning, via the controller, of energy output based on the sensor data for precision targeting of neural tissue. As shown, the end effector 214 communicates the tissue data (i.e., detection of neural tissue, as well as the morphology (i.e., physiological and bioelectrical properties) of neural tissue at respective locations at the target site(s)) to the console 104. In turn, console 104 (via the controller 107, monitoring system 108, and evaluation/feedback algorithms 110) is configured to process such data and determine a type of neural tissue at the target site, as well as other properties, such as depth of the neural tissue. The console 104 (via the controller 107, monitoring system 108, and evaluation/feedback algorithms 110) is further configured to determine a level of energy to be delivered by one or more of the plurality of electrodes based, at least in part, on the identified type of neural tissue, such that the energy delivered at each position by the one or more electrodes is sufficient to ablate neural tissue at each position and minimize and/or prevent damage to a surrounding or adjacent structure, such as an artery or arterial wall adjacent to the neural tissue at each position.

FIG. 14C is a block diagram illustrating delivery of energy (at diagnostic or therapeutic levels) to the target site specifically targeting neural tissue based on the tuned energy output from the controller. In one example, the level of energy output from the end effector may be at a diagnostic energy level useful for diagnosing a neurological condition based on feedback data captured by electrodes and/or sensors of the end effector. In another example, the level of energy output from the end effector may be at a therapeutic energy level sufficient to therapeutically modulate (e.g. ablate) the neural tissue while minimizing and/or preventing damage to a surrounding or adjacent structure, such as an artery or arterial wall adjacent to the neural tissue. Accordingly, the energy delivered is tuned to a specific level based on a prior determination of the type of neural tissue, such that resulting micro-lesions accommodate the specific neural tissue to be treated and the precise location (e.g., depth) of the neural tissue at a given site, which is important to prevent excessive damage and destruction to non-neural tissue when generating micro-lesions. In that manner, the invention solves the problem of causing unnecessary collateral damage to non-neural tissue when generating micro-lesions during a neuromodulation procedure, as the systems and methods are able to detect and identify nerve type, location, and other properties of intended neural tissue prior to neuromodulation treatment.

Accordingly, the neuromodulation energy can be applied to the tissue in a highly targeted manner and form micro-lesions to selectively modulate the target structure, while avoiding non-targeted structures (e.g., other neural tissue, blood vessels, etc.) and allowing the surrounding tissue structure to remain healthy for effective wound healing. In that manner, the present invention provides a neuromodulation treatment system providing unprecedented control to an operator so that an operator can perform an accurate, minimally invasive, and localized application of energy to target sites to cause multi-point interruption of neural signal without causing collateral damage or disruption to other, non-targeted tissue (e.g., blood vessels, other non-targeted neural tissue, etc.). More specifically, based on an initial feedback data from the treatment device prior to delivery of therapeutic energy, the specific type of neural tissue is identified as well as other properties of the neural tissue. In turn, the controller allows for the tuning of energy output such that resulting micro-lesions accommodate variations in neural tissue, including the specific type of neural tissue, depth of neural tissue, as well as other physiological, bioelectric, and/or thermal properties, which is critical in preventing excessive damage and destruction to surrounding structures during a treatment procedure.

Therefore, the system of the present invention provides a user-friendly, non-invasive means of treating rhinosinusitis conditions, including precise and focused application of energy to the intended target sites for therapeutic modulation of intended neural tissue, specifically the creation of multiple micro-lesion resulting in multi-point interruption of neural signals, without causing collateral damage or disruption to non-neural tissue (e.g., blood vessels, etc.) or non-targeted neural tissue. The creation of micro-lesions along a neural tissue is critical for various reasons. The creation of multiple micro-lesions, and resulting multi-point interruption, accounts for the various entry points and pathways associated with certain nerves, including the microforamina. The multiple micro-lesions further creates more than a single break along a nerve pathway, which is vital for longevity of the effect of treatment as it prevents functional alignment and regeneration of the nerve tissue. Furthermore, the controller is able to tailor the size and shape of any given micro-lesion at a respective location along a neural tissue by way of independent control of electrodes of the electrode array, thereby further enhancing the effectiveness of treatment by accounting for variations in neural tissue depth, for example, at any given location.

It should further be noted that, with reference to FIG. 14C, the end effector 114 can continue to sense tissue properties during and/or after treatment. Such sensed data from the end effector 214 can further include feedback data associated with the effect of either the stimulating energy at the diagnostic level or stimulating energy at the therapeutic level on neural tissue at any given location. For example, feedback data (sensed during delivery of stimulation energy at a diagnostic level to neural tissue) may be associated with bioelectrical properties of the neural tissue in response to a certain frequency of energy for eliciting such response during delivery of the stimulation energy from one or more of the plurality of electrodes 244. Such feedback data can be processed via the console 104 which, in turn, can provide diagnostic feedback to the operator. Similarly, feedback data (sensed during therapeutic neuromodulation of neural tissue) may be associated with efficacy of ablation of the neural tissue at each position during and/or after delivery of initial energy from one or more of the plurality of electrodes 244. Accordingly, in certain embodiments, the console 104 (via the controller 107, monitoring system 108, and evaluation/feedback algorithms 110) is configured to process such feedback data to determine if certain properties of the neural tissue undergoing treatment (i.e., tissue temperature, tissue impedance, etc.) reach predetermined thresholds for irreversible tissue damage.

The electrodes 244 are configured to be independently controlled and activated by the controller 107 (in conjunction with the evaluation/feedback algorithms 110) to thereby deliver energy independent of one another. Accordingly, the controller 107 can tune energy output individually for the one or more electrodes 244 after an initial level of energy has been delivered based, at least in part, on feedback data. For example, once the threshold is reached, the application of therapeutic neuromodulation energy can be terminated to allow the tissue to remain intact. In other embodiments, if the threshold has not been reached, the controller can maintain, reduce, or increase energy output to a given electrode 244 until such threshold is reached. Accordingly, the energy delivery of any given electrode 244 can automatically be tuned based on an evaluation/feedback algorithm (e.g., the evaluation/feedback algorithm 110 of FIG. 1A) stored on a console (e.g., the console 104 of FIG. 1A) operably coupled to the end effector 214. For example, at least some of the electrodes 244 may have different levels of energy to be delivered at respective positions sufficient to ablate neural tissue at the respective positions based on the feedback data received for the respective locations.

For example, in some embodiments, the controller 107 is configured to tune energy output from each of the plurality of electrodes 244 after an initial level of energy has been delivered based, at least in part, on feedback data received. The feedback data may be associated with efficacy of ablation of the neural tissue at each position during and/or after delivery of initial energy from each of the plurality of electrodes. The feedback data includes one or more properties associated with neural tissue at respective positions. The one or more properties may include, but are not limited to, physiological properties, bioelectric properties, and thermal properties. For example, the bioelectric properties may include, but are not limited to, complex impedance, resistance, reactance, capacitance, inductance, permittivity, conductivity, nerve firing voltage, nerve firing current, depolarization, hyperpolarization, magnetic field, and induced electromotive force.

The invention recognizes that knowing specific properties of neural tissue, including the morphology of the neural tissue and the specific nerve type, at a given target site prior to neuromodulation treatment provides an ability to more precisely target that neural tissue and minimize collateral damage to surrounding non-neural tissue (e.g., blood vessels). For example, the specific nerve type of a given neural tissue dictates the level of electrical stimulation required to elicit a desired effect. Furthermore, physical properties of the neural tissue, including depth of the neural tissue, further impacts the level of electrical stimulation necessary to result in effective therapeutic neuromodulation. For example, types of neural tissue may vary at certain target sites and neural tissue depth may vary depending on the specific region of the patient's body upon which treatment is to be applied.

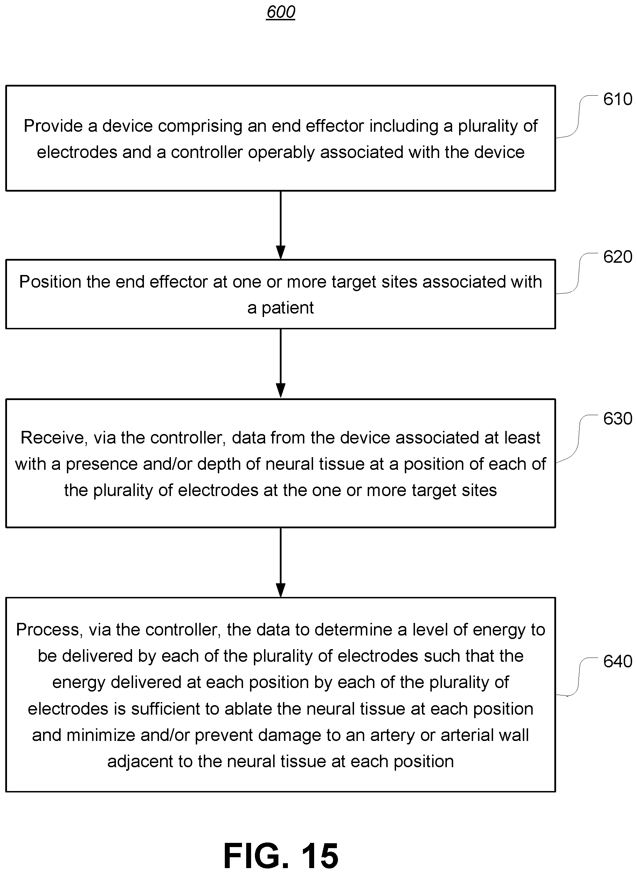

FIG. 15 is a flow diagram illustrating one embodiment of a non-claimed method 600 for treating a peripheral neurological condition. The method 600 includes providing a device comprising an end effector including a plurality of electrodes and a controller operably associated with the device (operation 610) and positioning the end effector at one or more target sites associated with a patient (operation 620). The method 400 further includes receiving, via the controller, data from the device associated at least with a presence and/or depth of neural tissue at a position of each of the plurality of electrodes at the one or more target sites (operation 630). At least a subset of the plurality of electrodes may be configured to sense at least the presence and/or depth of neural tissue at the position of each of the plurality of electrodes and deliver data associated with the presence and depth to the controller. For example, a subset of the plurality of electrodes may be configured to deliver non-therapeutic stimulating energy to respective positions at the one or more target sites at a frequency for locating neural tissue and further sense one or more properties, including at least the presence and/or depth of the neural tissue in response to the non-therapeutic stimulating energy. The delivery of non-therapeutic stimulating energy may further allow for the sensing of other properties, such as physiological properties, bioelectric properties, and thermal properties of neural tissue at respective locations.

The method 600 further includes processing, via the controller, the data to determine a level of energy to be delivered by each of the plurality of electrodes such that the energy delivered at each position by each of the plurality of electrodes is sufficient to ablate the neural tissue at each position and minimize and/or prevent damage to an artery or arterial wall adjacent to the neural tissue at each position (operation 640). The electrodes are configured to be independently controlled and activated by the controller to thereby deliver energy independent of one another. Accordingly, the neuromodulation energy can be applied to the tissue via individual electrodes at respective locations in a highly targeted manner and form micro-lesions to selectively modulate the target structure, while avoiding non-targeted structures (e.g., other neural tissue, blood vessels, etc.) and allowing the surrounding tissue structure to remain healthy for effective wound healing. The controller is configured to tune energy output from each of the plurality of electrodes after an initial level of energy has been delivered based, at least in part, on feedback data received from the device. The feedback data may be associated with efficacy of ablation of the neural tissue at each position during and/or after delivery of initial energy from each of the plurality of electrodes. The feedback data includes physiological properties, bioelectric properties, and thermal properties associated with the neural tissue.

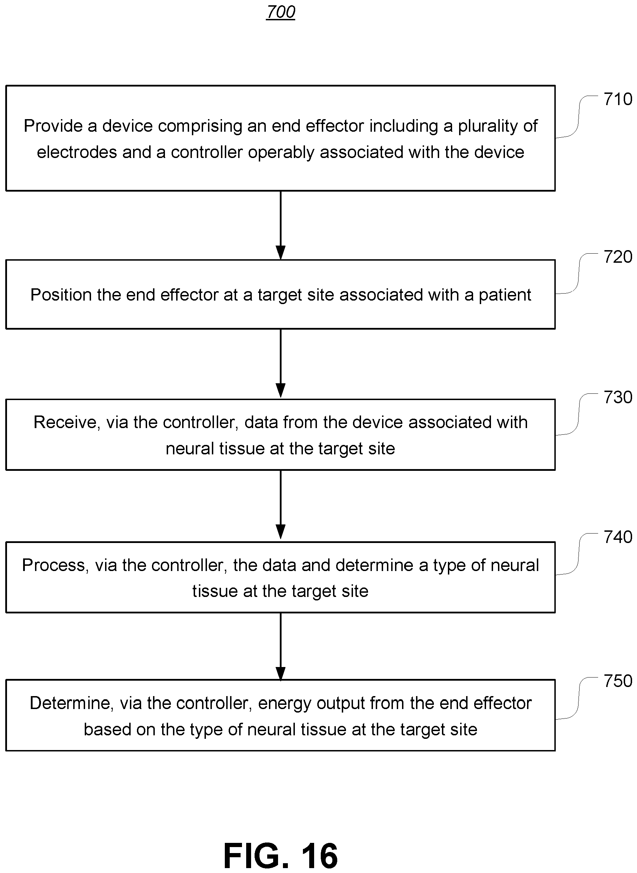

FIG. 16 is a flow diagram illustrating one embodiment of a non-claimed method 700 for diagnosing and/or treating a neurological condition of a patient. The method 700 includes providing a device comprising an end effector including a plurality of electrodes and a controller operably associated with the device (operation 710) and positioning the end effector at one or more target sites associated with a patient (operation 720).

The method 700 further includes receiving, via the controller, data from the device associated with neural tissue at the target site (operation 730). At least a subset of the plurality of electrodes may be configured to sense at least the presence of neural tissue at the position of each of the plurality of electrodes as well as morphology of the neural tissue and deliver such data associated with the presence and morphology to the controller. For example, a subset of the plurality of electrodes may be configured to deliver non-therapeutic stimulating energy to respective positions at the one or more target sites at a frequency for locating neural tissue and further sense one or more properties, including at least the presence and morphology of the neural tissue in response to the non-therapeutic stimulating energy. The morphology may include at least one of physiological properties and bioelectric properties of the neural tissue. The bioelectric properties may include, but are not limited to, complex impedance, resistance, reactance, capacitance, inductance, permittivity, conductivity, nerve firing voltage, nerve firing current, depolarization, hyperpolarization, magnetic field, and induced electromotive force.

The method 500 further includes processing, via the controller, the data to determine a type of neural tissue at the target site (operation 740). The controller is configured to determine neural tissue type based, at least in part, on the morphology. The method 500 further includes determining, via the controller, energy output from the end effector based on the type of neural tissue at the target site (operation 750). In some embodiments, the energy output from the end effector is at a diagnostic energy level or a therapeutic energy level. For example, in some embodiments, a subset of the plurality of electrodes can deliver non-therapeutic energy to the target site at a level sufficient to modulate the neural tissue for diagnostic feedback and minimize and/or prevent damage to non-target neural tissue and/or non-target anatomical structures at the target site. As such, in some embodiments, the method includes sensing, via a subset of the plurality of electrodes, one or more properties of the neural tissue in response to the non-therapeutic energy and delivering data associated with the one or more properties of the neural tissue to the controller for subsequent processing for diagnosing a neurological condition. In other embodiments, the method includes delivering, via a subset of the plurality of electrodes, therapeutic energy to the target site at a level sufficient to therapeutically modulate the neural tissue and minimize and/or prevent damage to non-target neural tissue and/or non-target anatomical structures at the target site.

The following provides a detailed description of the various capabilities of systems of the present invention, including, but not limited to, neuromodulation monitoring, feedback, and mapping capabilities, which, in turn, allowing for detection of anatomical structures and function, neural identification and mapping, and anatomical mapping, for example.

### Neuromodulation Monitoring, Feedback, and Mapping Capabilities

As previously described, the system 100 includes a console 104 to which the device 102 is to be connected. The console 104 is configured to provide various functions for the neuromodulation device 102, which may include, but is not limited to, controlling, monitoring, supplying, and/or otherwise supporting operation of the neuromodulation device 102. The console 104 can further be configured to generate a selected form and/or magnitude of energy for delivery to tissue or nerves at the target site via the end effector (214, 314), and therefore the console 104 may have different configurations depending on the treatment modality of the device 102. For example, when device 102 is configured for electrode-based, heat-element-based, and/or transducer-based treatment, the console 104 includes an energy generator 106 configured to generate RF energy (e.g., monopolar, bipolar, or multi-polar RF energy), pulsed electrical energy, microwave energy, optical energy, ultrasound energy (e.g., intraluminally-delivered ultrasound and/or HIFU), direct heat energy, radiation (e.g., infrared, visible, and/or gamma radiation), and/or another suitable type of energy. When the device 102 is configured for cryotherapeutic treatment, the console 104 can include a refrigerant reservoir (not shown), and can be configured to supply the device 102 with refrigerant. Similarly, when the device 102 is configured for chemical-based treatment (e.g., drug infusion), the console 104 can include a chemical reservoir (not shown) and can be configured to supply the device 102 with one or more chemicals.

In some embodiments, the console 104 may include a controller 107 communicatively coupled to the neuromodulation device 102. However, in the embodiments described herein, the controller 107 may generally be carried by and provided within the handle 118 of the neuromodulation device 102. The controller 107 is configured to initiate, terminate, and/or adjust operation of one or more electrodes provided by the end effector (214, 314) directly and/or via the console 104. For example, the controller 107 can be configured to execute an automated control algorithm and/or to receive control instructions from an operator (e.g., surgeon or other medical professional or clinician). For example, the controller 107 and/or other components of the console 104 (e.g., processors, memory, etc.) can include a computer-readable medium carrying instructions, which when executed by the controller 107, causes the device 102 to perform certain functions (e.g., apply energy in a specific manner, detect impedance, detect temperature, detect nerve locations or anatomical structures, perform nerve mapping, etc.). A memory includes one or more of various hardware devices for volatile and non-volatile storage, and can include both read-only and writable memory. For example, a memory can comprise random access memory (RAM), CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory.

The console 104 may further be configured to provide feedback to an operator before, during, and/or after a treatment procedure via mapping/evaluation/feedback algorithms 110. For example, the mapping/evaluation/feedback algorithms 110 can be configured to provide information associated with the location of nerves at the treatment site, the location of other anatomical structures (e.g., vessels) at the treatment site, the temperature at the treatment site during monitoring and modulation, and/or the effect of the therapeutic neuromodulation on the nerves at the treatment site. In certain embodiments, the mapping/evaluation/feedback algorithm 110 can include features to confirm efficacy of the treatment and/or enhance the desired performance of the system 100. For example, the mapping/evaluation/feedback algorithm 110, in conjunction with the controller 107 and the end effector (214, 314), can be configured to monitor neural activity and/or temperature at the treatment site during therapy and automatically shut off the energy delivery when the neural activity and/or temperature reaches a predetermined threshold (e.g., a threshold reduction in neural activity, a threshold maximum temperature when applying RF energy, or a threshold minimum temperature when applying cryotherapy). In other embodiments, the mapping/evaluation/feedback algorithm 110, in conjunction with the controller 107, can be configured to automatically terminate treatment after a predetermined maximum time, a predetermined maximum impedance or resistance rise of the targeted tissue (i.e., in comparison to a baseline impedance measurement), a predetermined maximum impedance of the targeted tissue), and/or other threshold values for biomarkers associated with autonomic function. This and other information associated with the operation of the system 100 can be communicated to the operator via a display 112 (e.g., a monitor, touchscreen, user interface, etc.) on the console 104 and/or a separate display (not shown) communicatively coupled to the console 104.

In various embodiments, the end effector (214, 314) and/or other portions of the system 100 can be configured to detect various bioelectric-parameters of the tissue at the target site, and this information can be used by the mapping/evaluation/feedback algorithms 110 to determine the anatomy at the target site (e.g., tissue types, tissue locations, vasculature, bone structures, foramen, sinuses, etc.), locate neural tissue, differentiate between different types of neural tissue, map the anatomical and/or neural structure at the target site, and/or identify neuromodulation patterns of the end effector (214, 314) with respect to the patient's anatomy. For example, the end effector (214, 314) can be used to detect resistance, complex electrical impedance, dielectric properties, temperature, and/or other properties that indicate the presence of neural fibers and/or other anatomical structures in the target region. In certain embodiments, the end effector (214, 314), together with the mapping/evaluation/feedback algorithms 110, can be used to determine resistance (rather than impedance) of the tissue (i.e., the load) to more accurately identify the characteristics of the tissue. The mapping/evaluation/feedback algorithms 110 can determine resistance of the tissue by detecting the actual power and current of the load (e.g., via the electrodes (244, 336)).

In some embodiments, the system 100 provides resistance measurements with a high degree of accuracy and a very high degree of precision, such as precision measurements to the hundredths of an Ohm (e.g., 0.01Ω) for the range of 1-50Ω. The high degree of resistance detection accuracy provided by the system 100 allows for the detection sub-microscale structures, including the firing of neural tissue, differences between neural tissue and other anatomical structures (e.g., blood vessels), and event different types of neural tissue. This information can be analyzed by the mapping/evaluation/feedback algorithms and/or the controller 107 and communicated to the operator via a high resolution spatial grid (e.g., on the display 112) and/or other type of display to identify neural tissue and other anatomy at the treatment site and/or indicate predicted neuromodulation regions based on the ablation pattern with respect to the mapped anatomy.

As previously described, in certain embodiments, each electrode (244, 336) can be operated independently of the other electrodes (244, 336). For example, each electrode can be individually activated and the polarity and amplitude of each electrode can be selected by an operator or a control algorithm executed by the controller 107. The selective independent control of the electrodes (244, 336) allows the end effector (214, 314) to detect information and deliver RF energy to highly customized regions. For example, a select portion of the electrodes (244, 336) can be activated to target specific neural fibers in a specific region while the other electrodes (244, 336) remain inactive. In certain embodiments, for example, electrodes (244, 336) may be activated across the portion of a strut that is adjacent to tissue at the target site, and the electrodes (244, 336) that are not proximate to the target tissue can remain inactive to avoid applying energy to non-target tissue. In addition, the electrodes (244, 336) can be individually activated to stimulate or therapeutically modulate certain regions in a specific pattern at different times (e.g., via multiplexing), which facilitates detection of anatomical parameters across a zone of interest and/or regulated therapeutic neuromodulation.

The electrodes (244, 336) can be electrically coupled to the energy generator 106 via wires (not shown) that extend from the electrodes (244, 336), through the shaft 116, and to the energy generator 106. When each of the electrodes (244, 336) is independently controlled, each electrode (244, 336) couples to a corresponding wire that extends through the shaft 116. This allows each electrode (244, 336) to be independently activated for stimulation or neuromodulation to provide precise ablation patterns and/or individually detected via the console 104 to provide information specific to each electrode (244, 336) for neural or anatomical detection and mapping. In other embodiments, multiple electrodes (244, 336) can be controlled together and, therefore, multiple electrodes (244, 336) can be electrically coupled to the same wire extending through the shaft 116. The energy generator 16 and/or components (e.g., a control module) operably coupled thereto can include custom algorithms to control the activation of the electrodes (244, 336). For example, the RF generator can deliver RF power at about 200-100 W to the electrodes (244, 336), and do so while activating the electrodes (244, 336) in a predetermined pattern selected based on the position of the end effector (214, 314) relative to the treatment site and/or the identified locations of the target nerves. In other embodiments, the energy generator 106 delivers power at lower levels (e.g., less than 1 W, 1-5 W, 5-15 W, 15-50 W, 50-150 W, etc.) for stimulation and/or higher power levels. For example, the energy generator 106 can be configured to delivery stimulating energy pulses of 1-3 W via the electrodes (244, 336) to stimulate specific targets in the tissue.

As previously described, the end effector (214, 314) can further include one or more temperature sensors disposed on the struts and/or other portions of the end effector (214, 314) and electrically coupled to the console 104 via wires (not shown) that extend through the shaft 116. In various embodiments, the temperature sensors can be positioned proximate to the electrodes (244, 336) to detect the temperature at the interface between tissue at the target site and the electrodes (244, 336). In other embodiments, the temperature sensors can penetrate the tissue at the target site (e.g., a penetrating thermocouple) to detect the temperature at a depth within the tissue. The temperature measurements can provide the operator or the system with feedback regarding the effect of the therapeutic neuromodulation on the tissue. For example, in certain embodiments the operator may wish to prevent or reduce damage to the tissue at the treatment site, and therefore the temperature sensors can be used to determine if the tissue temperature reaches a predetermined threshold for irreversible tissue damage. Once the threshold is reached, the application of therapeutic neuromodulation energy can be terminated to allow the tissue to remain intact and avoid significant tissue sloughing during wound healing. In certain embodiments, the energy delivery can automatically terminate based on the mapping/evaluation/feedback algorithm 110 stored on the console 104 operably coupled to the temperature sensors.

In certain embodiments, the system 100 can determine the locations and/or morphology of neural tissue and/or other anatomical structures before therapy such that the therapeutic neuromodulation can be applied to precise regions including target neural tissue, while avoiding negative effects on non-target structures, such as blood vessels. As described in further detail below, the system 100 can detect various bioelectrical parameters in an interest zone to determine the location and morphology of various neural tissue (e.g., different types of neural tissue, neuronal directionality, etc.) and/or other tissue (e.g., glandular structures, vessels, bony regions, etc.). In some embodiments, the system 100 is configured to measure bioelectric potential. To do so, one or more of the electrodes (244, 336) is placed in contact with an epithelial surface at a region of interest (e.g., a treatment site). Electrical stimuli (e.g., constant or pulsed currents at one or more frequencies) are applied to the tissue by one or more electrodes (244, 336) at or near the treatment site, and the voltage and/or current differences at various different frequencies between various pairs of electrodes (244, 336) of the end effector (214, 314) may be measured to produce a spectral profile or map of the detected bioelectric potential, which can be used to identify different types of tissues (e.g., vessels, neural tissue, and/or other types of tissue) in the region of interest. For example, current (i.e., direct or alternating current) can be applied to a pair of electrodes (244, 336) adjacent to each other and the resultant voltages and/or currents between other pairs of adjacent electrodes (244, 336) are measured. It will be appreciated that the current injection electrodes (244, 336) and measurement electrodes (244, 336) need not be adjacent, and that modifying the spacing between the two current injection electrodes (244, 336) can affect the depth of the recorded signals. For example, closely-spaced current injection electrodes (244, 336) provided recorded signals associated with tissue deeper from the surface of the tissue than further spaced apart current injection electrodes (244, 336) that provide recorded signals associated with tissue at shallower depths. Recordings from electrode pairs with different spacings may be merged to provide additional information on depth and localization of anatomical structures.

Further, complex impedance and/or resistance measurements of the tissue at the region of interest can be detected directly from current-voltage data provided by the bioelectric potential measurements while differing levels of frequency currents are applied to the tissue (e.g., via the end effector (214, 314)), and this information can be used to map the neural and anatomical structures by the use of frequency differentiation reconstruction. Applying the stimuli at different frequencies will target different stratified layers or cellular bodies or clusters. At high signal frequencies (e.g., electrical injection or stimulation), for example, cell membranes of the neural tissue do not impede current flow, and the current passes directly through the cell membranes. In this case, the resultant measurement (e.g., impedance, resistance, capacitance, and/or induction) is a function of the intracellular and extracellular tissue and liquids. At low signal frequencies, the membranes impede current flow to provide different defining characteristics of the tissues, such as the shapes of the cells or cell spacing. The stimulation frequencies can be in the megahertz range, in the kilohertz range (e.g., 400-500 kHz, 450-480 kHz, etc.), and/or other frequencies attuned to the tissue being stimulated and the characteristics of the device being used. The detected complex impedance or resistances levels from the zone of interest can be displayed to the user (e.g., via the display 112) to visualize certain structures based on the stimulus frequency.

Further, the inherent morphology and composition of the anatomical structures in a given region or zone of the patient react differently to different frequencies and, therefore, specific frequencies can be selected to identify very specific structures. For example, the morphology or composition of targeted structures for anatomical mapping may depend on whether the cells of tissue or other structure are membranonic, stratified, and/or annular. In various embodiments, the applied stimulation signals can have predetermined frequencies attuned to specific neural tissue, such as the level of myelination and/or morphology of the myelination. For example, second axonal parasympathetic structures are poorly myelinated than sympathetic nerves or other structures and, therefore, will have a distinguishable response (e.g., complex impedance, resistance, etc.) with respect to a selected frequency than sympathetic nerves. Accordingly, applying signals with different frequencies to the target site can distinguish the targeted parasympathetic nerves from the non-targeted sensory nerves, and therefore provide highly specific target sites for neural mapping before or after therapy and/or neural evaluation post-therapy. In some embodiments, the neural and/or anatomical mapping includes measuring data at a region of interest with at least two different frequencies to identify certain anatomical structures such that the measurements are taken first based on a response to an injection signal having a first frequency and then again based on an injection signal having a second frequency different from the first. For example, there are two frequencies at which hypertrophied (i.e., disease-state characteristics) sub-mucosal targets have a different electrical conductivity or permittivity compared to "normal" (i.e., healthy) tissue. Complex conductivity may be determined based on one or more measured physiological parameters (e.g., complex impedance, resistance, dielectric measurements, dipole measurements, etc.) and/or observance of one or more confidently known attributes or signatures. Furthermore, the system 100 can also apply neuromodulation energy via the electrodes (244, 336) at one or more predetermined frequencies attuned to a target neural structure to provide highly targeted ablation of the selected neural structure associated with the frequency(ies). This highly targeted neuromodulation also reduces the collateral effects of neuromodulation therapy to non-target sites/structures (e.g., blood vessels) because the targeted signal (having a frequency tuned to a target neural structure) will not have the same modulating effects on the non-target structures.

Accordingly, bioelectric properties, such as complex impedance and resistance, can be used by the system 100 before, during, and/or after neuromodulation therapy to guide one or more treatment parameters. For example, before, during, and/or after treatment, impedance or resistance measurements may be used to confirm and/or detect contact between one or more electrodes (244, 336) and the adjacent tissue. The impedance or resistance measurements can also be used to detect whether the electrodes (244, 336) are placed appropriately with respect to the targeted tissue type by determining whether the recorded spectra have a shape consistent with the expected tissue types and/or whether serially collected spectra were reproducible. In some embodiments, impedance or resistance measurements may be used to identify a boundary for the treatment zone (e.g., specific neural tissue that are to be disrupted), anatomical landmarks, anatomical structures to avoid (e.g., vascular structures or neural tissue that should not be disrupted), and other aspects of delivering energy to tissue.

The bioelectric information can be used to produce a spectral profile or map of the different anatomical features tissues at the target site, and the anatomical mapping can be visualized in a 3D or 2D image via the display 112 and/or other user interface to guide the selection of a suitable treatment site. This neural and anatomical mapping allows the system 100 to accurately detect and therapeutically modulate the postganglionic parasympathetic neural fibers that innervate the mucosa at numerous neural entrance points within a given zone or region of a patient. Further, because there are not any clear anatomical markers denoting the location of the SPF, accessory foramen, and microforamina, the neural mapping allows the operator to identify and therapeutically modulate nerves that would otherwise be unidentifiable without intricate dissection of the mucosa. In addition, anatomical mapping also allows the clinician to identify certain structures that the clinician may wish to avoid during therapeutic neural modulation (e.g., certain arteries). The neural and anatomical bioelectric properties detected by the system 100 can also be used during and after treatment to determine the real-time effect of the therapeutic neuromodulation on the treatment site. For example, the mapping/evaluation/feedback algorithms 110 can also compare the detected neural locations and/or activity before and after therapeutic neuromodulation, and compare the change in neural activity to a predetermined threshold to assess whether the application of therapeutic neuromodulation was effective across the treatment site.

In various embodiments, the system 100 can also be configured to map the expected therapeutic modulation patterns of the electrodes (244, 336) at specific temperatures and, in certain embodiments, take into account tissue properties based on the anatomical mapping of the target site. For example, the system 100 can be configured to map the ablation pattern of a specific electrode ablation pattern at the 45° C. isotherm, the 55° C. isotherm, the 65° C. isotherm, and/or other temperature/ranges (e.g., temperatures ranging from 45° C. to 70° C. or higher) depending on the target site and/or structure.

The system 100 may provide, via the display 112, three-dimensional views of such projected ablation patterns of the electrodes (244, 336) of the end effector (214, 314). The ablation pattern mapping may define a region of influence that each electrode (244, 336) has on the surrounding tissue. The region of influence may correspond to the region of tissue that would be exposed to therapeutically modulating energy based on a defined electrode activation pattern (i.e., one, two, three, four, or more electrodes on any given strut). In other words, the ablation pattern mapping can be used to illustrate the ablation pattern of any number of electrodes (244, 336), any geometry of the electrode layout, and/or any ablation activation protocol (e.g., pulsed activation, multi-polar/sequential activation, etc.).

In some embodiments, the ablation pattern may be configured such that each electrode (244, 336) has a region of influence surrounding only the individual electrode (244, 336) (i.e., a "dot" pattern). In other embodiments, the ablation pattern may be such that two or more electrodes (244, 336) may link together to form a sub-grouped regions of influence that define peanut-like or linear shapes between two or more electrodes (244, 336). In further embodiments, the ablation pattern can result in a more expansive or contiguous pattern in which the region of influence extends along multiple electrodes (244, 336) (e.g., along each strut). In still further embodiments, the ablation pattern may result in different regions of influence depending upon the electrode activation pattern, phase angle, target temperature, pulse duration, device structure, and/or other treatment parameters. The three-dimensional views of the ablation patterns can be output to the display 112 and/or other user interfaces to allow the clinician to visualize the changing regions of influence based on different durations of energy application, different electrode activation sequences (e.g., multiplexing), different pulse sequences, different temperature isotherms, and/or other treatment parameters. This information can be used to determine the appropriate ablation algorithm for a patient's specific anatomy. In other embodiments, the three-dimensional visualization of the regions of influence can be used to illustrate the regions from which the electrodes (244, 336) detect data when measuring bioelectrical properties for anatomical mapping. In this embodiment, the three dimensional visualization can be used to determine which electrode activation pattern should be used to determine the desired properties (e.g., impedance, resistance, etc.) in the desired area. In certain embodiments, it may be better to use dot assessments, whereas in other embodiments it may be more appropriate to detect information from linear or larger contiguous regions.

In some embodiments, the mapped ablation pattern is superimposed on the anatomical mapping to identify what structures (e.g., neural tissue, vessels, etc.) will be therapeutically modulated or otherwise affected by the therapy. An image may be provided to the surgeon which includes a digital illustration of a predicted or planned neuromodulation zone in relation to previously identified anatomical structures in a zone of interest. For example, the illustration may show numerous neural tissue and, based on the predicted neuromodulation zone, identifies which neural tissue are expected to be therapeutically modulated. The expected therapeutically modulated neural tissue may be shaded to differentiate them from the non-affected neural tissue. In other embodiments, the expected therapeutically modulated neural tissue can be differentiated from the non-affected neural tissue using different colors and/or other indicators. In further embodiments, the predicted neuromodulation zone and surrounding anatomy (based on anatomical mapping) can be shown in a three dimensional view (and/or include different visualization features (e.g., color-coding to identify certain anatomical structures, bioelectric properties of the target tissue, etc.). The combined predicted ablation pattern and anatomical mapping can be output to the display 112 and/or other user interfaces to allow the clinician to select the appropriate ablation algorithm for a patient's specific anatomy.

The imaging provided by the system 100 allows the clinician to visualize the ablation pattern before therapy and adjust the ablation pattern to target specific anatomical structures while avoiding others to prevent collateral effects. For example, the clinician can select a treatment pattern to avoid blood vessels, thereby reducing exposure of the vessel to the therapeutic neuromodulation energy. This reduces the risk of damaging or rupturing vessels and, therefore, prevents immediate or latent bleeding. Further, the selective energy application provided by the neural mapping reduces collateral effects of the therapeutic neuromodulation, such as tissue sloughing off during wound healing (e.g., 1-3 weeks post ablation), thereby reducing the aspiration risk associated with the neuromodulation procedure.

The system 100 can be further configured to apply neuromodulation energy (via the electrodes (244, 336)) at specific frequencies attuned to the target neural structure and, therefore, specifically target desired neural tissue over non-target structures. For example, the specific neuromodulation frequencies can correspond to the frequencies identified as corresponding to the target structure during neural mapping. As described above, the inherent morphology and composition of the anatomical structures react differently to different frequencies. Thus, frequency-tuned neuromodulation energy tailored to a target structure does not have the same modulating effects on non-target structures. More specifically, applying the neuromodulation energy at the target-specific frequency causes ionic agitation in the target neural structure, leading to differentials in osmotic potentials of the targeted neural tissue and dynamic changes in neuronal membronic potentials (resulting from the difference in intra-cellular and extra-cellular fluidic pressure). This causes degeneration, possibly resulting in vacuolar degeneration and, eventually, necrosis at the target neural structure, but is not expected to functionally affect at least some non-target structures (e.g., blood vessels). Accordingly, the system 100 can use the neural-structure specific frequencies to both (1) identify the locations of target neural tissue to plan electrode ablation configurations (e.g., electrode geometry and/or activation pattern) that specifically focus the neuromodulation on the target neural structure; and (2) apply the neuromodulation energy at the characteristic neural frequencies to selectively ablate the neural tissue responsive to the characteristic neural frequencies. For example, the end effector (214, 314) of the system 100 may selectively stimulate and/or modulate parasympathetic fibers, sympathetic fibers, sensory fibers, alpha/beta/delta fibers, C-fibers, anoxic terminals of one or more of the foregoing, insulated over non-insulated fibers (regions with fibers), and/or other neural tissue. In some embodiments, the system 100 may also selectively target specific cells or cellular regions during anatomical mapping and/or therapeutic modulation, such as smooth muscle cells, sub-mucosal glands, goblet cells, and stratified cellular regions within a given tissue type. Therefore, the system 100 provides highly selective neuromodulation therapy specific to targeted neural tissue, and reduces the collateral effects of neuromodulation therapy to non-target structures (e.g., blood vessels).

The present disclosure provides a non-claimed method of anatomical mapping and therapeutic neuromodulation. The method includes expanding an end effector (i.e., end effector (214, 314)) at a zone of interest ("interest zone"). For example, the end effector (214, 314) can be expanded such that at least some of the electrodes (244, 336) are placed in contact with tissue at the interest zone. The expanded device can then take bioelectric measurements via the electrodes (244, 336) and/or other sensors to ensure that the desired electrodes are in proper contact with the tissue at the interest zone. In some embodiments, for example, the system 100 detects the impedance and/or resistance across pairs of the electrodes (244, 336) to confirm that the desired electrodes have appropriate surface contact with the tissue and that all of the electrodes are (244, 336) functioning properly.

The method continues by optionally applying an electrical stimulus to the tissue, and detecting bioelectric properties of the tissue to establish baseline norms of the tissue. For example, the method can include measuring resistance, complex impedance, current, voltage, nerve firing rate, neuromagnetic field, muscular activation, and/or other parameters that are indicative of the location and/or function of neural tissue and/or other anatomical structures (e.g., glandular structures, blood vessels, etc.). In some embodiments, the electrodes (244, 336) send one or more stimulation signals (e.g., pulsed signals or constant signals) to the interest zone to stimulate neural activity and initiate action potentials. The stimulation signal can have a frequency attuned to a specific target structure (e.g., a specific neural structure, a glandular structure, a vessel) that allows for identification of the location of the specific target structure. The specific frequency of the stimulation signal is a function of the host permeability and, therefore, applying the unique frequency alters the tissue attenuation and the depth into the tissue the RF energy will penetrate. For example, lower frequencies typically penetrate deeper into the tissue than higher frequencies.

Pairs of the non-stimulating electrodes (244, 336) of the end effector (214, 314) can then detect one or more bioelectric properties of the tissue that occur in response to the stimulus, such as impedance or resistance. For example, an array of electrodes (e.g., the electrodes (244, 336)) can be selectively paired together in a desired pattern (e.g., multiplexing the electrodes (244, 336)) to detect the bioelectric properties at desired depths and/or across desired regions to provide a high level of spatial awareness at the interest zone. In certain embodiments, the electrodes (244, 336) can be paired together in a time-sequenced manner according to an algorithm (e.g., provided by the mapping/evaluation/feedback algorithms 110). In various embodiments, stimuli can be injected into the tissue at two or more different frequencies, and the resultant bioelectric responses (e.g., action potentials) in response to each of the injected frequencies can be detected via various pairs of the electrodes (244, 336). For example, an anatomical or neural mapping algorithm can cause the end effector (214, 314) to deliver pulsed RF energy at specific frequencies between different pairs of the electrodes (244, 336) and the resultant bioelectric response can be recorded in a time sequenced rotation until the desired interest zone is adequately mapped (i.e., "multiplexing"). For example, the end effector (214, 314) can deliver stimulation energy at a first frequency via adjacent pairs of the electrodes (244, 336) for a predetermined time period (e.g., 1-50 milliseconds), and the resultant bioelectric activity (e.g., resistance) can be detected via one or more other pairs of electrodes (244, 336) (e.g., spaced apart from each other to reach varying depths within the tissue). The end effector (214, 314) can then apply stimulation energy at a second frequency different from the first frequency, and the resultant bioelectric activity can be detected via the other electrodes. This can continue when the interest zone has been adequately mapped at the desired frequencies. As described in further detail below, in some embodiments the baseline tissue bioelectric properties (e.g., nerve firing rate) are detected using static detection methods (without the injection of a stimulation signal).

After detecting the baseline bioelectric properties, the information can be used to map anatomical structures and/or functions at the interest zone. For example, the bioelectric properties detected by the electrodes (244, 336) can be amazed via the mapping/evaluation/feedback algorithms 110, and an anatomical map can be output to a user via the display 112. In some embodiments, complex impedance, dielectric, or resistance measurements can be used to map parasympathetic nerves and, optionally, identify neural tissue in a diseased state of hyperactivity. The bioelectric properties can also be used to map other non-target structures and the general anatomy, such as blood vessels, bone, and/or glandular structures. The anatomical locations can be provided to a user (e.g., on the display 112) as a two-dimensional map (e.g., illustrating relative intensities, illustrating specific sites of potential target structures) and/or as a three-dimensional image. This information can be used to differentiate structures on a submicron, cellular level and identify very specific target structures (e.g., hyperactive parasympathetic nerves). The method can also predict the ablation patterns of the end effector (214, 314) based on different electrode neuromodulation protocol and, optionally, superimpose the predicted neuromodulation patterns onto the mapped anatomy to indicate to the user which anatomical structures will be affected by a specific neuromodulation protocol. For example, when the predicted neuromodulation pattern is displayed in relation to the mapped anatomy, a clinician can determine whether target structures will be appropriately ablated and whether non-target structures (e.g., blood vessels) will be undesirably exposed to the therapeutic neuromodulation energy. Thus, the method can be used for planning neuromodulation therapy to locate very specific target structures, avoid non-target structures, and select electrode neuromodulation protocols.

Once the target structure is located and a desired electrode neuromodulation protocol has been selected, the method continues by applying therapeutic neuromodulation to the target structure. The neuromodulation energy can be applied to the tissue in a highly targeted manner that forms micro-lesions to selectively modulate the target structure, while avoiding non-targeted blood vessels and allowing the surrounding tissue structure to remain healthy for effective wound healing. In some embodiments, the neuromodulation energy can be applied in a pulsed manner, allowing the tissue to cool between modulation pulses to ensure appropriate modulation without undesirably affecting non-target tissue. In some embodiments, the neuromodulation algorithm can deliver pulsed RF energy between different pairs of the electrodes (244, 336) in a time sequenced rotation until neuromodulation is predicted to be complete (i.e., "multiplexing"). For example, the end effector (214, 314) can deliver neuromodulation energy (e.g., having a power of 5-10 W (e.g., 7 W, 8 W, 9 W) and a current of about 50-100 mA) via adjacent pairs of the electrodes (244, 336) until at least one of the following conditions is met: (a) load resistance reaches a predefined maximum resistance (e.g., 350Ω); (b) a thermocouple temperature associated with the electrode pair reaches a predefined maximum temperature (e.g., 80° C.); or (c) a predetermined time period has elapsed (e.g., 10 seconds). After the predetermined conditions are met, the end effector (214, 314) can move to the next pair of electrodes in the sequence, and the neuromodulation algorithm can terminate when all of the load resistances of the individual pairs of electrodes is at or above a predetermined threshold (e.g., 100Ω). In various embodiments, the RF energy can be applied at a predetermined frequency (e.g., 450-500 kHz) and is expected to initiate ionic agitation of the specific target structure, while avoiding functional disruption of non-target structures.

During and/or after neuromodulation therapy, the method continues by detecting and, optionally, mapping the post-therapy bioelectric properties of the target site. This can be performed in a similar manner as described above. The post-therapy evaluation can indicate if the target structures (e.g., hyperactive parasympathetic nerves) were adequately modulated or ablated. If the target structures are not adequately modulated (i.e., if neural activity is still detected in the target structure and/or the neural activity has not decreased), the method can continue by again applying therapeutic neuromodulation to the target. If the target structures were adequately ablated, the neuromodulation procedure can be completed.

### Detection of Anatomical Structures and Function

Various embodiments of the present technology can include features that measure bioelectric, dielectric, and/or other properties of tissue at target sites to determine the presence, location, and/or activity of neural tissue and other anatomical structures and, optionally, map the locations of the detected neural tissue and/or other anatomical structures. For example, the present technology can be used to detect glandular structures and, optionally, their mucoserous functions and/or other functions. The present technology can also be configured to detect vascular structures (e.g., arteries) and, optionally, their arterial functions, volumetric pressures, and/or other functions. The mapping features discussed below can be incorporated into any the system 100 and/or any other devices disclosed herein to provide an accurate depiction of nerves at the target site.

Neural and/or anatomical detection can occur (a) before the application of a therapeutic neuromodulation energy to determine the presence or location of neural tissue and other anatomical structures (e.g., blood vessels, glands, etc.) at the target site and/or record baseline levels of neural activity; (b) during therapeutic neuromodulation to determine the real-time effect of the energy application on the neural fibers at the treatment site; and/or (c) after therapeutic neuromodulation to confirm the efficacy of the treatment on the targeted structures (e.g., nerves glands, etc.). This allows for the identification of very specific anatomical structures (even to the micro-scale or cellular level) and, therefore, provides for highly targeted neuromodulation. This enhances the efficacy and efficiency of the neuromodulation therapy. In addition, the anatomical mapping reduces the collateral effects of neuromodulation therapy to non-target sites. Accordingly, the targeted neuromodulation inhibits damage or rupture of blood vessels (i.e., inhibits undesired bleeding) and collateral damage to tissue that may be of concern during wound healing (e.g., when damaged tissue sloughs off).

In certain embodiments, the systems disclosed herein can use bioelectric measurements, such as impedance, resistance, voltage, current density, and/or other parameters (e.g., temperature) to determine the anatomy, in particular the neural, glandular, and vascular anatomy, at the target site. The bioelectric properties can be detected after the transmission of a stimulus (e.g., an electrical stimulus, such as RF energy delivered via the electrodes (244, 336); i.e., "dynamic" detection) and/or without the transmission of a stimulus (i.e., "static" detection).

Dynamic measurements include various embodiments to excite and/or detect primary or secondary effects of neural activation and/or propagation. Such dynamic embodiments involve the heightened states of neural activation and propagation and use this dynamic measurement for nerve location and functional identification relative to the neighboring tissue types. For example, a method of dynamic detection can include: (1) delivering stimulation energy to a treatment site via a treatment device (e.g., the end effector) to excite parasympathetic nerves at the treatment site; (2) measuring one or more physiological parameters (e.g., resistance, impedance, etc.) at the treatment site via a measuring/sensing array of the treatment device (e.g., the electrodes (244, 336)); (4) based on the measurements, identifying the relative presence and position of parasympathetic nerves at the treatment site; and (5) delivering ablation energy to the identified parasympathetic nerves to block the detected para-sympathetic nerves.

Static measurements include various embodiments associated with specific native properties of the stratified or cellular composition at or near the treatment site. The static embodiments are directed to inherent biologic and electrical properties of tissue types at or near the treatment site, the stratified or cellular compositions at or near the treatment site, and contrasting both foregoing measurements with tissue types adjacent the treatment site (and that are not targeted for neuromodulation). This information can be used to localize specific targets (e.g., parasympathetic fibers) and non-targets (e.g., vessels, sensory nerves, etc.). For example, a method of static detection can include: (1) before ablation, utilizing a measuring/sensing array of a treatment device (e.g., the electrodes (244, 336)) to determine one or more baseline physiological parameters; (2) geometrically identifying inherent tissue properties within a region of interest based on the measured physiological parameters (e.g., resistance, impedance, etc.); (3) delivering ablation energy to one or more nerves within the region of via treatment device interest; (4) during the delivery of the ablation energy, determining one or more mid-procedure physiological parameters via the measuring/sensing array; and (5) after the delivery of ablation energy, determining one or more post-procedure physiological parameters via the measurement/sensing array to determine the effectiveness of the delivery of the ablation energy on blocking the nerves that received the ablation energy.

After the initial static and/or dynamic detection of bioelectric properties, the location of anatomical features can be used to determine where the treatment site(s) should be with respect to various anatomical structures for therapeutically effective neuromodulation of the targeted nerves. The bioelectric and other physiological properties described herein can be detected via electrodes (e.g., the electrodes (244, 336) of the end effector (214, 314)), and the electrode pairings on a device (e.g., end effector (214, 314)) can be selected to obtain the bioelectric data at specific zones or regions and at specific depths of the targeted regions. The specific properties detected at or surrounding target neuromodulation sites and associated methods for obtaining these properties are described below. These specific detection and mapping methods discussed below are described with reference to the system 100, although the methods can be implemented on other suitable systems and devices that provide for anatomical identification, anatomical mapping and/or neuromodulation therapy.

### Neural Identification and Mapping

In many neuromodulation procedures, it is beneficial to identify the portions of the nerves that fall within a zone and/or region of influence (referred to as the "interest zone") of the energy delivered by a neuromodulation device 102, as well as the relative three-dimensional position of the neural tissue relative to the neuromodulation device 102. Characterizing the portions of the neural tissue within the interest zone and/or determining the relative positions of the neural tissue within the interest zone enables the clinician to (1) selectively activate target neural tissue over non-target structures (e.g., blood vessels), and (2) sub-select specific targeted neural tissue (e.g., parasympathetic nerves) over non-target neural tissue (e.g., sensory nerves, subgroups of neural tissue, neural tissue having certain compositions or morphologies). The target structures (e.g., parasympathetic nerves) and non-target structures (e.g., blood vessels, sensory nerves, etc.) can be identified based on the inherent signatures of specific structures, which are defined by the unique morphological compositions of the structures and the bioelectrical properties associated with these morphological compositions. For example, unique, discrete frequencies can be associated with morphological compositions and, therefore, be used to identify certain structures. The target and non-target structures can also be identified based on relative bioelectrical activation of the structures to sub-select specific neural structures. Further, target and non-target structures can be identified by the differing detected responses of the structures to a tailored injected stimuli. For example, the systems described herein can detect the magnitude of response of structures and the difference in the responses of anatomical structures with respect to differing stimuli (e.g., stimuli injected at different frequencies).

At least for purposes of this disclosure, a nerve can include the following portions that are defined based on their respective orientations relative to the interest zone: terminating neural tissue (e.g., terminating axonal structures), branching neural tissue (e.g., branching axonal structures), and travelling neural tissue (e.g., travelling axonal structures). For example, terminating neural tissue enter the zone but do not exit. As such, terminating neural tissue are terminal points for neuronal signaling and activation. Branching neural tissue are nerves that enter the interest zone and increase number of nerves exiting the interest zone. Branching neural tissue are typically associated with a reduction in relative geometry of nerve bundle. Travelling neural tissue are nerves that enter the interest zone and exit the zone with no substantially no change in geometry or numerical value.

The system 100 can be used to detect voltage, current, complex impedance, resistance, permittivity, and/or conductivity, which are tied to the compound action potentials of nerves, to determine and/or map the relative positions and proportionalities of nerves in the interest zone. Neuronal cross-sectional area ("CSA") is expected to be due to the increase in axonic structures. Each axon is a standard size. Larger nerves (in cross-sectional dimension) have a larger number of axons than nerves having smaller cross-sectional dimensions. The compound action responses from the larger nerves, in both static and dynamic assessments, are greater than smaller nerves. This is at least in part because the compound action potential is the cumulative action response from each of the axons. When using static analysis, for example, the system 100 can directly measure and map impedance or resistance of nerves and, based on the determined impedance or resistance, determine the location of nerves and/or relative size of the nerves. In dynamic analysis, the system 100 can be used to apply a stimulus to the interest zone and detect the dynamic response of the neural tissue to the stimulus. Using this information, the system 100 can determine and/or map impedance or resistance in the interest zone to provide information related to the neural positions or relative nerve sizes. Neural impedance mapping can be illustrated by showing the varying complex impedance levels at a specific location at differing cross-sectional depths. In other embodiments, neural impedance or resistance can be mapped in a three-dimensional display.

Identifying the portions and/or relative positions of the nerves within the interest zone can inform and/or guide selection of one or more treatment parameters (e.g., electrode ablation patterns, electrode activation plans, etc.) of the system 100 for improving treatment efficiency and efficacy. For example, during neural monitoring and mapping, the system 100 can identify the directionality of the nerves based at least in part on the length of the neural structure extending along the interest zone, relative sizing of the neural tissue, and/or the direction of the action potentials. This information can then be used by the system 100 or the clinician to automatically or manually adjust treatment parameters (e.g., selective electrode activation, bipolar and/or multipolar activation, and/or electrode positioning) to target specific nerves or regions of nerves. For example, the system 100 can selectively activate specific electrodes (244, 336), electrode combinations (e.g., asymmetric or symmetric), and/or adjust the bi-polar or multi-polar electrode configuration. In some embodiments, the system 100 can adjust or select the waveform, phase angle, and/or other energy delivery parameters based on the nerve portion/position mapping and/or the nerve proportionality mapping. In some embodiments, structure and/or properties of the electrodes (244, 336) themselves (e.g., material, surface roughening, coatings, cross-sectional area, perimeter, penetrating, penetration depth, surface-mounted, etc.) may be selected based on the nerve portion and proportionality mapping.

In various embodiments, treatment parameters and/or energy delivery parameters can be adjusted to target on-axis or near axis travelling neural tissue and/or avoid the activation of traveling neural tissue that are at least generally perpendicular to the end effector (214, 314). Greater portions of the on-axis or near axis travelling neural tissue are exposed and susceptible to the neuromodulation energy provided by the end effector (214, 314) than a perpendicular travelling neural structure, which may only be exposed to therapeutic energy at a discrete cross-section. Therefore, the end effector (214, 314) is more likely to have a greater effect on the on-axis or near axis travelling neural tissue. The identification of the neural structure positions (e.g., via complex impedance or resistance mapping) can also allow targeted energy delivery to travelling neural tissue rather than branching neural tissue (typically downstream of the travelling neural tissue) because the travelling neural tissue are closer to the nerve origin and, therefore, more of the nerve is affected by therapeutic neuromodulation, thereby resulting in a more efficient treatment and/or a higher efficacy of treatment. Similarly, the identification of neural structure positions can be used to target travelling and branching neural tissue over terminal neural tissue. In some embodiments, the treatment parameters can be adjusted based on the detected neural positions to provide a selective regional effect. For example, a clinician can target downstream portions of the neural tissue if only wanting to influence partial effects on very specific anatomical structures or positions.

In various embodiments, neural locations and/or relative positions of nerves can be determined by detecting the nerve-firing voltage and/or current over time. An array of the electrodes (244, 336) can be positioned in contact with tissue at the interest zone, and the electrodes (244, 336) can measure the voltage and/or current associated with nerve-firing. This information can optionally be mapped (e.g., on a display 112) to identify the location of nerves in a hyper state (i.e., excessive parasympathetic tone). Rhinitis is at least in part the result of over-firing nerves because this hyper state drives the hyper-mucosal production and hyper-mucosal secretion. Therefore, detection of nerve firing rate via voltage and current measurements can be used to locate the portions of the interest region that include hyper-parasympathetic neural function (i.e., nerves in the diseased state). This allows the clinician to locate specific nerves (i.e., nerves with excessive parasympathetic tone) before neuromodulation therapy, rather than simply targeting all parasympathetic nerves (including non-diseased state parasympathetic nerves) to ensure that the correct tissue is treated during neuromodulation therapy. Further, nerve firing rate can be detected during or after neuromodulation therapy so that the clinician can monitor changes in nerve firing rate to validate treatment efficacy. For example, recording decreases or elimination of nerve firing rate after neuromodulation therapy can indicate that the therapy was effective in therapeutically treating the hyper/diseased nerves.

In various embodiments, the system 100 can detect neural activity using dynamic activation by injecting a stimulus signal (i.e., a signal that temporarily activates nerves) via one or more of the electrodes (244, 336) to induce an action potential, and other pairs of electrodes (244, 336) can detect bioelectric properties of the neural response. Detecting neural tissue using dynamic activation involves detecting the locations of action potentials within the interest zone by measuring the discharge rate in neurons and the associated processes. The ability to numerically measure, profile, map, and/or image fast neuronal depolarization for generating an accurate index of activity is a factor in measuring the rate of discharge in neurons and their processes. The action potential causes a rapid increase in the voltage across nerve fiber and the electrical impulse then spreads along the fiber. As an action potential occurs, the conductance of a neural cell membrane changes, becoming about 40 times larger than it is when the cell is at rest. During the action potential or neuronal depolarization, the membrane resistance diminishes by about 80 times, thereby allowing an applied current to enter the intracellular space as well. Over a population of neurons, this leads to a net decrease in the resistance during coherent neuronal activity, such as chronic para-sympathetic responses, as the intracellular space will provide additional conductive ions. The magnitude of such fast changes has been estimated to have local resistivity changes with recording near DC is 2.8-3.7% for peripheral nerve bundles.

Detecting neural tissue using dynamic activation includes detecting the locations of action potentials within the interest zone by measuring the discharge rate in neurons and the associated processes. The basis of each this discharge is the action potential, during which there is a depolarization of the neuronal membrane of up to 110 mV or more, lasting approximately 2 milliseconds, and due to the transfer of micromolar quantities of ions (e.g., sodium and potassium) across the cellular membrane. The complex impedance or resistance change due to the neuronal membrane falls from 1000 to 25 Ωcm. The introduction of a stimulus and subsequent measurement of the neural response can attenuate noise and improve signal to noise ratios to precisely focus on the response region to improve neural detection, measurement, and mapping.

In some embodiments, the difference in measurements of physiological parameters (e.g., complex impedance, resistance, voltage) over time, which can reduce errors, can be used to create a neural profiles, spectrums, or maps. For example, the sensitivity of the system 100 can be improved because this process provides repeated averaging to a stimulus. As a result, the mapping function outputs can be a unit-less ratio between the reference and test collated data at a single frequency and/or multiple frequencies and/or multiple amplitudes. Additional considerations may include multiple frequency evaluation methods that consequently expand the parameter assessments, such as resistivity, admittivity, center frequency, or ratio of extra- to intracellular resistivity.

In some embodiments, the system 100 may also be configured to indirectly measure the electrical activity of neural tissue to quantify the metabolic recovery processes that accompany action potential activity and act to restore ionic gradients to normal. These are related to an accumulation of ions in the extracellular space. The indirect measurement of electrical activity can be approximately a thousand times larger (in the order of millimolar), and thus are easier to measure and can enhance the accuracy of the measured electrical properties used to generate the neural maps.

The system 100 can perform dynamic neural detection by detecting nerve-firing voltage and/or current and, optionally, nerve firing rate over time, in response to an external stimulation of the nerves. For example, an array of the electrodes (244, 336) can be positioned in contact with tissue at the interest zone, one or more of the electrodes (244, 336) can be activated to inject a signal into the tissue that stimulates the nerves, and other electrodes (244, 336) of the electrode array can measure the neural voltage and/or current due to nerve firing in response to the stimulus. This information can optionally be mapped (e.g., on a display 112) to identify the location of nerves and, in certain embodiments, identify parasympathetic nerves in a hyper state (e.g., indicative of Rhinitis or other diseased state). The dynamic detection of neural activity (voltage, current, firing rate, etc.) can be performed before neuromodulation therapy to detect target nerve locations to select the target site and treatment parameters to ensure that the correct tissue is treated during neuromodulation therapy. Further, dynamic detection of neural activity can be performed during or after neuromodulation therapy to allow the clinician to monitor changes in neural activity to validate treatment efficacy. For example, recording decreases or elimination of neural activity after neuromodulation therapy can indicate that the therapy was effective in therapeutically treating the hyper/diseased nerves.

In some embodiments, a stimulating signal can be delivered to the vicinity of the targeted nerve via one or more penetrating electrodes (e.g., microneedles that penetrate tissue) associated with the end effector (214, 314) and/or a separate device. The stimulating signal generates an action potential, which causes smooth muscle cells or other cells to contract. The location and strength of this contraction can be detected via the penetrating electrode(s) and, thereby, indicate to the clinician the distance to the nerve and/or the location of the nerve relative to the stimulating needle electrode. In some embodiments, the stimulating electrical signal may have a voltage of typically 1-2 mA or greater and a pulse width of typically 100-200 microseconds or greater. Shorter pulses of stimulation result in better discrimination of the detected contraction, but may require more current. The greater the distance between the electrode and the targeted nerve, the more energy is required to stimulate. The stimulation and detection of contraction strength and/or location enables identification of how close or far the electrodes are from the nerve, and therefore can be used to localize the nerve spatially. In some embodiments, varying pulse widths may be used to measure the distance to the nerve. As the needle becomes closer to the nerve, the pulse duration required to elicit a response becomes less and less.

To localize nerves via muscle contraction detection, the system 100 can vary pulse-width or amplitude to vary the energy (Energy=pulse-width*amplitude) of the stimulus delivered to the tissue via the penetrating electrode(s). By varying the stimulus energy and monitoring muscle contraction via the penetrating electrodes and/or other type of sensor, the system 100 can estimate the distance to the nerve. If a large amount of energy is required to stimulate the nerve/contract the muscle, the stimulating/penetrating electrode is far from the nerve. As the stimulating/penetrating electrode, moves closer to the nerve, the amount of energy required to induce muscle contraction will drop. For example, an array of penetrating electrodes can be positioned in the tissue at the interest zone and one or more of the electrodes can be activated to apply stimulus at different energy levels until they induce muscle contraction. Using an iterative process, localize the nerve (e.g., via the mapping/evaluation/feedback algorithm 110).

In some embodiments, the system 100 can measure the muscular activation from the nerve stimulus (e.g., via the electrodes (244, 336)) to determine neural positioning for neural mapping, without the use of penetrating electrodes. In this embodiment, the treatment device targets the smooth muscle cells' varicosities surrounding the submucosal glands and the vascular supply, and then the compound muscle action potential. This can be used to summate voltage response from the individual muscle fiber action potentials. The shortest latency is the time from stimulus artifact to onset of the response. The corresponding amplitude is measured from baseline to negative peak and measured in millivolts (mV). Nerve latencies (mean±SD) in adults typically range about 2-6 milliseconds, and more typically from about 3.4±0.8 to about 4.0±0.5 milliseconds.

In some embodiments, the system 100 can record a neuromagnetic field outside of the nerves to determine the internal current of the nerves without physical disruption of the nerve membrane. Without being bound by theory, the contribution to the magnetic field from the current inside the membrane is two orders of magnitude larger than that from the external current, and that the contribution from current within the membrane is substantially negligible. Electrical stimulation of the nerve in tandem with measurements of the magnetic compound action fields ("CAFs") can yield sequential positions of the current dipoles such that the location of the conduction change can be estimated (e.g., via the least-squares method). Visual representation (e.g., via the display 112) using magnetic contour maps can show normal or non-normal neural characteristics (e.g., normal can be equated with a characteristic quadrupolar pattern propagating along the nerve), and therefore indicate which nerves are in a diseases, hyperactive state and suitable targets for neuromodulation.

During magnetic field detection, an array of the electrodes (244, 336) can be positioned in contact with tissue at the interest zone and, optionally, one or more of the electrodes (244, 336) can be activated to inject an electrical stimulus into the tissue. As the nerves in the interest zone fire (either in response to a stimulus or in the absence of it), the nerve generates a magnetic field (e.g., similar to a current carrying wire), and therefore changing magnetic fields are indicative of the nerve nerve-firing rate. The changing magnetic field caused by neural firing can induce a current detected by nearby sensor wire (e.g., the sensor 314) and/or wires associated with the nearby electrodes (244, 336). By measuring this current, the magnetic field strength can be determined. The magnetic fields can optionally be mapped (e.g., on a display 112) to identify the location of nerves and select target nerves (nerves with excessive parasympathetic tone) before neuromodulation therapy to ensure that the desired nerves are treated during neuromodulation therapy. Further, the magnetic field information can be used during or after neuromodulation therapy so that the clinician can monitor changes in nerve firing rate to validate treatment efficacy.

In other embodiments, the neuromagnetic field is measured with a Hall Probe or other suitable device, which can be integrated into the end effector (214, 314) and/or part of a separate device delivered to the interest zone. Alternatively, rather than measuring the voltage in the second wire, the changing magnetic field can be measured in the original wire (i.e. the nerve) using a Hall probe. A current going through the Hall probe will be deflected in the semiconductor. This will cause a voltage difference between the top and bottom portions, which can be measured. In some aspects of this embodiments, three orthogonal planes are utilized.

In some embodiments, the system 100 can be used to induce electromotive force ("EMF") in a wire (i.e., a frequency-selective circuit, such as a tunable/LC circuit) that is tunable to resonant frequency of a nerve. In this embodiment, the nerve can be considered to be a current carrying wire, and the firing action potential is a changing voltage. This causes a changing current which, in turn, causes a changing magnetic flux (i.e., the magnetic field that is perpendicular to the wire). Under Faraday's Law of Induction/Faraday's Principle, the changing magnetic flux induces EMF (including a changing voltage) in a nearby sensor wire (e.g., integrated into the end effector (214, 314), the sensor 314, and/or other structure), and the changing voltage can be measured via the system 100.

In further embodiments, the sensor wire (e.g., the sensor 314) is an inductor and, therefore, provides an increase of the magnetic linkage between the nerve (i.e., first wire) and the sensor wire (i.e., second wire), with more turns for increasing effect. (e.g., V2,rms=V1,rms (N2/N1)). Due to the changing magnetic field, a voltage is induced in the sensor wire, and this voltage can be measured and used to estimate current changes in the nerve. Certain materials can be selected to enhance the efficiency of the EMF detection. For example, the sensor wire can include a soft iron core or other high permeability material for the inductor.

During induced EMF detection, the end effector (214, 314) and/or other device including a sensor wire is positioned in contact with tissue at the interest zone and, optionally, one or more of the electrodes (244, 336) can be activated to inject an electrical stimulus into the tissue. As the nerves in the interest zone fire (either in response to a stimulus or in the absence of it), the nerve generates a magnetic field (e.g., similar to a current carrying wire) that induces a current in the sensor wire (e.g., the sensor 314). This information can be used to determine neural location and/or map the nerves (e.g., on a display 112) to identify the location of nerves and select target nerves (nerves with excessive parasympathetic tone) before neuromodulation therapy to ensure that the desired nerves are treated during neuromodulation therapy. EMF information can also be used during or after neuromodulation therapy so that the clinician can monitor changes in nerve firing rate to validate treatment efficacy.

In some embodiments, the system 100 can detect magnetic fields and/or EMF generated at a selected frequency that corresponds to a particular type of nerve. The frequency and, by extension, the associated nerve type of the detected signal can be selected based on an external resonant circuit. Resonance occurs on the external circuit when it is matched to the frequency of the magnetic field of the particular nerve type and that nerve is firing. In manner, the system 100 can be used to locate a particular sub-group/type of nerves.

In some embodiments, the system 100 can include a variable capacitor frequency-selective circuit to identify the location and/or map specific nerves (e.g., parasympathetic nerve, sensory nerve, nerve fiber type, nerve subgroup, etc.). The variable capacitor frequency-selective circuit can be defined by the sensor 314 and/or other feature of the end effector (214, 314). Nerves have different resonant frequencies based on their function and structure. Accordingly, the system 100 can include a tunable LC circuit with a variable capacitor (C) and/or variable inductor (L) that can be selectively tuned to the resonant frequency of desired nerve types. This allows for the detection of neural activity only associated with the selected nerve type and its associated resonant frequency. Tuning can be achieved by moving the core in and out of the inductor. For example, tunable LC circuits can tune the inductor by: (i) changing the number of coils around the core; (ii) changing the cross-sectional area of the coils around the core; (iii) changing the length of the coil; and/or (iv) changing the permeability of the core material (e.g., changing from air to a core material). Systems including such a tunable LC circuit provide a high degree of dissemination and differentiation not only as to the activation of a nerve signal, but also with respect to the nerve type that is activated and the frequency at which the nerve is firing.

### Anatomical Mapping

In various embodiments, the system 100 is further configured to provide minimally-invasive anatomical mapping that uses focused energy current/voltage stimuli from a spatially localized source (e.g., the electrodes (244, 336)) to cause a change in the conductivity of the of the tissue at the interest zone and detect resultant biopotential and/or bioelectrical measurements (e.g., via the electrodes (244, 336)). The current density in the tissue changes in response to changes of voltage applied by the electrodes (244, 336), which creates a change in the electric current that can be measured with the end effector (214, 314) and/or other portions of the system 100. The results of the bioelectrical and/or biopotential measurements can be used to predict or estimate relative absorption profilometry to predict or estimate the tissue structures in the interest zone. More specifically, each cellular construct has unique conductivity and absorption profiles that can be indicative of a type of tissue or structure, such as bone, soft tissue, vessels, nerves, types of nerves, and/or certain neural tissue. For example, different frequencies decay differently through different types of tissue. Accordingly, by detecting the absorption current in a region, the system 100 can determine the underlying structure and, in some instances, to a sub-microscale, cellular level that allows for highly specialized target localization and mapping. This highly specific target identification and mapping enhances the efficacy and efficiency of neuromodulation therapy, while also enhancing the safety profile of the system 100 to reduce collateral effects on non-target structures.

To detect electrical and dielectric tissue properties (e.g., resistance, complex impedance, conductivity, and/or, permittivity as a function of frequency), the electrodes (244, 336) and/or another electrode array is placed on tissue at an interest region, and an internal or external source (e.g., the generator 106) applies stimuli (current/voltage) to the tissue. The electrical properties of the tissue between the source and the receiver electrodes (244, 336) are measured, as well as the current and/or voltage at the individual receiver electrodes (244, 336). These individual measurements can then be converted into an electrical map/image/profile of the tissue and visualized for the user on the display 112 to identify anatomical features of interest and, in certain embodiments, the location of firing nerves. For example, the anatomical mapping can be provided as a color-coded or gray-scale three-dimensional or two-dimensional map showing differing intensities of certain bioelectric properties (e.g., resistance, impedance, etc.), or the information can be processed to map the actual anatomical structures for the clinician. This information can also be used during neuromodulation therapy to monitor treatment progression with respect to the anatomy, and after neuromodulation therapy to validate successful treatment. In addition, the anatomical mapping provided by the bioelectrical and/or biopotential measurements can be used to track the changes to non-target tissue (e.g., vessels) due to neuromodulation therapy to avoid negative collateral effects. For example, a clinician can identify when the therapy begins to ligate a vessel and/or damage tissue, and modify the therapy to avoid bleeding, detrimental tissue ablation, and/or other negative collateral effects.

Furthermore, the threshold frequency of electric current used to identify specific targets can subsequently be used when applying therapeutic neuromodulation energy. For example, the neuromodulation energy can be applied at the specific threshold frequencies of electric current that are target neuronal-specific and differentiated from other non-targets (e.g., blood vessels, non-target nerves, etc.). Applying ablation energy at the target-specific frequency results in an electric field that creates ionic agitation in the target neural structure, which leads to differentials in osmotic potentials of the targeted neural tissue. These osmotic potential differentials cause dynamic changes in neuronal membronic potentials (resulting from the difference in intra-cellular and extra-cellular fluidic pressure) that lead to vacuolar degeneration of the targeted neural tissue and, eventually, necrosis. Using the highly targeted threshold neuromodulation energy to initiate the degeneration allows the system 100 to deliver therapeutic neuromodulation to the specific target, while surrounding blood vessels and other non-target structures are functionally maintained.

In some embodiments, the system 100 can further be configured to detect bioelectrical properties of tissue by non-invasively recording resistance changes during neuronal depolarization to map neural activity with electrical impedance, resistance, bio-impedance, conductivity, permittivity, and/or other bioelectrical measurements. Without being bound by theory, when a nerve depolarizes, the cell membrane resistance decreases (e.g., by approximately 80×) so that current will pass through open ion channels and into the intracellular space. Otherwise the current remains in the extracellular space. For non-invasive resistance measurements, tissue can be stimulated by applying a current of less than 100 Hz, such as applying a constant current square wave at 1 Hz with an amplitude less than 25% (e.g., 10%) of the threshold for stimulating neuronal activity, and thereby preventing or reducing the likelihood that the current does not cross into the intracellular space or stimulating at 2 Hz. In either case, the resistance and/or complex impedance is recorded by recording the voltage changes. A complex impedance or resistance map or profile of the area can then be generated.

For impedance/conductivity/permittivity detection, the electrodes (244, 336) and/or another electrode array are placed on tissue at an interest region, and an internal or external source (e.g., the generator 106) applies stimuli to the tissue, and the current and/or voltage at the individual receiver electrodes (244, 336) is measured. The stimuli can be applied at different frequencies to isolate different types of nerves. These individual measurements can then be converted into an electrical map/image/profile of the tissue and visualized for the user on the display 112 to identify anatomical features of interest. The neural mapping can also be used during neuromodulation therapy to select specific nerves for therapy, monitor treatment progression with respect to the nerves and other anatomy, and validate successful treatment.

In some embodiments of the neural and/or anatomical detection methods described above, the procedure can include comparing the mid-procedure physiological parameter(s) to the baseline physiological parameter(s) and/or other, previously-acquired mid-procedure physiological parameter(s) (within the same energy delivery phase). Such a comparison can be used to analyze state changes in the treated tissue. The mid-procedure physiological parameter(s) may also be compared to one or more predetermined thresholds, for example, to indicate when to stop delivering treatment energy. In some embodiments of the present technology, the measured baseline, mid-, and post-procedure parameters include a complex impedance. In some embodiments of the present technology, the post-procedure physiological parameters are measured after a pre-determined time period to allow the dissipation of the electric field effects (ionic agitation and/or thermal thresholds), thus facilitating accurate assessment of the treatment.

In some embodiments, the anatomical mapping methods described above can be used to differentiate the depth of soft tissues within the nasal mucosa. The depth of mucosa on the turbinates is relatively deep while the depth off the turbinate is relatively shallow and, therefore, identifying the tissue depth in the present technology also identifies positions within the nasal mucosa and where precisely to target. Further, by providing the micro-scale spatial impedance mapping of epithelial tissues as described above, the inherent unique signatures of stratified layers or cellular bodies can be used as identifying the region of interest. For example, different regions have larger or small populations of specific structures, such as submucosal glands, so target regions can be identified via the identification of these structures.

In some embodiments, the system 100 includes additional features that can be used to detect anatomical structures and map anatomical features. For example, the system 100 can include an ultrasound probe for identification of neural tissue and/or other anatomical structures. Higher frequency ultrasound provides higher resolution, but less depth of penetration. Accordingly, the frequency can be varied to achieve the appropriate depth and resolution for neural/anatomical localization. Functional identification may rely on the spatial pulse length ("SPL") (wavelength multiplied by number of cycles in a pulse). Axial resolution (SPL/2) may also be determined to locate nerves.

In some embodiments, the system 100 can further be configured to emit stimuli with selective parameters that suppress rather than fully stimulate neural activity. for example, in embodiments where the strength-duration relationship for extracellular neural stimulation is selected and controlled, a state exists where the extracellular current can hyperpolarize cells, resulting in suppression rather than stimulation spiking behavior (i.e., a full action potential is not achieved). Both models of ion channels, HH and RGC, suggest that it is possible to hyperpolarize cells with appropriately designed burst extracellular stimuli, rather than extending the stimuli. This phenomenon could be used to suppress rather than stimulate neural activity during any of the embodiments of neural detection and/or modulation described herein. In various embodiments, the system 100 could apply the anatomical mapping techniques disclosed herein to locate or detect the targeted vasculature and surrounding anatomy before, during, and/or after treatment.

### Equivalents

Various modifications of the invention and many further embodiments thereof, in addition to those shown and described herein, will become apparent to those skilled in the art from the full contents of this document, including references to the scientific and patent literature cited herein. The subject matter herein contains important information, exemplification and guidance that can be adapted to the practice of this invention in its various embodiments and equivalents thereof.

Reference throughout this specification to "one embodiment" or "an embodiment" means that a particular feature, structure, or characteristic described in connection with the embodiment is included in at least one embodiment. Thus, appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The terms and expressions which have been employed herein are used as terms of description and not of limitation, and there is no intention, in the use of such terms and expressions, of excluding any equivalents of the features shown and described (or portions thereof), and it is recognized that various modifications are possible within the scope of the claims.

### EXAMPLES

The following description provides details and results of a study concerning the effects of targeted therapeutic nasal neuromodulation in accordance with the systems and methods of the present invention. The study was performed to determine the effectiveness of RF technology in porcine nasal cavity in vivo, as a step towards translation of this technology for treatment of chronic rhinitis in humans. More specifically, the objective of the study was to characterize damage induced in nasal mucosa and submucosal structures following RF ablation via an end effector consistent with the present disclosure. In particular, the study involved use of end effector 214 including the basket electrode array, for example, which is part of the bipolar RF device affording multi-directional multiplexing control of RF current therefrom. The specific investigation interests relate to the deterministic identification and evaluation of changes to the functional characteristics of autonomic and vascular supply to the nasal mucosal tissues following the specific application of RF energy.

### Protocols

The experimental animals included female domestic swine(s), approximately 40 to 50 kg in weight. The animals were procured and delivered 1-3 days prior to the procedures. Experimental ablation devices under review/study were used to deliver radiofrequency energy. Following induction of anesthesia, the animal was placed in a dorsal recumbency, and the end effector was inserted into the nasal cavity via the nostril/nares. The anesthesia protocol is noted in Table 1 below:

| **Table 1: Anesthesia Protocol** | | | |
|---|---|---|---|
| **Drug/Compound** | **Dosage** | **Route** | **Frequency** |
| Isoflurane | 1-3 % MAC | Inhalation | Duration of anesthesia |
| Atropine | 0.05 mg/kg | Intramuscular | Once |
| Telazol | 4.4 mg/kg | Intramuscular | Once |
| Xylazine | 2.2 mg/kg | Intramuscular | Once |
| Buprenorphine* (0.3 mg/ml) | 0.005 - 0.1 mg/kg | Intramuscular | Under veterinary direction. At anesthesia recovery and 2nd dose at 8 - 10 hours later. Then, every 8 - 12 hours as needed per veterinary direction. |
| Flunixin meglumine* (50 mg/ml) | 1 - 2.2 mg/kg | Intramuscular | Under veterinary direction. At anesthesia recovery and 2nd dose at 8 - 10 hours later, if needed. Then, every 12 - 24 hours as needed, per veterinary direction. |

The end effector was advanced to posterior regions of the nasal cavity and expanded. Up to three experimental ablations were performed in each nasal cavity (at target depths of 3 cm, 6 cm, and 9 cm relative to the entrance of the nasal cavity, with two sites at each location; rotated through 180°). In particular, the target depths of 3 cm, 6 cm, and 9 cm were measured from a proximal end of the end effector in a direction towards the shaft of the handheld device. For example, the end effector was inserted into the nostril to a depth such that a 9 cm marking along the shaft was positioned at the entrance to the nostril. Following ablation, the end effector was rotated through 180°, and application was repeated. The end effector was then retracted to the 6 cm position for ablation, then rotated through 180° and application repeated. Finally, the end effector was retracted to the 3 cm position for ablation and was rotated through 180° for ablation. The same process was then repeated in the contralateral nostril. This approach facilitated the generation of six treatment sites per cavity; the bi-lateral application enabled a total of twelve possible treat sites per animal.

Following completion of ablations, the animals were euthanized and tissue samples were harvested for histopathologic analysis. Tissue were fixed in 10% buffered neutral formalin and routinely processed, embedded in paraffin, sectioned at 5 µm, and stained with Haemotoxylin and Eosin (H&E) for microscopic examination and later interpreted by a board-certified pathologist (C.G.).

### Results

Experiments were carried out on non-survival (i.e., pigs euthanized following completion of ablations shortly thereafter) and survival pigs (i.e., pigs euthanized following completion of ablations). The following provides a description of the experimental results of non-survival and survival pigs having undergone targeted nasal neuromodulation in accordance with the systems and methods of the present invention.

### Non-Survival Pig

In accordance with the previously described experimental protocols, the end effector was advanced to posterior regions of the nasal cavity and expanded. Three experimental treatment sites were performed in each nasal cavity (at target depths of 3 cm, 6 cm, and 9 cm at respective energy levels, with two sites at each location; rotated through 180°). The energy was delivered at respective power levels depending on the target depth. For example, in some embodiments, energy delivered at each target depth of 3 cm, 6 cm, and 9 cm was between 1.5 W and 9 W. In some embodiments, the energy delivered was in the range of 1.5 W to 2.5 W. In some embodiments, the energy delivered was in the range of 2.5 W to 5 W. In some embodiments, the energy delivered was in the range of 5.5 W to 7 W. In some embodiments, the energy delivered was in the range of 7.5 W to 9 W.

Following ablation, tissue samples were harvested via a conventional sectioning and tissue sample extraction techniques, which included sectioning of the nasal cavity into respective portions (i.e., left and right nasal cavities) and initial observations concerning post-ablation results were noted. The gross and detailed observations are noted in Table 2, provided below:

| **Table 2: Observations (Non-Survival Pig)** | | |
|---|---|---|
| **Gross Observation(s)** | | |
| Surface lesion presence | Yes | No |
| | 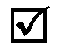 | 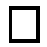 |
| Surface lesion shape | Dot | Linear |
| | 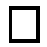 | 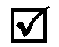 |
| Wide Collateral tissue damage | Yes | No |
| | 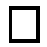 | 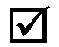 |

| **Detailed Observation(s)** | | |
|---|---|---|
| Sub mucosal Lesion presence | Yes | No |
| | 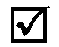 | 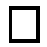 |
| Sub mucosal Lesion depth | Shallow | Deep |
| | 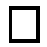 | 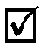 |
| Sub mucosal lesion shape parameter | Clear demarcation | No demarcation |
| | 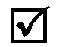 | 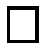 |
| Neural presence | Yes | No |
| | 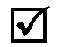 | 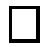 |
| Neural depth | Shallow | Deep |
| | 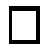 | 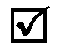 |
| Neural damage | Yes | No |
| | 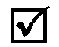 | 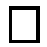 |
| Blood vessel presence | Yes | No |
| | 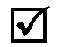 | 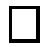 |
| Blood vessel depth | Shallow | Deep |
| | 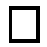 | 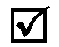 |
| Blood vessel damage | Yes | No |
| | 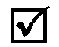 | 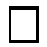 |
| Blood vessel rupture | Yes | No |
| | 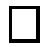 | 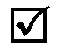 |
| Desiccation | Yes | No |
| | 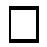 | 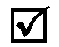 |
| Wound healing evident | Yes | No |
| | 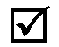 | 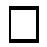 |

Following completion of ablations, the animals were euthanized and tissue samples were harvested for histopathologic analysis. Tissue were fixed in 10% buffered neutral formalin and routinely processed, embedded in paraffin, sectioned at 5 µm, and stained with Haemotoxylin and Eosin (H&E) for microscopic examination and later interpreted by a board-certified pathologist (C.G.).

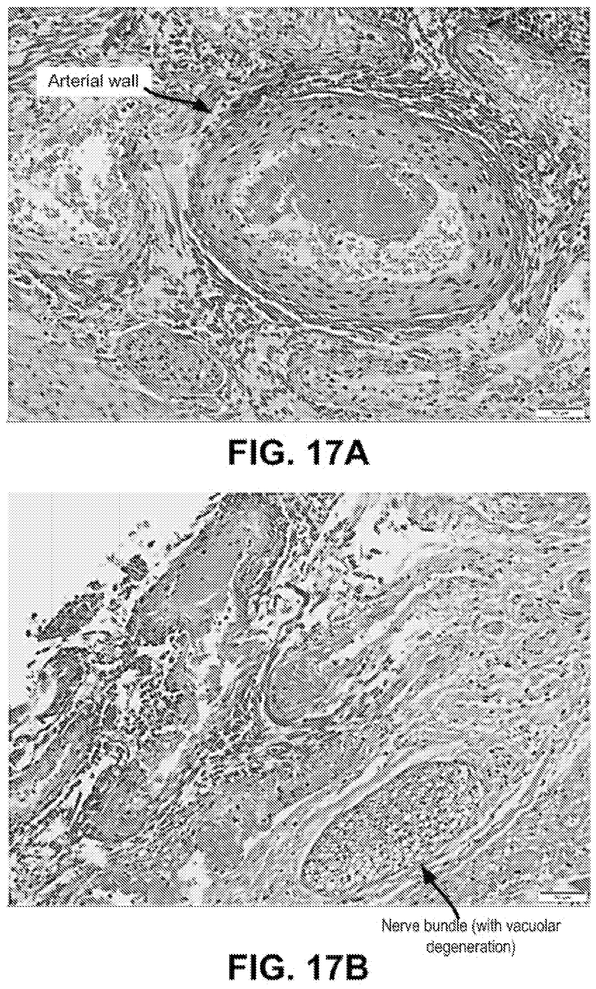

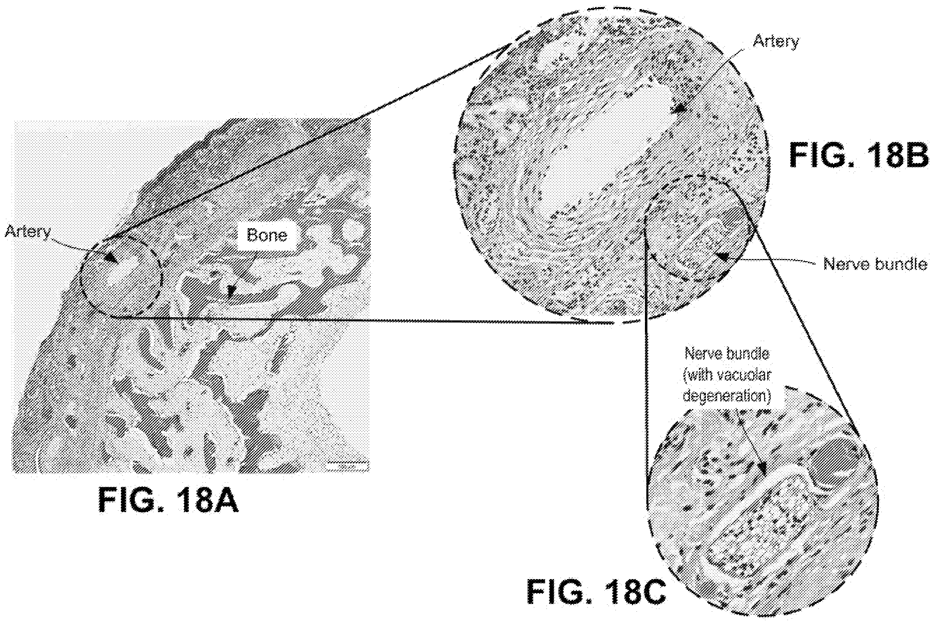

FIGS. 17A, 17B, 18A, 18B, and 18C are histological images of tissue samples from a nasal cavity of non-survival pig stained with H&E having undergone a targeted therapeutic nasal neuromodulation in accordance with the systems and methods of the present invention.

The tissue sample is from a female domestic swine (non-survival pig) having undergone a neuromodulation treatment utilizing the device 102 equipped with an end effector 214. Following induction of anesthesia, the animal was placed in a dorsal recumbency, and the end effector 214 was inserted into the nasal cavity via the nostril/nares. The end effector 214 was advanced to posterior regions of the nasal cavity and expanded. Up to 3 experimental ablations were performed in each nasal cavity (at target depths of 9 cm, 6 cm, and 3 cm with 2 sites at each location, rotated through 180°). The depth of 9 cm, 6 cm and 3 cm was measured from proximal end of the end effector 214. Following completion of ablations, the animals were euthanized. Following euthanasia, tissue samples were harvested for histopathologic analysis. Tissue were fixed in 10% buffered neutral formalin and routinely processed, embedded in paraffin, sectioned at 5 µm, and stained with H&E for microscopic examination.

Referring to FIGS. 17A and 18A, multiple congested blood vessels are shown with diffuse interstitial edema in the surrounding connective tissue. A deep-set nerve bundle is further shown in the images of FIGS. 17A and 18A. Histopathologic analysis of the image reveals that there was a focally extensive area of vascular congestion in the mucosa and submucosa and collagen bundles in the congested area showed marked degeneration characterized by hypereosinophilia with diffuse interstitial edema. The analysis further reveals that overlying mucosa was ulcerated and contained infiltrates of small to moderate numbers of mixed inflammatory cells composed of neutrophils, macrophages and few lymphocytes. The vascular endothelium is separated from the vascular wall with early-stage thrombi formation characterized by accumulation of fibrin admixed with blood, and underlying bone in the area of ablation showed no significant microscopic lesions.

As a result of the targeted therapeutic nasal neuromodulation treatment via the systems of the present invention, the nerve bundle in the congested area had vacuolar degeneration characterized by marked swelling of nerve bundles with clear spaces, including nerve bundles adjacent to the areas of vascular thrombosis. FIGS. 18B and 18C are magnified views of a portion of the histological image of FIG. 18A illustrating a deep-set nerve bundle with axonal vacuolar degeneration as a result of targeted therapeutic nasal neuromodulation in accordance with the systems and methods of the present invention. Accordingly, the precision targeting techniques of the systems and methods of the present invention causes vacuolar degeneration of targeted neural tissue for treatment of a rhinosinusitis condition while avoiding collateral damage to surrounding structures, such as blood vessels and/or other neural structures. For example, the energy delivered may cause necrosis or necrotic-like cell death of the neural tissue. In some embodiments, the energy delivered may cause ionic agitation resulting in vacuolar degeneration of the neural tissue and/or necrosis or necrotic-like cell death of the neural tissue. In some embodiments, the energy delivered causes ionic agitation by way of an osmotic potential change through which extracellular fluid to neural tissue passes into the nerve tubules, thereby resulting in vacuolar degeneration and/or necrosis or necrotic-like cell death of the neural tissue.

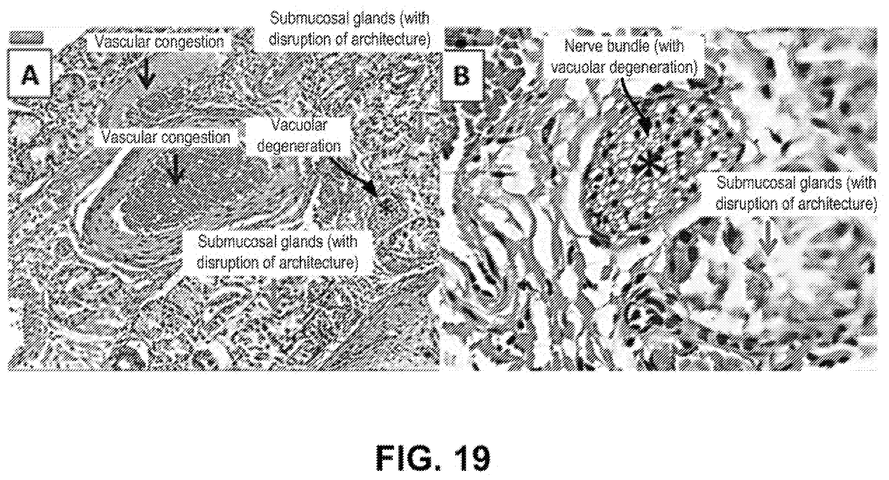

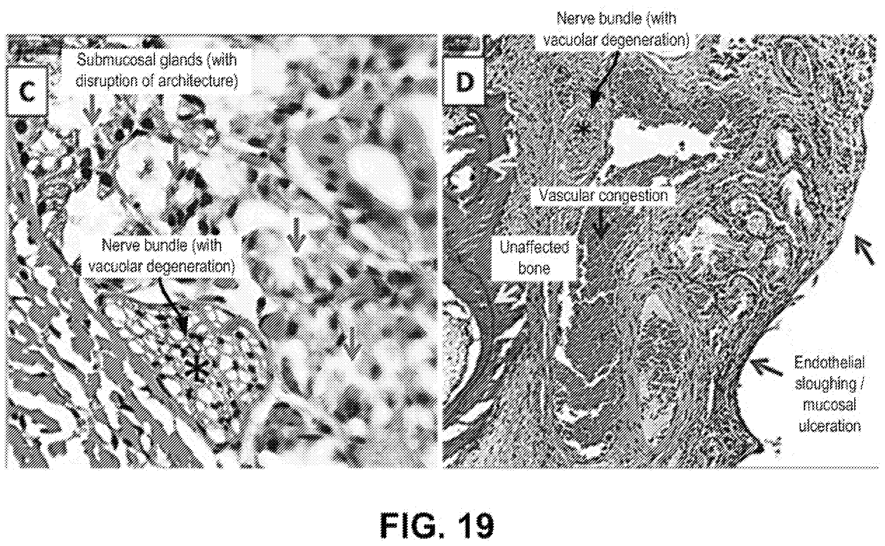

FIGS. 19A-19D are additional histological images of tissue sample from a nasal cavity stained with H&E having undergone a targeted therapeutic nasal neuromodulation in accordance with the systems and methods of the present invention. The figures (FIGS. 19A-19D) illustrate ablation zones showing nerve bundles with vacuolar degeneration, marked vascular congestion with occasional endothelial sloughing, mucosal ulceration, submucosal glands showing disruption of architecture and unaffected bone (yellow arrows). FIG. 19B provides a higher resolution view of the nerve bundle that underwent vacuolar degeneration observed in FIG. 19A. The histopathological analysis of the H&E stained sections from gross lesions and their representative images are further illustrated in FIGS. 19A-19D. The overall observations are as follows: there was a focally extensive area of mucosal ulceration and the nerve bundles showed marked vacuolar degeneration and interstitial edema in the center of the ablation zone (see FIGS. 19A-19C; the blood vessels were markedly congested with occasional sloughing of the endothelium (see FIGS. 19A and 19D); the mucosal glandular cells showed clear spaces (edema) with slight disruption of the architecture and the glandular ducts were mild to moderately dilated (see FIGS. 19A-19C); the interstitial connective tissue was expanded by edema and infiltrates of small numbers of mixed inflammatory cells; the underlying bone in the area of ablation showed no significant microscopic lesions (see FIG. 19D); and the unablated areas of the nasal mucosa showed no significant microscopic lesions.

### Non-Survival Pig

In accordance with the previously described experimental protocols, the end effector was advanced to posterior regions of the nasal cavity and expanded. Three experimental ablations were performed in each nasal cavity (at target depths of 3 cm, 6 cm, and 9 cm at respective energy levels, with two sites at each location; rotated through 180°). The energy was delivered at respective power levels depending on the target depth. For example, in some embodiments, energy delivered at each target depth of 3 cm, 6 cm, and 9 cm was between 1.5 W and 9 W. In some embodiments, the energy delivered was in the range of 1.5 W to 2.5 W. In some embodiments, the energy delivered was in the range of 2.5 W to 5 W. In some embodiments, the energy delivered was in the range of 5.5 W to 7 W. In some embodiments, the energy delivered was in the range of 7.5 W to 9 W.

Following ablation, tissue samples were harvested via a conventional sectioning and tissue sample extraction techniques, which included sectioning of the nasal cavity into respective portions (i.e., left and right nasal cavities) and initial observations concerning post-ablation results were noted. The gross and detailed observations are noted in Table 3, provided below:

| **Table 3: Observations (Non-Survival Pig)** | | |
|---|---|---|
| **Gross Observation(s)** | | |
| Surface lesion presence | Yes | No |
| | 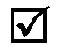 | 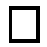 |
| Surface lesion shape | Dot | Linear |
| | 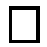 | 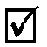 |
| Wide Collateral tissue damage | Yes | No |
| | 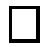 | 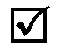 |

| **Detailed Observation(s)** | | |
|---|---|---|
| Sub mucosal Lesion presence | Yes | No |
| | 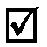 | 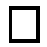 |
| Sub mucosal Lesion depth | Shallow | Deep |
| | 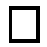 | 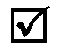 |
| Sub mucosal lesion shape parameter | Clear demarcation | No demarcation |
| | 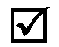 | 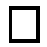 |
| Neural presence | Yes | No |
| | 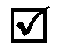 | 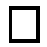 |
| Neural depth | Shallow | Deep |
| | 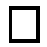 | 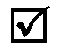 |
| Neural damage | Yes | No |
| | 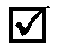 | 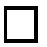 |
| Blood vessel presence | Yes | No |
| | 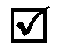 | 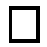 |
| Blood vessel depth | Shallow | Deep |
| | 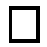 | 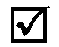 |
| Blood vessel damage | Yes | No |
| | 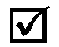 | 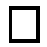 |
| Blood vessel rupture | Yes | No |
| | 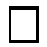 | 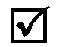 |
| Desiccation | Yes | No |
| | 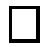 | 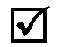 |
| Wound healing evident | Yes | No |
| | 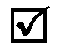 | 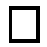 |

Following completion of ablations, the animals were euthanized and tissue samples were harvested for histopathologic analysis. Tissue were fixed in 10% buffered neutral formalin and routinely processed, embedded in paraffin, sectioned at 5 µm, and stained with H&E for microscopic examination and later interpreted by a board-certified pathologist (C.G.).

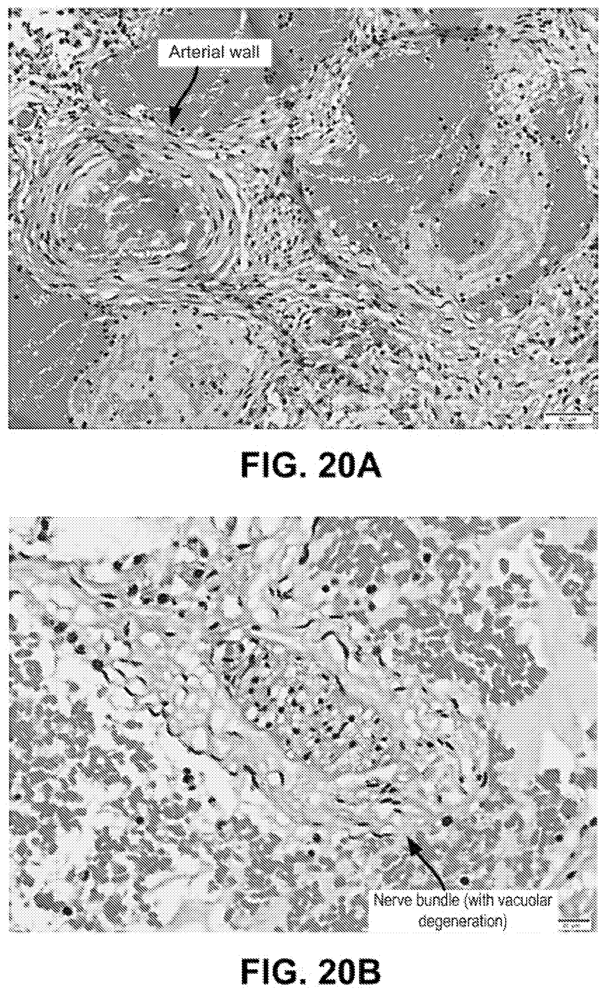

FIGS. 20A and 20B are histological images of tissue samples from a nasal cavity of non-survival pig stained with H&E having undergone a targeted therapeutic nasal neuromodulation in accordance with the systems and methods of the present invention. The tissue sample is from a female domestic swine (non-survival pig) having undergone a neuromodulation treatment utilizing the device 102 equipped with an end effector 214. Following induction of anesthesia, the animal was placed in a dorsal recumbency, and the end effector 214 was inserted into the nasal cavity via the nostril/nares. The end effector 214 was advanced to posterior regions of the nasal cavity and expanded. Up to 3 experimental ablations were performed in each nasal cavity (at target depths of 9 cm, 6 cm, and 3 cm with 2 sites at each location, rotated through 180°). The depth of 9 cm, 6 cm and 3 cm was measured from proximal end of the end effector 214. Following completion of ablations, the animals were euthanized. Following euthanasia, tissue samples were harvested for histopathologic analysis. Tissue were fixed in 10% buffered neutral formalin and routinely processed, embedded in paraffin, sectioned at 5 µm, and stained with H&E for microscopic examination.

Referring to FIG. 20A, multiple congested blood vessels are shown with diffuse interstitial edema in the surrounding connective tissue. A deep-set nerve bundle is further shown in the images of FIG. 20B. Histopathologic analysis of the image reveals that there was a focally extensive area of vascular congestion in the mucosa and submucosa and collagen bundles in the congested area showed marked degeneration characterized by hypereosinophilia with diffuse interstitial edema. The analysis further reveals that overlying mucosa was ulcerated and contained infiltrates of small to moderate numbers of mixed inflammatory cells composed of neutrophils, macrophages and few lymphocytes. The vascular endothelium is separated from the vascular wall with early-stage thrombi formation characterized by accumulation of fibrin admixed with blood, and underlying bone in the area of ablation showed no significant microscopic lesions. Accordingly, the systems and methods of the present disclosure further provide for ablation procedures that allow for the targeting of vascular occlusion via thrombosis by way of endothelial layer detachment, which triggers a coagulation cascade, all while not damaging/rupturing the blood vessel walls.

As a result of the targeted therapeutic nasal neuromodulation treatment via the systems of the present invention, the nerve bundle in the congested area had vacuolar degeneration characterized by marked swelling of nerve bundles with clear spaces, including nerve bundles adjacent to the areas of vascular thrombosis. FIG. 20B is a view of a portion of the histological image of FIG. 20A, illustrating a deep-set nerve bundle with axonal vacuolar degeneration as a result of targeted therapeutic nasal neuromodulation in accordance with the systems and methods of the present invention.

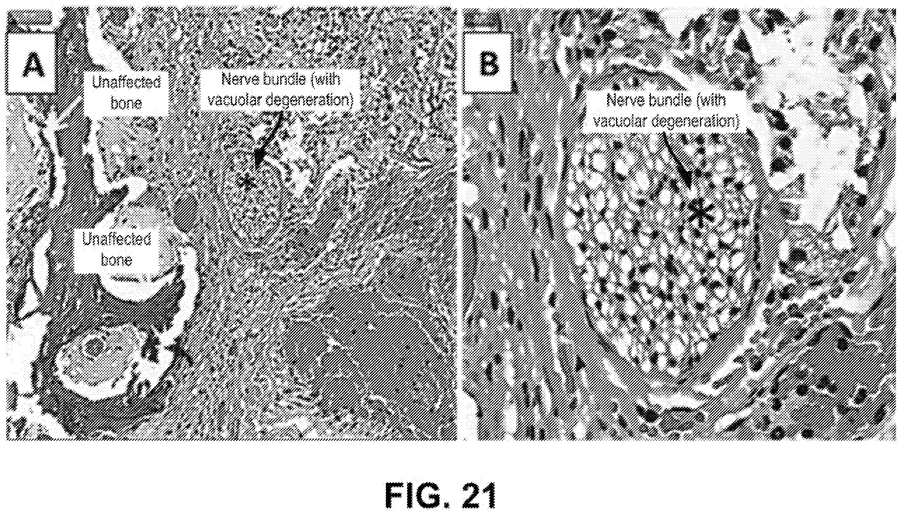

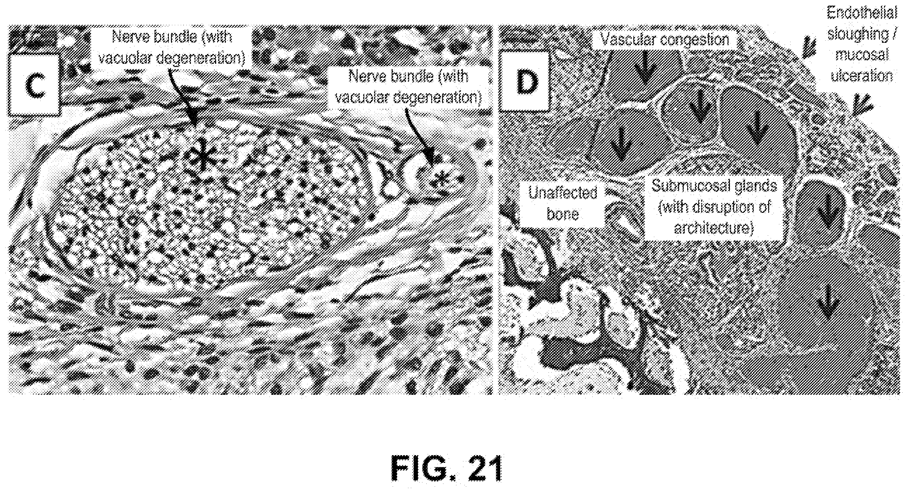

FIGS. 21A-21D are additional histological images of tissue sample from a nasal cavity stained with H&E having undergone a targeted therapeutic nasal neuromodulation in accordance with the systems and methods of the present invention. The figures (FIGS. 21A-21D) illustrate ablation zones showing the nerve bundle with vacuolar degeneration, as well as marked congestion of blood vessels with endothelial sloughing and fibrin-thrombi formation, mucosal ulceration with underlying mucosal glandular cell edema (red arrows), and unaffected turbinate bone. The histopathological analysis of the H&E stained sections from gross lesions and their representative images are shown in FIGS. 21A-21D. The summary of microscopic observations are as follows: in one of the ablated regions, approximately a 3.2 cm linear area of the nasal mucosa was ulcerated, wherein the nerve bundles showed marked vacuolar degeneration and interstitial edema (see FIGS. 21A-21C); the submucosa had extensive area of vascular congestion, wherein the large congested vessels showed occasional endothelial sloughing (see FIGS. 21A and 21D); the mucosal glandular cells show clear spaces (edema) along with slight disruption of the architecture and the central ductular lumens were dilated (see FIG. 21A and 21D); the interstitial connective tissue had edema and infiltrates of small numbers of mixed inflammatory cells; the underlying bone in the area of ablation showed no significant microscopic lesions (see FIGS. 21A and 21D); and the unablated areas of the nasal mucosa showed no significant microscopic lesions.

### Survival Pig

In accordance with the previously described experimental protocols, the end effector was advanced to posterior regions of the nasal cavity and expanded. Two experimental ablations were performed in each nasal cavity (at target depths of 6 cm and 9 cm at respective energy levels, with two sites at each location; rotated through 180°). The energy was delivered at respective power levels depending on the target depth. For example, in some embodiments, energy delivered at each target depth of 3 cm, 6 cm, and 9 cm was between 1.5 W and 9 W. In some embodiments, the energy delivered was in the range of 1.5 W to 2.5 W. In some embodiments, the energy delivered was in the range of 2.5 W to 5 W. In some embodiments, the energy delivered was in the range of 5.5 W to 7 W. In some embodiments, the energy delivered was in the range of 7.5 W to 9 W.

Following ablation, tissue samples were harvested via a conventional sectioning and tissue sample extraction techniques, which included sectioning of the nasal cavity into respective portions (i.e., left and right nasal cavities) and initial observations concerning post-ablation results were noted. The gross and detailed observations are noted in Table 4, provided below:

| **Table 4: Observations (Survival Pig)** | | |
|---|---|---|
| **Gross Observation(s)** | | |
| Surface lesion presence | Yes | No |
| | 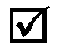 | 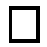 |
| Surface lesion shape | Dot | Linear |
| | 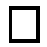 | 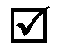 |
| Wide Collateral tissue damage | Yes | No |
| | 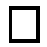 | 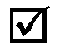 |

| **Detailed Observation(s)** | | |
|---|---|---|
| Sub mucosal Lesion presence | Yes | No |
| | 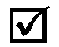 | 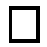 |
| Sub mucosal Lesion depth | Shallow | Deep |
| | 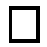 | 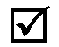 |
| Sub mucosal lesion shape parameter | Clear demarcation | No demarcation |
| | 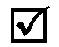 | 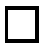 |
| Neural presence | Yes | No |
| | 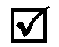 | 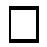 |
| Neural depth | Shallow | Deep |
| | 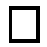 | 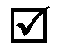 |
| Neural damage | Yes | No |
| | 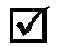 | 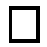 |
| Blood vessel presence | Yes | No |
| | 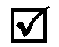 | 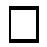 |
| Blood vessel depth | Shallow | Deep |
| | 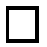 | 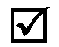 |
| Blood vessel damage | Yes | No |
| | 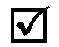 | 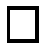 |
| Blood vessel rupture | Yes | No |
| | 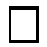 | 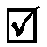 |
| Desiccation | Yes | No |
| | 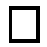 | 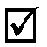 |
| Wound healing evident | Yes | No |
| | 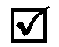 | 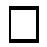 |

Following completion of ablations, the animals were euthanized and tissue samples were harvested for histopathologic analysis. Tissue were fixed in 10% buffered neutral formalin and routinely processed, embedded in paraffin, sectioned at 5 µm, and stained with H&E for microscopic examination and later interpreted by a board-certified pathologist (C.G.).

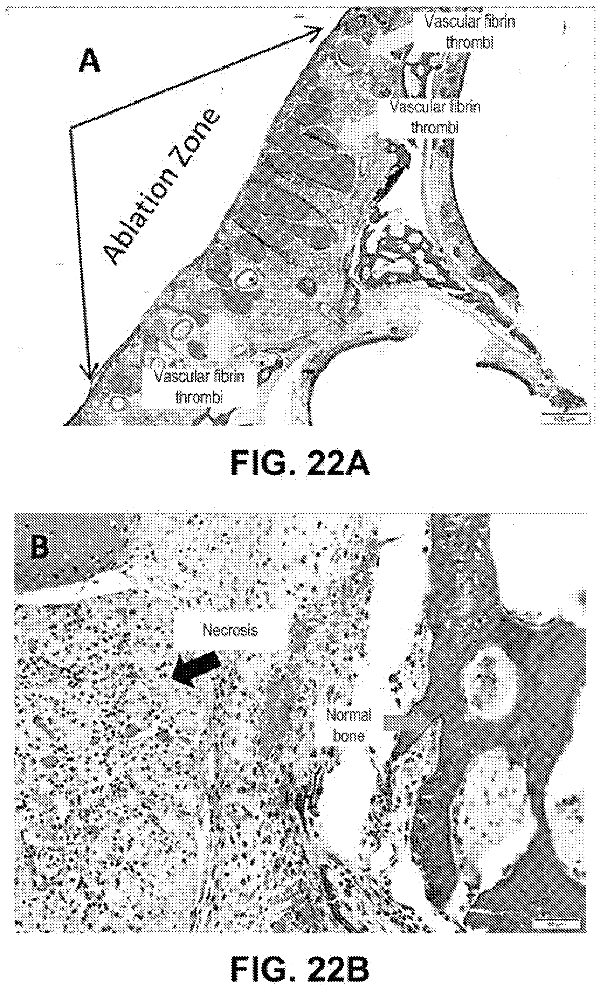

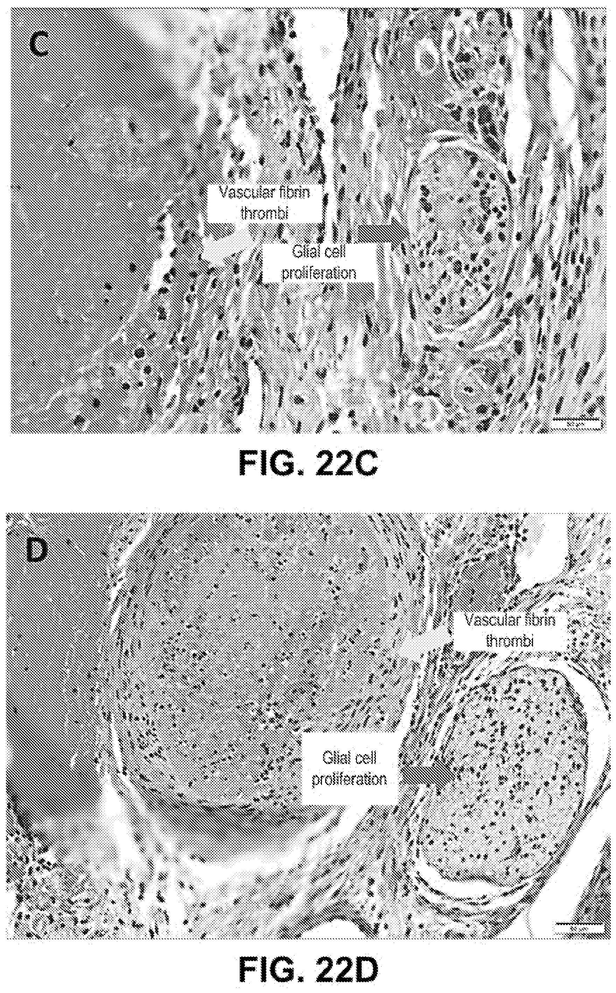

FIGS. 22A, 22B, 22C, and 22D are histological images of a tissue sample from a nasal cavity of survival pig stained with H&E having undergone a targeted therapeutic nasal neuromodulation in accordance with the systems and methods of the present invention.

The tissue sample is from a female domestic swine (survival pig) having undergone a neuromodulation treatment utilizing the device 102 equipped with an end effector 214. Following induction of anesthesia, the animal was placed in a dorsal recumbency, and the end effector 214 was inserted into the nasal cavity via the nostril/nares. The end effector 214 was advanced to posterior regions of the nasal cavity and expanded. Two experimental ablations were performed in each nasal cavity (at target depths of 9 cm and 6 cm with 2 sites at each location, rotated through 180°). The depth of 9 cm and 6 cm was measured from proximal end of the end effector 214. Approximately 48 hours following completion of ablations, the animals were euthanized. Following euthanasia, tissue samples were harvested for histopathologic analysis. Tissue were fixed in 10% buffered neutral formalin and routinely processed, embedded in paraffin, sectioned at 5 µm, and stained with H&E for microscopic examination.

FIG. 22A is a subgross image of the ablation zone showing marked vascular congestion, mucosal ulceration with occasional vascular fibrin thrombi. The image of FIG. 22B illustrates mucosal glands showing marked necrosis with normal bone. The areas that were in contact with the probe showed a focally extensive area of vascular congestion in the mucosa and submucosa and the vascular lumen was markedly dilated and filled with fibrin thrombi that often occlude the lumen. The collagen bundles in the congested area showed marked degeneration characterized by hypereosinophilia with diffuse interstitial edema. The overlying mucosa was ulcerated and contained infiltrates of small to moderate numbers of mixed inflammatory cells composed of neutrophils, macrophages, and few lymphocytes. The images of FIGS. 22C and 22D illustrate nerve bundles including glial cell proliferation. The nerve bundles in the ablated area show increased glial cell proliferation with occasional large nucleated cells with dense chromatin. The mucosal glands adjacent to the vascular thrombosis showed coagulative necrosis characterized by hypereosinophilia and contain cellular and nuclear debris. The underlying bone in the area of ablation showed no significant microscopic lesions (see FIG. 22B).

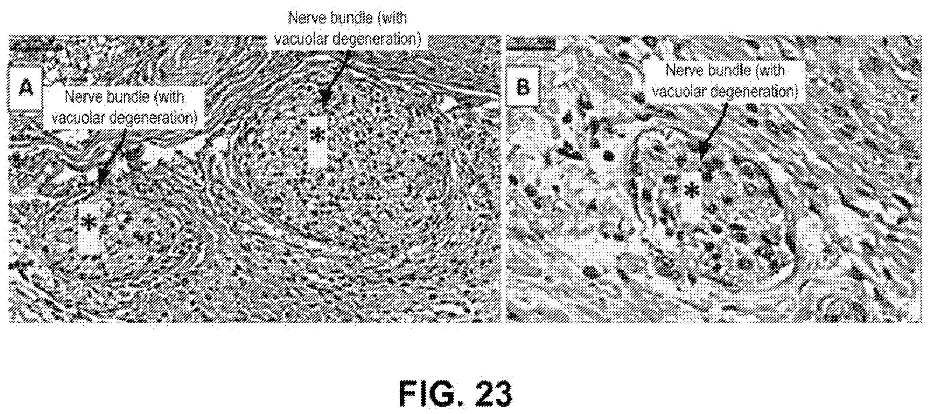

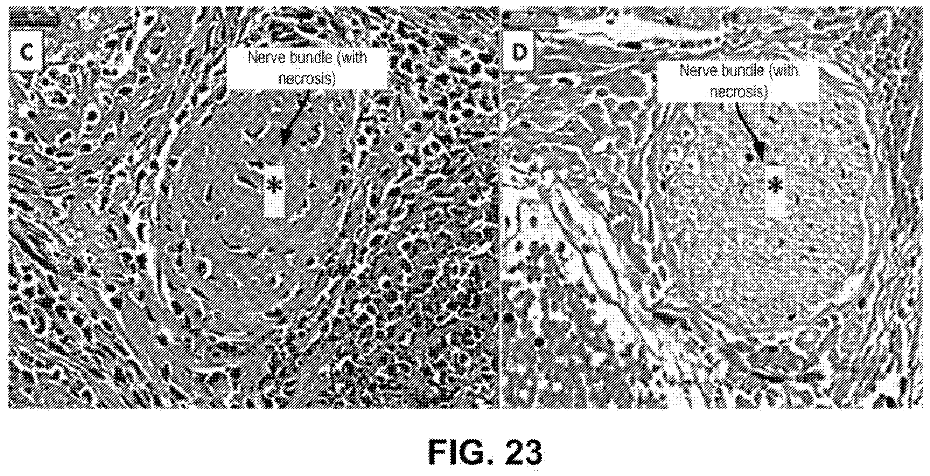

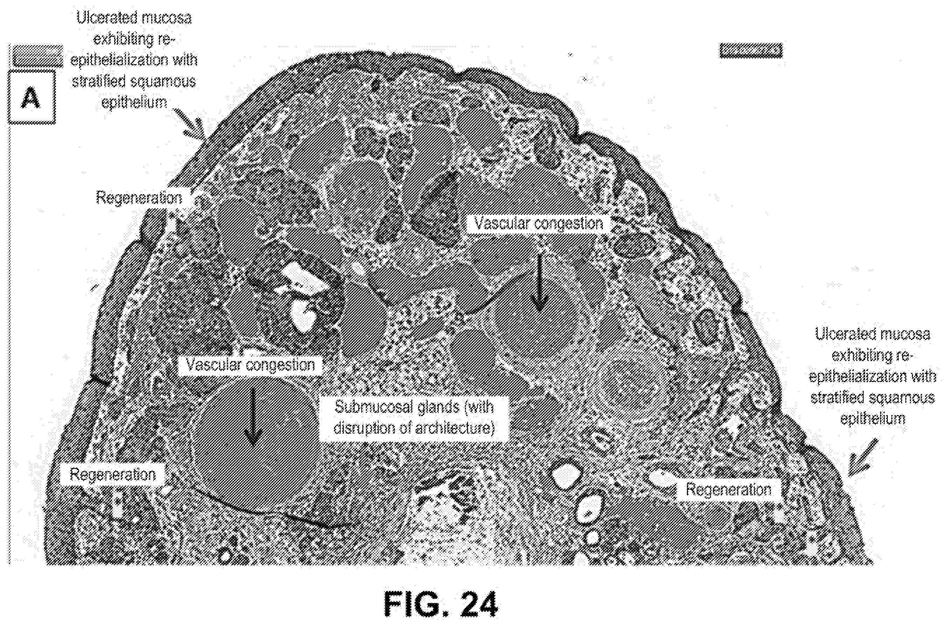

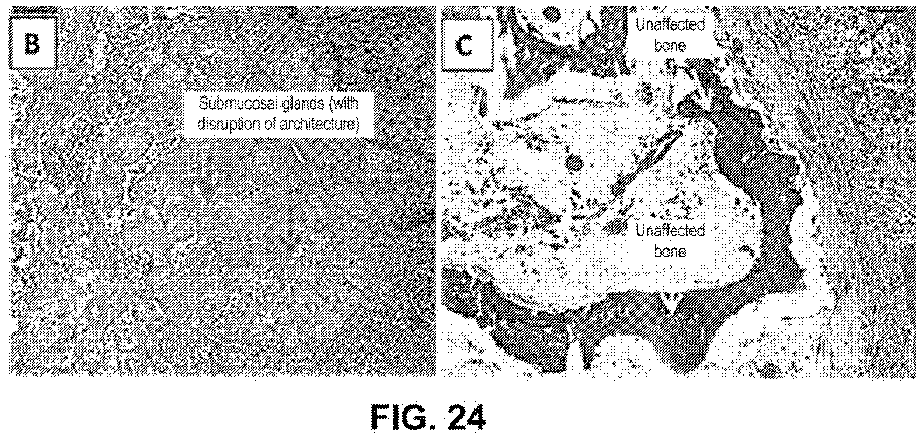

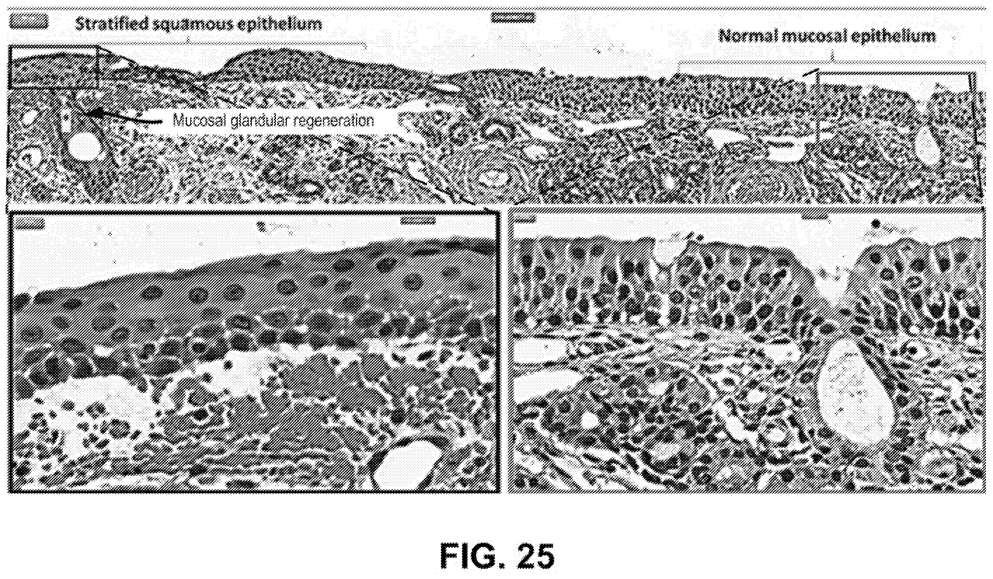

FIGS. 23A-23D, 24A-24C, and 25 are additional histological images of tissue sample from a nasal cavity stained with H&E having undergone a targeted therapeutic nasal neuromodulation in accordance with the systems and methods of the present invention. FIGS. 23A-23B illustrate the degeneration of the nerve bundles and FIGS. 23C and 23D illustrate the necrosis of nerve bundles. As shown in FIGS. 24A-24C, ablation zone shows marked vasculature congestion with fibrin thrombi formation, mucosal glandular degeneration, necrosis and regeneration, as well as ulcerated mucosa showing re-epithelialization with stratified squamous epithelium, and unaffected bone. FIG. 25 is a representative image of the nasal mucosa showing re-epithelialized nasal mucosa lined by stratified squamous epithelium with mucosal glandular regeneration in the submucosa, with inserts showing the epithelial tissue at a higher resolution. The histopathological analysis of the H&E stained sections from gross lesions and their representative images are shown in FIGS. 23A-23D, 24A-24C, and 25. The summary of microscopic observations are as follows: as shown in FIGS. 23A-23D, the nerve bundles showed significant morphological changes which include, marked vacuolar degeneration in the edge of ablation zone with complete coagulative necrosis in the center of the ablation zone. There was diffuse interstitial edema, hemorrhage and cellular debris; the congested blood vessels were filled with fibrin thrombi and diffuse transmural necrosis (vasculitis), and the arterial wall(s) were diffusely pale with loss of cellular details (coagulative necrosis and vasculitis) (see FIGS. 24A-24C); the submucosal glands had undergone coagulative necrosis with ghosts of glandular architecture and cell debris (see FIG. 24A-24C); as expected, normal wound healing was evident on microscopic examination which include re-epithelialization of the ulcerated nasal mucosa lined by stratified squamous epithelium, submucosal fibrous connective tissue proliferation (see FIG. 25); other microscopic changes include projection of fronds of mucosal epithelium into the submucosa forming ducts lined by stratified squamous epithelium, occasional mucosal glandular regeneration and fibrosis, and neo-vascularization in the areas surrounding the congested blood vessels in ablation zone (see FIGS. 24A-24C); and the underlying bone in the ablated zone showed no significant microscopic lesions (see FIG. 24C).

### In Vivo Study/Evaluation Summary

The non-survival studies allowed for a better understanding of the extent of acute tissue injury following ablation. The nerve bundles had undergone marked vacuolar degeneration and the blood vessels had consistent vascular endothelial separation and were freely floating in the lumen while the vessel wall remained intact. The separation of the vascular endothelium from the vascular wall may have contributed to vascular thrombosis of the affected blood vessel (noticed in survival pig). The collagen bundles in the connective tissue showed marked degeneration with interstitial edema which is consistent with acute effects often noticed with thermal injury.

In the survival pig, the vessel lumens were often occluded by fibrin thrombi and the adjacent mucosal glands show coagulative necrosis. The necrosis in the mucosal glands and connective tissue could be secondary to hypoxia caused by fibrin thrombi occluding the vascular lumens (infarction). Such data suggests that the ablation procedure does not cause bystander tissue damage, as the changes noticed in the mucosal glands were secondary to tissue hypoxia. The underlying bone showed no significant microscopic lesions in both survival and non-survival pigs.

Accordingly, the systems and methods of the present disclosure further provide for ablation procedures that allow for the targeting of vascular occlusion via thrombosis by way of endothelial layer detachment, which triggers a coagulation cascade, all while not damaging/rupturing the blood vessel walls. The ablation procedure provides for a direct effect on the submucosal glands (the glands responsible for mucus production of the nasal cavity). The submucosal glands are hyperstimulated in rhinitis and over produce and secrete mucus, which results in rhinorrhea. The data of the examples provided herein is evidence that the ablation procedures provided by the systems and methods of the present disclosure have a direct impact on rhinorrhea via a 2-pronged approach: nerve degeneration; and hypoxia (i.e., removing blood supply).

The nerve bundles in the ablation zone in survival pig often had increased number of glial cells in the neurons suggestive of changes in response to thermal ablation. The increase in glial cell proliferation and presence of numerous immature enlarged nuclei in the nerve bundles represent inflammation response to ablation

The initial radio-frequency ablation studies in non-survival pigs showed marked vascular congestion with frequent endothelial separation and formation of early fibrin thrombi. The collagen bundles often show differential staining suggestive of exposure to thermal injury. The nerve bundles show marked vacuolar degeneration which is suggestive of possible edema from early thermal injury secondary to ablation. The changes noticed in both the non-survival animals are similar which are consistent with acute thermal injury. Following therapeutic trauma/injury and within the first 48 hours of survival in all of the pig(s) (survival study), the blood vessels are filled with organized thrombus which completely occluded the lumens. The adjacent mucous glands show marked necrosis and the collagen bundles show loss of nuclei (necrosis). Accordingly, based on the results of the studies, the ablation procedures consistent with the present disclosure are able to trigger endothelial detachment and thereby initiate the thrombotic cascade without impacting the integrity of the arterial wall, thereby preventing rupture.

In summary, the lesions noticed in both non-survival and survival study suggests that the ablation procedure is much localized and results in neuronal degeneration and vascular endothelial damage resulting in vascular thrombosis and hypoxia (infarction) with tissue necrosis. The vessel wall remained intact in all the experimental procedures and often limiting the ablation induced damage to the endothelium. The underlying bone within the ablated area showed no significant lesions.

The microscopic lesions noticed in the tissues are localized and noticed in proximity to the end effector. The nerve bundles within the area of ablation show acute changes including marked vacuolar degeneration and within the first 48 hours post ablation the nerve bundles show increased glial cells proliferation with some immature dense granular large nuclei (karyomegaly). The increase in glial cells is suggestive of inflammation secondary to thermal injury, this is a normal response often noticed with injury.

## Claims

1. A system (100) for treating a condition within a nasal cavity of a patient, the system comprising:
a treatment device (102) comprising a shaft (116) and a multi-segment end effector (314) extending from the shaft (116) and for delivering energy to one or more target sites within the nasal cavity of the patient, the multi-segment end effector (314) comprising a first segment (322) and a separate second segment (324) longitudinally spaced apart from the first segment (322), wherein each of the first and second segments (322, 324) is transformable between a retracted configuration and an expanded deployed configuration, and each of the first and second segments (322, 324) comprises a plurality of electrodes (336); and
a controller (107) operably associated with the treatment device (102) and configured to:
receive data from the treatment device (102) associated at least with presence and/or a depth/location of neural tissue at a position of each of the plurality of electrodes (336) within the nasal cavity; and
process the data to determine a level of energy to be delivered by each of the plurality of electrodes (336) such that the energy delivered at each position by each of the plurality of electrodes (336) is sufficient to modulate/ablate the neural tissue at each position and minimize and/or prevent damage to an artery or arterial wall adjacent to the neural tissue at each position.

2. The system (100) of claim 1, wherein a subset of the plurality of electrodes (336) is configured to sense at least the presence and/or depth/location of neural tissue at the position of each of the plurality of electrodes (336) and deliver data associated with the depths to the controller (107).

3. The system (100) of claim 2, wherein a subset of the plurality of electrodes (336) is configured to deliver non-therapeutic stimulating energy to respective positions within the nasal cavity at a frequency/waveform for locating neural tissue and further sense one or more properties, including at least the presence and/or depth/location of the neural tissue in response to the non-therapeutic stimulating energy.

4. The system (100) of claim 1, wherein the controller (107) is configured to tune energy output from each of the plurality of electrodes (336) after an initial level of energy has been delivered based, at least in part, on feedback data received from the device (102).

5. The system (100) of claim 4, wherein the feedback data is associated with efficacy of ablation of the neural tissue at each position during and/or after delivery of initial energy from each of the plurality of electrodes (336).

6. The system (100) of claim 5, wherein the feedback data comprises one or more properties associated with neural tissue at respective positions within the nasal cavity, the one or more properties comprising at least one of physiological properties, bioelectric properties, and thermal properties.

7. The system (100) of claim 6, wherein a subset of the plurality of electrodes (336) is configured to deliver non-therapeutic stimulating energy to respective positions within the nasal cavity at a frequency for locating neural tissue and sense one or more properties associated with the neural tissue in response to the non-therapeutic stimulating energy, wherein the properties comprise at least one of physiological properties, bioelectric properties, and thermal properties.

8. The system (100) of claim 7, wherein the bioelectric properties comprise at least one of complex impedance, resistance, reactance, capacitance, inductance, permittivity, conductivity, nerve firing voltage, nerve firing current, depolarization, hyperpolarization, magnetic field, and induced electromotive force.

9. The system (100) of claim 1, wherein at least some of the electrodes (336) are configured to be independently controlled (107) and activated by the controller (107) to thereby deliver energy independent of one another.

10. The system (100) of claim 9, wherein at least some of the electrodes (336) have different levels of energy to be delivered at respective positions sufficient to ablate neural tissue at the respective positions.

11. The system (100) of claim 1, wherein the nasal condition comprises rhinosinusitis.

## Patentansprüche

1. System (100) zum Behandeln einer Erkrankung in einer Nasenhöhle eines Patienten, wobei das System Folgendes umfasst:
eine Behandlungsvorrichtung (102), die einen Schaft (116) und einen aus mehreren Segmenten bestehenden Endeffektor (314) umfasst, der sich von dem Schaft (116) aus erstreckt und zum Abgeben von Energie an eine oder mehrere Zielstellen innerhalb der Nasenhöhle des Patienten dient, wobei der aus mehreren Segmenten bestehende Endeffektor (314) ein erstes Segment (322) und ein separates zweites Segment (324) umfasst, das in Längsrichtung von dem ersten Segment (322) beabstandet ist, wobei jedes von dem ersten und dem zweiten Segment (322, 324) zwischen einer eingefahrenen Konfiguration und einer ausgefahrenen, entfalteten Konfiguration umwandelbar ist, und jedes von dem ersten und dem zweiten Segment (322, 324) eine Vielzahl von Elektroden (336) umfasst; und
eine Steuerung (107), die mit der Behandlungsvorrichtung (102) wirkverbunden und zu Folgendem konfiguriert ist:
Empfangen von Daten von der Behandlungsvorrichtung (102), die mindestens mit einem Vorhandensein und/oder einer Tiefe/einem Standort von Nervengewebe an einer Position jeder der Vielzahl von Elektroden (336) innerhalb der Nasenhöhle verbunden sind; und
Verarbeiten der Daten, um ein Energieniveau zu bestimmen, das durch jede der Vielzahl von Elektroden (336) abzugeben ist, sodass die an jeder Position durch jede der Vielzahl von Elektroden (336) abgegebene Energie ausreicht, um das Nervengewebe an jeder Position zu modulieren/abzutragen und eine Beschädigung einer Arterie oder Arterienwand angrenzend an das Nervengewebe an jeder Position zu minimieren und/oder zu verhindern.

2. System (100) nach Anspruch 1, wobei eine Untergruppe der Vielzahl von Elektroden (336) dazu konfiguriert ist, mindestens das Vorhandensein und/oder die Tiefe/den Standort von Nervengewebe an der Position jeder der Vielzahl von Elektroden (336) zu erfassen und mit den Tiefen verbundene Daten an die Steuerung (107) zu liefern.

3. System (100) nach Anspruch 2, wobei eine Untergruppe der Vielzahl von Elektroden (336) dazu konfiguriert ist, nichttherapeutische Stimulationsenergie an jeweilige Positionen innerhalb der Nasenhöhle mit einer Frequenz/Wellenform zum Lokalisieren von Nervengewebe abzugeben und ferner eine oder mehrere Eigenschaften, die mindestens das Vorhandensein und/oder die Tiefe/den Standort des Nervengewebes enthalten, als Reaktion auf die nichttherapeutische Stimulationsenergie zu erfassen.

4. System (100) nach Anspruch 1, wobei die Steuerung (107) dazu konfiguriert ist, eine Energieausgabe von jeder der Vielzahl von Elektroden (336) abzustimmen, nachdem ein anfängliches Energieniveau abgegeben wurde, basierend mindestens teilweise auf Rückkopplungsdaten, die von der Vorrichtung (102) empfangen wurden.

5. System (100) nach Anspruch 4, wobei die Rückkopplungsdaten mit einer Wirksamkeit einer Abtragung des Nervengewebes an jeder Position während und/oder nach dem Abgeben der anfänglichen Energie von jeder der Vielzahl von Elektroden (336) verbunden sind.

6. System (100) nach Anspruch 5, wobei die Rückkopplungsdaten eine oder mehrere Eigenschaften umfassen, die mit Nervengewebe an jeweiligen Positionen innerhalb der Nasenhöhle verbunden sind, wobei die eine oder mehreren Eigenschaften mindestens eines von physiologischen Eigenschaften, bioelektrischen Eigenschaften und thermischen Eigenschaften umfassen.

7. System (100) nach Anspruch 6, wobei eine Untergruppe der Vielzahl von Elektroden (336) dazu konfiguriert ist, nichttherapeutische Stimulationsenergie an jeweilige Positionen innerhalb der Nasenhöhle mit einer Frequenz zum Lokalisieren von Nervengewebe abzugeben und eine oder mehrere Eigenschaften, die mit dem Nervengewebe verbunden sind, als Reaktion auf die nichttherapeutische Stimulationsenergie zu erfassen, wobei die Eigenschaften mindestens eines von physiologischen Eigenschaften, bioelektrischen Eigenschaften und thermischen Eigenschaften umfassen.

8. System (100) nach Anspruch 7, wobei die bioelektrischen Eigenschaften mindestens eines von komplexer Impedanz, Widerstand, Reaktanz, Kapazität, Induktivität, Permittivität, Leitfähigkeit, Nervenerregungsspannung, Nervenerregungsstrom, Depolarisation, Hyperpolarisation, Magnetfeld und induzierter elektromotorischer Kraft umfassen.

9. System (100) nach Anspruch 1, wobei mindestens einige der Elektroden (336) dazu konfiguriert sind, unabhängig voneinander gesteuert (107) zu werden und durch die Steuerung (107) aktiviert zu werden, um dadurch unabhängig voneinander Energie abzugeben.

10. System (100) nach Anspruch 9, wobei mindestens einige der Elektroden (336) unterschiedliche Energieniveaus aufweisen, die an jeweiligen Positionen abzugeben sind und die ausreichen, um Nervengewebe an den jeweiligen Positionen abzutragen.

11. System (100) nach Anspruch 1, wobei die Nasenerkrankung eine Rhinosinusitis umfasst.

## Revendications

1. Système (100) destiné au traitement d'une affection à l'intérieur d'une cavité nasale d'un patient, le système comprenant :
un dispositif de traitement (102) comprenant une tige (116) et un effecteur terminal multi-segment (314) s'étendant à partir de la tige (116) et destiné à délivrer de l'énergie à un ou plusieurs sites cibles à l'intérieur de la cavité nasale du patient, l'effecteur terminal multi-segment (314) comprenant un premier segment (322) et un second segment distinct (324) espacé longitudinalement du premier segment (322), chacun des premier et second segments (322, 324) pouvant être transformé entre une configuration rétractée et une configuration déployée étendue, et chacun des premier et second segments (322, 324) comprenant une pluralité d'électrodes (336) ; et
un dispositif de commande (107) associé fonctionnellement au dispositif de traitement (102) et configuré pour :
recevoir des données en provenance du dispositif de traitement (102) associées au moins à la présence et/ou à la profondeur/l'emplacement d'un tissu neural au niveau de la position de chacune de la pluralité d'électrodes (336) à l'intérieur de la cavité nasale ; et
traiter les données pour déterminer un niveau d'énergie à délivrer par chacune de la pluralité d'électrodes (336) de sorte que l'énergie délivrée au niveau de chaque position par chacune de la pluralité d'électrodes (336) soit suffisante pour moduler/ablater le tissu neural au niveau de chaque position et minimiser et/ou prévenir les dommages à une artère ou à une paroi artérielle adjacente au tissu neural au niveau de chaque position.

2. Système (100) selon la revendication 1, un sous-ensemble de la pluralité d'électrodes (336) étant configuré pour détecter au moins la présence et/ou la profondeur/l'emplacement d'un tissu neural au niveau de la position de chacune de la pluralité d'électrodes (336) et délivrer des données associées aux profondeurs au dispositif de commande (107).

3. Système (100) selon la revendication 2, un sous-ensemble de la pluralité d'électrodes (336) étant configuré pour délivrer une énergie de stimulation non thérapeutique à des positions respectives à l'intérieur de la cavité nasale à une fréquence/forme d'onde de façon à localiser le tissu neural et détecter en outre une ou plusieurs propriétés, comprenant au moins la présence et/ou la profondeur/l'emplacement du tissu neural en réponse à l'énergie de stimulation non thérapeutique.

4. Système (100) selon la revendication 1, ledit dispositif de commande (107) étant configuré pour régler l'émission d'énergie à partir de chacune de la pluralité d'électrodes (336) après la délivrance d'un niveau initial d'énergie sur la base, au moins en partie, de données de rétroaction reçues en provenance du dispositif (102).

5. Système (100) selon la revendication 4, lesdites données de rétroaction étant associées à l'efficacité de l'ablation du tissu neural au niveau de chaque position durant et/ou après avoir délivré l'énergie initiale à partir de chacune de la pluralité d'électrodes (336).

6. Système (100) selon la revendication 5, lesdites données de rétroaction comprenant une ou plusieurs propriétés associées à un tissu neural au niveau de positions respectives dans la cavité nasale, lesdites une ou plusieurs propriétés comprenant au moins une propriété parmi les propriétés physiologiques, les propriétés bioélectriques et les propriétés thermiques.

7. Système (100) selon la revendication 6, un sous-ensemble de la pluralité d'électrodes (336) étant configuré pour délivrer une énergie de stimulation non thérapeutique au niveau des positions respectives à l'intérieur de la cavité nasale à une fréquence permettant de localiser le tissu neural et de détecter une ou plusieurs propriétés associées avec le tissu neuronal en réponse à l'énergie de stimulation non thérapeutique, lesdites propriétés comprenant au moins une propriété parmi les propriétés physiologiques, les propriétés bioélectriques et les propriétés thermiques.

8. Système (100) selon la revendication 7, lesdites propriétés bioélectriques comprenant au moins une propriété parmi l'impédance complexe, la résistance, la réactance, la capacité, l'inductance, la permittivité, la conductivité, la tension d'activation nerveuse, le courant d'activation nerveuse, la dépolarisation, l'hyperpolarisation, le champ magnétique et la force électromotrice induite.

9. Système (100) selon la revendication 1, au moins certaines des électrodes (336) étant configurées pour être commandées indépendamment (107) et activées par le dispositif de commande (107) pour ainsi délivrer l'énergie indépendamment les unes des autres.

10. Système (100) selon la revendication 9, au moins certaines des électrodes (336) comportant différents niveaux d'énergie à délivrer au niveau de positions respectives, suffisants pour ablater le tissu neural au niveau des positions respectives.

11. Système (100) selon la revendication 1, ladite affection nasale comprenant la rhino-sinusite.
